# EUROPEAN PATENT SPECIFICATION

(11) **EP 3 818 155 B1**
(45) Date of publication and mention of the grant of the patent: **06.09.2023**
(21) Application number: 19734413.8
(22) Date of filing: 03.07.2019
(51) Int. Cl.: C12N 9/88, C12P 5/02

(54) **3-METHYLCROTONIC ACID DECARBOXYLASE (MDC) VARIANTS**
3-METHYLCROTONSÄURE-DECARBOXYLASE(MDC)-VARIANTEN
VARIANTES DE DÉCARBOXYLASE D'ACIDE 3-MÉTHYLCROTONIQUE (MDC)

(30) Priority: 03.07.2018 EP 18181522
(43) Date of publication of application: 12.05.2021
(73) Proprietor: Global Bioenergies, 91000 Evry (FR)
(72) Inventor: STRICHER, François, 67202 Wolfisheim (FR); VILLIERS, Benoît, 37000 Tours (FR)
(74) Representative: Vossius & Partner Patentanwälte Rechtsanwälte mbB
(86) International application number: PCT/EP2019/067788
(87) International publication number: WO 2020/007886

(56) References cited:
- WO-A1-2017/191239
- WO-A2-2017/085167
- DATABASE UniProt [Online] 4 March 2015 (2015-03-04), "RecName: Full=UbiD-like decarboxylase {ECO:0000256|HAMAP-Rule:MF_01983}; EC=4.1.1.- {ECO:0000256|HAMAP-Rule:MF_01983}; AltName: Full=Ferulic acid decarboxylase-like protein {ECO:0000256|HAMAP-Rule:MF_01983};", XP002787741, retrieved from EBI accession no. UNIPROT:A0A0A8EV26 Database accession no. A0A0A8EV26 cited in the application
- DATABASE UniProt [Online] 13 April 2016 (2016-04-13), "RecName: Full=UbiD-like decarboxylase {ECO:0000256|HAMAP-Rule:MF_01983}; EC=4.1.1.- {ECO:0000256|HAMAP-Rule:MF_01983}; AltName: Full=Ferulic acid decarboxylase-like protein {ECO:0000256|HAMAP-Rule:MF_01983};", XP002794753, retrieved from EBI accession no. UNIPROT:A0A101TBL5 Database accession no. A0A101TBL5
- DATABASE UniProt [Online] 2 November 2016 (2016-11-02), "RecName: Full=UbiD-like decarboxylase {ECO:0000256|HAMAP-Rule:MF_01983}; EC=4.1.1.- {ECO:0000256|HAMAP-Rule:MF_01983}; AltName: Full=Ferulic acid decarboxylase-like protein {ECO:0000256|HAMAP-Rule:MF_01983};", XP002794754, retrieved from EBI accession no. UNIPROT:A0A1B2GL43 Database accession no. A0A1B2GL43
- DATABASE REfseq [Online] 23 July 2017 (2017-07-23), "UBID family decarboxylase [Streptomyces sp.NRRL S-1448].", XP002794755,accession no. ebi Database accession no. WP_030411030
- DATABASE UniProt [Online] 12 April 2017 (2017-04-12), "RecName: Full=UbiD-like decarboxylase {ECO:0000256|HAMAP-Rule:MF_01983}; EC=4.1.1.- {ECO:0000256|HAMAP-Rule:MF_01983}; AltName: Full=Ferulic acid decarboxylase-like protein {ECO:0000256|HAMAP-Rule:MF_01983};", XP002787742, retrieved from EBI accession no. UNIPROT:A0A1S2PMH4 Database accession no. A0A1S2PMH4

## Description

Described are 3-methylcrotonic acid decarboxylase (MDC) variants showing an improved activity in converting 3-methylcrotonic acid into isobutene as well as methods for the production of isobutene using such enzyme variants.

A large number of chemical compounds are currently derived from petrochemicals. Alkenes (such as ethylene, propylene, the different butenes, or else the pentenes, for example) are used in the plastics industry, for example for producing polypropylene or polyethylene, and in other areas of the chemical industry and that of fuels.

Butylene exists in four forms, one of which, isobutene (also referred to as isobutylene), enters into the composition of methyl-tert-butyl-ether (MTBE), an anti-knock additive for automobile fuel. Isobutene can also be used to produce isooctene, which in turn can be reduced to isooctane (2,2,4-trimethylpentane); the very high octane rating of isooctane makes it the best fuel for so-called "gasoline" engines. Alkenes such as isobutene are currently produced by catalytic cracking of petroleum products (or by a derivative of the Fischer-Tropsch process in the case of hexene, from coal or gas). The production costs are therefore tightly linked to the price of oil. Moreover, catalytic cracking is sometimes associated with considerable technical difficulties which increase process complexity and production costs.

The production by a biological pathway of alkenes such as isobutene is called for in the context of a sustainable industrial operation in harmony with geochemical cycles. The first generation of biofuels consisted in the fermentative production of ethanol, as fermentation and distillation processes already existed in the food processing industry. The production of second generation biofuels is in an exploratory phase, encompassing in particular the production of long chain alcohols (butanol and pentanol), terpenes, linear alkanes and fatty acids. Two recent reviews provide a general overview of research in this field: Ladygina et al. (Process Biochemistry 41 (2006), 1001) and Wackett (Current Opinions in Chemical Biology 21 (2008), 187).

Different routes for the enzymatic generation of isobutene have previously been described; see, e.g., Fujii et al. (Appl. Environ. Microbiol. 54 (1988), 583); Gogerty et al. (Appl. Environm. Microbiol. 76 (2010), 8004-8010) and van Leeuwen et al. (Appl. Microbiol. Biotechnol. 93 (2012), 1377-1387) and WO2010/001078.

In addition to these routes, there are also alternative routes for the provision of isobutene utilizing the enzymatic conversion of 3-methylcrotonic acid into isobutene by a decarboxylation reaction. A decarboxylation is a chemical reaction that removes a carboxyl group and releases carbon dioxide (CO₂).

The decarboxylation of 3-methylcrotonic acid has already been suggested in US-A1-2009/0092975 while there is no experimental evidence for this conversion. In US-A1-2009/0092975, a nucleic acid sequence called PAD1 derived from *Saccharomyces cerevisiae* is described and is disclosed to encode a decarboxylation enzyme. This enzyme is suggested to be useful as a selectable marker in a recombinant organism while it is described that a "weak acid" may be used as the selecting agent. 3-methylcrotonic acid is mentioned, among many others, as a potential "weak acid". However, it was only later found that the above PAD1, in reality, does not provide for the decarboxylase activity.

In fact, the bacterial *ubiD* and *ubiX* or the homologous eukaryotic *fdc1* and *pad1* genes have been implicated in the non-oxidative reversible decarboxylation. The combined action of phenylacrylic acid decarboxylase (PAD) and ferulic acid decarboxylase (FDC) is considered to be essential for the decarboxylation of phenylacrylic acid in *Saccharomyces cerevisiae* (J. Biosci. Bioeng. 109, (2010), 564-569; AMB Express, 5:12 (2015) 1-5; ACS Chem. Biol. 10 (2015), 1137-1144).

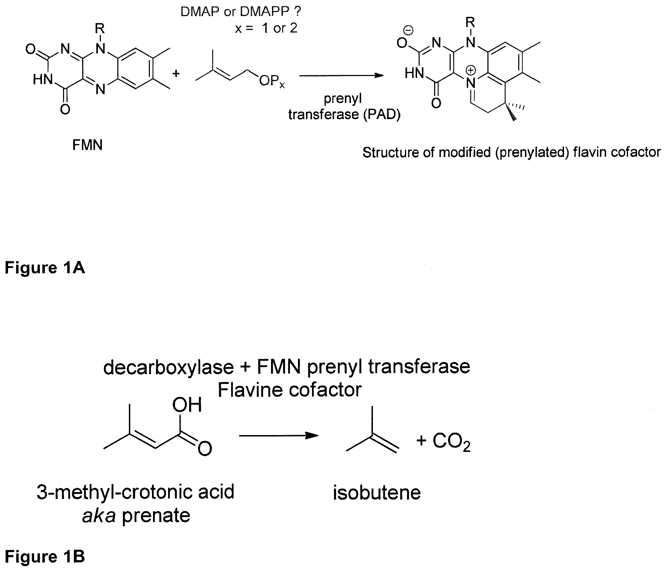

Recently, the above enzyme family described as phenylacrylic acid decarboxylase (PAD) was characterized as an FMN prenyl-transferase and no longer as a decarboxylase. It has been shown that Fdc1 (but not PAD) is solely responsible for the reversible decarboxylase activity and that it requires a new type of cofactor, namely a prenylated flavin synthesized by the associated UbiX (or Pad1) protein. Thus, the real enzymatic activity of this PAD enzyme has been identified as the transformation of a flavin mononucleotide (FMN) cofactor with a prenyl moiety (from di-methyl-allyl-phosphate or pyrophosphate called DMAP or DMAPP). This reaction is shown in **Figure 1A****.**

Accordingly, in contrast to the prior art's belief, the real decarboxylase is the Ferulic Acid Decarboxylase (FDC) in association with the modified FMN (prenylated-FMN). This reaction is shown in **Figure 1B****.** This mechanism of the Ferulic Acid Decarboxylase (FDC) in association with the modified FMN (prenylated-FMN) (the latter provided by the PAD enzyme) was recently described and involves a surprising enzymatic mechanism, i.e., an α,β-unsaturated acid decarboxylation via a 1,3-dipolar cyclo-addition. Moreover, the structure of this FDC decarboxylase has recently been elucidated (Nature 522 (2015), 497-501; Nature, 522 (2015), 502-505; Appl. Environ. Microbiol. 81 (2015), 4216- 4223).

Moreover, methods for the production of isobutene comprising the enzymatic conversion of 3-methylcrotonic acid into isobutene utilizing the above enzymes has previously been described wherein corresponding enzymes have artificially been implemented in a pathway which ultimately leads to the production of isobutene (WO 2017/085176). In WO 2017/085176 FMN-dependent decarboxylase enzymes catalysing the decarboxylation of 3-methylcrotonic acid into isobutene have been described which belong to the 3-polyprenyl-4-hydroxybenzoate decarboxylase (UbiD) family of enzymes while, *inter alia,* an UbiD enzyme derived from *Hypocrea atroviridis* (UniProt Accession number Q9NLP8) has been described.

Moreover, previously, improved variants of enzymes which are capable of converting 3-methylcrotonic acid into isobutene have been descried while the above FDC of *Hypocrea atroviridis* has been used as a model enzyme (WO 2017/191239).

Although the above means and methods allow to produce isobutene from 3-methylcrotonic acid while means and methods which allow to increase the production of isobutene from 3-methylcrotonic acid have already been described, there is still a need for further improvements, in particular as regards a further increase in efficiency of the process so as to make it more suitable for industrial purposes.

The present application addresses this need by providing the embodiments as defined in the claims.

In a first aspect, the present invention provides a variant of a 3-methylcrotonic acid decarboxylase (MDC) showing an improved activity in converting 3-methylcrotonic acid into isobutene over the corresponding MDC from which it is derived.

An improved enzyme variant or an enzyme variant capable of catalyzing a reaction with increased activity is defined as an enzyme variant which differs from the wildtype enzyme and which catalyzes the conversion of 3-methylcrotonic acid into isobutene so that the specific activity of the enzyme variant is higher than the specific activity of the wildtype enzyme for at least one given concentration of a 3-methylcrotonic acid (preferably any 3-methylcrotonic acid higher than 0 M and up to 1 M). A specific activity is defined as the number of moles of substrate converted to moles of product by unit of time by mole of enzyme. K_{cat} (turnover number) is the specific activity at saturating concentration of substrate.

In particular, in accordance with this first aspect, the present invention provides enzymes which are capable of converting 3-methylcrotonic acid into isobutene with a turnover rate of at least 1 ×10⁻³ s⁻¹ of 3-methylcrotonic acid into isobutene. Such enzymes can be provided by effecting mutations at specific positions in an 3-methylcrotonic acid decarboxylase (MDC) and the variants obtained by effecting such mutations show an improved activity in catalyzing the conversion of 3-methylcrotonic acid into isobutene. In a preferred embodiment, the enzyme is capable of converting 3-methylcrotonic acid into isobutene with a turnover rate of at least 2×10⁻³ s⁻¹ of 3-methylcrotonic acid into isobutene and in a particularly preferred embodiment of at least 4×10⁻³ s⁻¹. In a most preferred embodiment, the enzyme has a turnover rate of at least 10×10⁻³ s⁻¹ or at least 1s⁻¹, or at least 10s⁻¹ and even more preferably of at least 100s⁻¹ of 3-methylcrotonic acid into isobutene. The corresponding wild-type enzyme has a turnover rate of about 1 ×10⁻³ s⁻¹ of 3-methylcrotonic acid into isobutene.

In the context of the present invention, an "improved activity" means that the activity of the enzyme in question is at least 10%, preferably at least 20%, more preferably at least 30% or 50%, even more preferably at least 70% or 80% and particularly preferred at least 90% or 100% higher than that of the enzyme from which the variant is derived, preferably higher than that of the enzyme represented by SEQ ID NO: 1. In even more preferred embodiments the improved activity may be at least 150%, at least 200%, at least 300%, at least 750% or at least 1000% higher than that of the corresponding enzyme from which the variant is derived, preferably higher than that of the enzyme represented by SEQ ID NO:1. In a particularly preferred embodiment, the activity is measured by using an assay with purified enzyme and chemically synthesized substrates, as described below. The improved activity of a variant can be measured as a higher isobutene production in a given time under defined conditions, compared with the parent enzyme. This improved activity can result from a higher turnover number, e.g. a higher kcat value. It can also result from a lower Km value. It can also result from a higher kcat/Km value. Finally, it can result from a higher solubility, or stability of the enzyme. The degree of improvement can be measured as the improvement in isobutene production. The degree of improvement can also be measured in terms of kcat improvement, of kcat/Km improvement, or in terms of Km decrease, in terms of soluble protein production or in terms of protein stability.

In another embodiment, the enzyme variants which the present invention provides are capable of converting 3-methylcrotonic acid into isobutene with an activity which is at least 1.25 times as high compared to the turnover rate of the corresponding wild type enzyme having the amino acid sequence as shown in SEQ ID NO:1. In a more preferred embodiment, the enzyme variants which are capable of converting 3-methylcrotonic acid into isobutene have a turnover rate (i.e., a k_{cat}-value) which is at least 2 times, at least 3 times, at least 5 times or even at least 10 times as high compared to the turnover rate of the corresponding wild type enzyme having the amino acid sequence as shown in SEQ ID NO:1. In even more preferred embodiments, the turnover rate is at least 100 times or even at least 500 times as high compared to that of the corresponding wild type enzyme having the amino acid sequence as shown in SEQ ID NO:1.

Such enzyme variants are obtained by effecting mutations at specific positions in the amino acid sequence of an MDC and the variants obtained by effecting such mutations show an improved activity in catalyzing the conversion of 3-methylcrotonic acid into isobutene. The activity of an enzyme capable of converting 3-methylcrotonic acid into isobutene may be determined by methods known to the person skilled in the art. In one embodiment, this activity is determined as described in the Examples appended hereto. In a particular embodiment this activity can be measured by incubating the enzyme, preferably a cell lysate containing the overexpressed recombinant protein, in vitro. Alternatively, a purified enzyme can be used or an in vivo assay.

More specifically, the activity of the MDC variants for the conversion of 3-methylcrotonic acid into isobutene can be assessed by an enzymatic in vitro assay based on purified proteins and on the detection of isobutene by gas chromatography. The turnover rate of the enzyme to be assessed may be examined as outlined in the following: Michaelis-Menten k_{cat} and Kₘ steady state kinetic constants for the reaction of conversion of 3-methylcrotonic acid into isobutene may be determined using the following protocol:
The enzymatic assay for quantifying the conversion of 3-methylcrotonic acid into isobutene is carried out in a 2 ml glass vial at 30°C in a 50mM potassium phosphate pH 7.5 buffer; 20 mM NaCl, 3 mM MgCl₂, 5mM DTT, 0.5 mg/ml of a purified enzyme of the MDC variant to be tested, 100 µl of a lysate containing a FMN prenyltransferase (i.e., a Flavin prenyltransferase UbiX protein from *E*. *coli* expressed and prepared as outlined further below) as well as different concentrations of the substrate 3-methylcrotonic acid ranging from 0 to 128 mM. A control without an MDC enzyme is performed in parallel. After 60 minutes, the reaction is stopped by incubating at 80°C for 2 min. The rate of isobutene production is quantified by gas chromatography as follows.

The isobutene formed in the reaction headspace is analysed by gas chromatography (GC) equipped with a flame ionization detector (FID). For the GC headspace analysis, one ml of the headspace gas is separated in a Bruker GC-450 system equipped with a GS-alumina column (30 m × 0.53 mm) (Agilent) using isothermal mode at 130°C. Nitrogen is used as carrier gas with a flow rate of 6 ml/min. The enzymatic reaction product is identified by comparison with an isobutene standard. Under these GC conditions, the retention time of isobutene is 2.42 min. From the rate of isobutene production, and using the Michaelis-Menten approximation, the enzyme catalytic efficiency can then be computed. The production rates of isobutene (mole of PV/mole enzyme/sec) are plotted as a function of the concentration of 3-methylcrotonic acid and the curve is fitted using the Michaelis Menten equation (V=(Vₘₐₓ*(substrate))/(Kₘ+(substrate))) to extract the k_{cat} (s⁻¹) and the Kₘ values (mM).

The MDC variant to be tested can be provided according to the following protocol: The MDC to be tested is subcloned into the pETDuetTM-1 co-expression vector. The vector contains a stretch of 6 histidine codons after the methionine initiation codon of the ferulic acid decarboxylases in order to provide an affinity tag for purification.

Competent *E*. *coli* BL21 (DE3) cells (Novagen) are transformed with this vector according to standard heat shock procedures and plated out onto LB-agar plates supplemented with the appropriate antibiotic. Cells are grown overnight at 30°C until individual colonies reach the desired size. A single colony is then picked and individually transferred into 5 ml of liquid LB medium supplemented with the appropriate antibiotic. Cell growth is carried out with shaking for 16 hours at 30°C. The LB culture of the transformed cells is used to inoculate a culture using ZYM-5052 auto-induction medium (Studier FW, Prot. Exp. Pur. 41, (2005), 207-234) and the culture is grown with shaking (160 rpm) at 30°C during 24 h. The cells are collected by centrifugation at 4°C, 10,000 rpm for 20 min and the pellets are frozen and stored at - 80°C. The pellets containing the overexpressed protein of a 500 ml of cultured cells is thawn on ice and resuspended in 15 ml of 50 mM potassium phosphate buffer containing 200 mM NaCl, 10 mM MgCl₂, 10 mM imidazole and 1 mM DTT. Twenty microliters of lysonase (Novagen) is added and the cells are incubated for 10 minutes at room temperature and then returned to ice for 20 minutes. Cell lysis is then completed by sonication for 2 × 15 seconds.

The bacterial extracts are then clarified by centrifugation at 4°C, 4000 rpm for 40 min. The clarified bacterial lysates are loaded onto a PROTINO-2000 Ni-TED column (Macherey-Nagel) allowing adsorption of 6-His tagged proteins. Columns are washed and the enzymes of interest are eluted with 6 ml of 50 mM potassium phosphate buffer containing 250 mM imidazole. Eluates are then concentrated, desalted on a Amicon Ultra-4 10 kDa filter unit (Millipore) and enzymes are resuspended in 50 mM potassium phosphate buffer containing 1 mM DTT and 20 mM NaCl. Protein concentrations are determined by direct UV 280 nm measurement on a NanoDrop 1000 spectrophotometer (Thermo Scientific) or by a Bradford assay (BioRad).

Correspondingly, the cDNA of a Flavin prenyltransferase UbiX protein from *E. coli* is cloned and recombinantly expressed, purified and quantified.

As described in the above enzymatic in vitro assay for determining the activity of the MDC variants of the present invention, UbiX does not necessarily have to be provided in a recombinantly expressed and subsequently purified manner. Therefore, UbiX may alternatively also be provided in the form of a UbiX-containing cell lysate whithout purifying it as described in the following.

The Flavin prenyltransferase UbiX protein from *E*. *coli* is cloned in the vector pCAN. The Flavin prenyltransferase UbiX protein from *E. coli* was purchased from NAIST (Nara Institute of Science and Technology, Japan, ASKA collection).

Competent *E*. *coli* BL21 (DE3) cells (Novagen) are transformed with this vector according to standard heat shock procedures and plated out onto LB-agar plates supplemented with the appropriate antibiotic. Cells are grown overnight at 30°C until individual colonies reach the desired size. A single colony is then picked and individually transferred into 5 ml of liquid LB medium supplemented with the appropriate antibiotic. Cell growth is carried out with shaking for 16 hours at 30°C. The LB culture of the transformed cells is used to inoculate a ZYM-5052 auto-induction medium (Studier FW, Prot. Exp. Pur. 41, (2005), 207-234) and the culture is grown with shaking (160 rpm) using at 30°C during 24 h. The cells are collected by centrifugation at 4°C, 10,000 rpm for 20 min and the pellets are stored at -80°C. Pellets from 500 ml of cultured cells are thawed on ice and resuspended in 15 ml of 50 mM potassium phosphate buffer containing 200 mM NaCl, 10 mM MgCl₂, 10 mM imidazole and 1 mM DTT. Twenty microliters of lysonase (Novagen) is added. Cells are incubated 10 minutes at room temperature and then returned to ice for 20 minutes. Cell lysis is completed by sonication for 2 × 15 seconds. The cellular lysate containing the UbiX protein is kept on ice.

Alternatively to the above *in vitro* assays, the activity of the MDC variants for the conversion of 3-methylcrotonic acid into isobutene can be assessed by an *in vivo* testing. This coupled *in vivo* assay is based on the use of a bacterial strain transformed with an expression vector that contains the coding sequences leading to the production of the MDC variant and the Flavin prenyltransferase UbiX protein from *E. coli* (SEQ ID NO:2). Thus, the MDC variant to be tested is subcloned into a pETDuet^{™} -1 co-expression vector (Novagen) in addition to the cDNA of the Flavin prenyltransferase UbiX protein from *E*. *coli.*

The MDC variant of the present invention to be tested is used to catalyze the decarboxylation reaction of 3-methylcrotonic acid into isobutene while the Flavin prenyltransferase UbiX protein from *E. coli* provides the modified flavin cofactor. Thus, in the coupled *in vivo* assay, a bacterial strain is used which is transformed with the above expression vector.

The transformed strain is first plated out onto LB-agar plates supplemented with the appropriate antibiotic. Cells are then grown overnight at 30°C until individual colonies reach the desired size. Single colonies are then picked and individually transferred into either 50 or 500 µL of liquid LB medium supplemented with the appropriate antibiotic. Cell growth is carried out with shaking for 20 hours at 30°C. The LB cultures are used to inoculate 300 µL in 384 deepwell microplates or 1 mL in 96 deepwell microplates of auto-induction medium (Studier FW, Prat. Exp. Pur. 41, (2005), 207-234) supplemented with the appropriate antibiotic and grown in a shaking incubator set at 700rpm and 85% humidity for 24h at 30°C in order to produce the two types of recombinant enzymes. The cell pellet containing these overexpressed recombinant enzymes is then resuspended in 40 µL of minimum medium (pH 7.5, Phosphate 100 mM, Glucose 10g.L⁻¹, MgSO₄ 1mM) supplemented with 10mM 3-methylcrotonic acid in 384 deepwell microplates or in 400 µL of minimum medium (pH 7.5, Phosphate 100 mM, Glucose 10g.L⁻¹, MgSO₄ 1mM) supplemented with 10 mM 3-methylcrotonic acid in 96 deepwell microplates and incubated for a further 2 or 4 hours in a shaking incubator at 37°C, 700 rpm. During this step, the MDC variant catalyses the decarboxylation of 3-methylcrotonic acid into isobutene. After 5 min inactivation at 80°C, the isobutene produced is quantified by gas chromatography as follows. 100 µL of headspace gases from each enzymatic reaction are injected in a Brucker GC-450 system equipped with a Flame Ionization Detector (FID). Compounds present in samples are separated by chromatography using a RTX-1 column at 100°C with a 1 mL/min constant flow of nitrogen as carrier gas. Upon injection, peak areas of isobutene are calculated.

By providing the above described enzyme variant, the present invention allows to dramatically increase the production efficiency of isobutene from 3-methylcrotonic acid.

The term "3-methylcrotonic acid decarboxylase (MDC)" refers to an enzyme which can catalyze the decarboxylation of 3-methylcrotonic acid into isobutene. A decarboxylation is a chemical reaction that removes a carboxyl group and releases carbon dioxide. This activity can be measured by methods known in the art and as described above. In a preferred embodiment, the MDC is a Ferulic Acid Decarboxylase (FDC) or is derived from such an enzyme. FDCs belong to the enzyme class EC 4.1.1.-. As mentioned above, it has originally been described that an FDC in association with a modified FMN (prenylated-FMN) is capable of catalyzing an α,β-unsaturated decarboxylation via a 1,3-dipolar cyclo-addition and, more specifically, capable of catalyzing the decarboxylation of 3-methylcrotonic acid into isobutene. Thus, in the context of the present invention, the term FDC relates to enzymes capable of catalyzing the decarboxylation of 3-methylcrotonic acid into isobutene, preferably when provided with a prenylated FMN.

FDC enzymes have, e.g., been described in Saccharomyces cerevisiae, Enterobacter sp., Bacillus pumilus, Aspergillus niger or Candida dubliniensis. Hence, in preferred embodiments, the FDC is derived from Saccharomyces cerevisiae (Uniprot accession number Q03034), Enterobacter sp. (Uniprot accession number V3P7U0), Bacillus pumilus (Uniprot accession number Q45361), Aspergillus niger (Uniprot accession number A2R0P7) or Candida dubliniensis (Uniprot accession number B9WJ66). In more preferred embodiments, the FDC is a 3-polyprenyl-4-hydroxybenzoate decarboxylase (UbiD). 3-polyprenyl-4-hydroxybenzoate decarboxylases have, e.g., been described in Hypocrea atroviridis, Sphaerulina musiva, Penecillinum requeforti, Fusarium oxysporum f. sp. lycopersici, Saccharomyces kudriavzevii, Saccaromyces cerevisiae, Aspergillus parasiticus, Candida albicans, Grosmannia clavigera, Escherichia coli, Bacillus megaterium, Methanothermobacter sp. CaT2 or Mycobacterium chelonae 1518. Hence, in more preferred embodiments, the FDC enzyme variant capable of catalyzing the decarboxylation of 3-methylcrotonic acid into isobutene is derived from a 3-polyprenyl-4-hydroxybenzoate decarboxylase (UbiD) from Hypocrea atroviridis (UniProt Accession number G9NLP8), Sphaerulina musiva (UniProt Accession number M3DF95), Penecillinum requeforti (UniProt Accession number W6QKP7), Fusarium oxysporum f. sp. lycopersici (UniProt Accession number W9LTH3), Saccharomyces kudriavzevii (UniProt Accession number J8TRN5), Saccaromyces cerevisiae, Aspergillus parasiticus, Candida albicans, Grosmannia clavigera, Escherichia coli (Uniprot accession number P0AAB4), Bacillus megaterium (Uniprot accession number D5DTL4), Methanothermobacter sp. CaT2 (Uniprot accession number T2GKK5) or Mycobacterium chelonae 1518 (Uniprot accession number X8EX86). Preferably, the MDC is an enzyme which is associated with and/or depends on an FMN prenyl transferase. As mentioned above, the enzymatic conversion of 3-methylcrotonic acid into isobutene utilizing an FMN-dependent decarboxylase is preferably associated with an FMN prenyl transferase and relies on a reaction of two consecutive steps catalyzed by the two enzymes, i.e., the FMN-dependent decarboxylase (catalyzing the actual decarboxylation of 3-methylcrotonic acid into isobutene) with an associated FMN prenyl transferase which provides the modified flavin cofactor. The flavin cofactor may preferably be FMN or FAD. FMN (flavin mononucleotide; also termed riboflavin-5'-phosphate) is a biomolecule produced from riboflavin (vitamin B2) by the enzyme riboflavin kinase and functions as prosthetic group of various reactions. FAD (flavin adenine dinucleotide) is a redox cofactor, more specifically a prosthetic group, involved in several important reactions in metabolism. The FMN prenyl transferases which may be associated with the MDC variants of the present invention are described in more detail further below.

The present invention provides now improved variants of enzymes which are capable of converting 3-methylcrotonic acid into isobutene. The inventors used as a model enzyme the FDC, more specifically the UbiD-like decarboxylase of *Streptomyces sp.* 769 (UniProt Accession number A0A0A8EV26; the corresponding encoding gene GZL_07100) shown in SEQ ID NO:1 and could show that it is possible to provide variants of this enzyme which show increased activity with respect to the conversion of 3-methylcrotonic acid into isobutene.

The model enzyme, i.e., the UbiD-like decarboxylase of *Streptomyces sp. 769,* as used by the inventors has the amino acid sequence as shown in SEQ ID NO:1.

In one preferred embodiment the variants of the present invention are characterized by the feature that they are derived from an MDC, more preferably from an MDC having the amino acid sequence shown in SEQ ID NO:1 or a highly related sequence (at least 80% identical) and in which mutations are effected at one or more of the below indicated positions and by the feature that they show the ability to convert 3-methylcrotonic acid into isobutene and that they can do this with an improved activity. The variant according to the present invention is derived from a sequence which shows at least 80% sequence identity to SEQ ID NO:1 and in which one or more substitutions and/or deletions and/or insertions at the positions indicated herein have been effected.

However, the teaching of the present invention is not restricted to the MDC enzyme of *Streptomyces sp. 769* shown in SEQ ID NO:1 which had been used as a model enzyme but can be extended to MDC enzymes from other organisms or to enzymes which are structurally related to SEQ ID NO:1 such as, e.g., truncated variants of the enzyme. Thus, the present invention also relates to variants of MDCs which are structurally related to the *Streptomyces sp. 769* sequence (SEQ ID NO: 1) and which show one or more substitutions at positions corresponding to any of the positions as indicated herein. The term "structurally related" refers to MDCs which show a sequence identity of at least n% to the sequence shown in SEQ ID NO:1 with n being an integer between 80 and 100, preferably 81, 82, 83, 84, 85, 86, 87, 88, 89, 90, 91, 92, 93, 94, 95, 96, 97, 98 or 99. In a preferred embodiment the structurally related MDC stems from a bacteria, more preferably from an organism of the phylum of Actinobacteria, even more preferably from an organism of the class of Actinobacteria, the order of Actinomycetales, the family of Streptomycetaceae or the genus Streptomyces sp., most preferably *Streptomyces sp. 769.*

Thus, in one embodiment, the variant of an MDC according to the present invention has or preferably is derived from a sequence which is at least n % identical to SEQ ID NO:1 with n being an integer between 80 and 100, preferably 81, 82, 83, 84, 85, 86, 87, 88, 89, 90, 91, 92, 93, 94, 95, 96, 97, 98 or 99, and it has (a) substitution(s) and/or (a) deletion and/or (an) insertion(s) at a position as indicated herein.

In more preferred embodiments, the variant of an MDC according to the present invention has or preferably is derived from a sequence which is at least n % identical to SEQ ID NO:1 with n being preferably 94 or 97, and it has (a) substitution(s) at a position as indicated herein.

When the sequences which are compared do not have the same length, the degree of identity either refers to the percentage of amino acid residues in the shorter sequence which are identical to amino acid residues in the longer sequence or to the percentage of amino acid residues in the longer sequence which are identical to amino acid residues in the shorter sequence. Preferably, it refers to the percentage of amino acid residues in the shorter sequence which are identical to amino acid residues in the longer sequence. The degree of sequence identity can be determined according to methods well known in the art using preferably suitable computer algorithms such as CLUSTAL.

When using the Clustal analysis method to determine whether a particular sequence is, for instance, at least 60% identical to a reference sequence default settings may be used or the settings are preferably as follows: Matrix: blosum 30; Open gap penalty: 10.0; Extend gap penalty: 0.05; Delay divergent: 40; Gap separation distance: 8 for comparisons of amino acid sequences. For nucleotide sequence comparisons, the Extend gap penalty is preferably set to 5.0.

In a preferred embodiment ClustalW2 is used for the comparison of amino acid sequences. In the case of pairwise comparisons/alignments, the following settings are preferably chosen: Protein weight matrix: BLOSUM 62; gap open: 10; gap extension: 0.1. In the case of multiple comparisons/alignments, the following settings are preferably chosen: Protein weight matrix: BLOSUM 62; gap open: 10; gap extension: 0.2; gap distance: 5; no end gap. Preferably, the degree of identity is calculated over the complete length of the sequence.

Amino acid residues located at a position corresponding to a position as indicated herein in the amino acid sequence shown in SEQ ID NO:1 can be identified by the skilled person by methods known in the art. For example, such amino acid residues can be identified by aligning the sequence in question with the sequence shown in SEQ ID NO:1 and by identifying the positions which correspond to the below indicated positions of SEQ ID NO:1. The alignment can be done with means and methods known to the skilled person, e.g. by using a known computer algorithm such as the Lipman-Pearson method (Science 227 (1985), 1435) or the CLUSTAL algorithm. It is preferred that in such an alignment maximum homology is assigned to conserved amino acid residues present in the amino acid sequences.

In a preferred embodiment ClustalW2 is used for the comparison of amino acid sequences. In the case of pairwise comparisons/alignments, the following settings are preferably chosen: Protein weight matrix: BLOSUM 62; gap open: 10; gap extension: 0.1. In the case of multiple comparisons/alignments, the following settings are preferably chosen: Protein weight matrix: BLOSUM 62; gap open: 10; gap extension: 0.2; gap distance: 5; no end gap.

When the amino acid sequences of MDCs are aligned by means of such a method, regardless of insertions or deletions that occur in the amino acid sequences, the positions of the corresponding amino acid residues can be determined in each of the MDCs.

In the context of the present invention, "substituted with another amino acid residue" means that the respective amino acid residues at the indicated position can be substituted with any other possible amino acid residues, e.g. naturally occurring amino acids or non-naturally occurring amino acids (Brustad and Arnold, Curr. Opin. Chem. Biol. 15 (2011), 201-210), preferably with an amino acid residues selected from the group consisting of alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine and valine. Preferred substitutions for certain positions are indicated further below. Moreover, the term "substituted" or "substitution" also means that the respective amino acid residue at the indicated position is modified.

Such modifications include naturally occurring modifications and non-naturally occurring modifications. Naturally occurring modifications include but are not limited to eukaryotic post-translational modification, such as attachment of functional groups (e.g. acetate, phosphate, hydroxyl, lipids (myristoylation of glycine residues) and carbohydrates (e.g. glycosylation of arginine, asparagine etc.). Naturally occurring modifications also encompass the change in the chemical structure by citrullination, carbamylation and disulphide bond formation between cysteine residues; attachment of co-factors (FMN or FAD that can be covalently attached) or the attachment of peptides (e.g. ubiquitination or sumoylation).

Non-naturally occurring modifications include, e.g., in vitro modifications such as biotinylation of lysine residue or the inclusion of non-canonical amino acids (see Liu and Schultz, Annu. Rev. Biochem. 79 (2010), 413-44 and Wang et al., Chem. Bio. 2009 March 27; 16 (3), 323-336; doi:101016/jchembiol.2009.03.001).

In the context of the present disclosure, for reference only, it is disclosed that "deleted" or "deletion" means that the amino acid at the corresponding position is deleted. In the context of the present disclosure, for reference only, it is disclosed that "inserted" or "insertion" means that at the respective position one or two, preferably one amino acid residue is inserted, preferably in front of the indicated position.

In a second aspect, the present invention provides a variant of a 3-methylcrotonic acid decarboxylase (MDC) showing an improved activity in converting 3-methylcrotonic acid into isobutene over the corresponding MDC from which it is derived, wherein the MDC variant is characterized in that it shows one or more substitutions in comparison to the corresponding sequence from which it is derived and wherein these substitutions occur at one or more of the positions corresponding to positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462 and 473 in the amino acid sequence shown in SEQ ID NO:1.

The present invention relates in a preferred embodiment to an MDC variant having an amino acid sequence as shown in SEQ ID NO:1 or an amino acid sequence having at least 80% sequence identity to SEQ ID NO:1, in which one or more amino acid residues at a position selected from the group consisting of positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462 and 473 in the amino acid sequence shown in SEQ ID NO:1 or at a position corresponding to any of these positions, are substituted with another amino acid residue at one or more of these positions and wherein said MDC variant has an improved activity in converting 3-methylcrotonic acid into isobutene.

Thus, the present invention relates in a preferred embodiment to a variant of a 3-methylcrotonic acid decarboxylase (MDC) showing an improved activity in converting 3-methylcrotonic acid into isobutene over the MDC of SEQ ID NO:1, wherein said variant is characterized in that:
it has an amino acid sequence having at least 80 % sequence identity to SEQ ID NO:1; and
it shows one or more amino acid substitutions with an another amino acid residue at a position selected from the group consisting of positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462 and 473 in the amino acid sequence shown in SEQ ID NO:1 or at a position corresponding to any of these positions.

The present invention relates in a preferred embodiment to an MDC variant having an amino acid sequence as shown in SEQ ID NO:1 or an amino acid sequence having at least 80% sequence identity to SEQ ID NO:1, in which one or more amino acid residues at a position selected from the group consisting of positions 406, 198, 240, 390, 402, 408 and 449 in the amino acid sequence shown in SEQ ID NO:1 or at a position corresponding to any of these positions, are substituted with another amino acid residue at one or more of these positions and wherein said MDC variant has an improved activity in converting 3-methylcrotonic acid into isobutene.

Thus, the present invention relates in a preferred embodiment to a variant of a 3-methylcrotonic acid decarboxylase (MDC) showing an improved activity in converting 3-methylcrotonic acid into isobutene over the MDC of SEQ ID NO:1, wherein said variant is characterized in that:
it has an amino acid sequence having at least 80 % sequence identity to SEQ ID NO:1; and
it shows one or more amino acid substitutions with an another amino acid residue at a position selected from the group consisting of positions 406, 198, 240, 390, 402, 408 and 449 in the amino acid sequence shown in SEQ ID NO:1 or at a position corresponding to any of these positions.

According to one embodiment, the present invention relates to any of the above-described MDC variants having an amino acid sequence as shown in SEQ ID NO:1 or an amino acid sequence having at least 80% sequence identity to SEQ ID NO:1 in which
(1) an amino acid residue at position 198 in the amino acid sequence shown in SEQ ID NO:1 or at a position corresponding to this position, is substituted with glutamine or tryptophan; and/or
(2) an amino acid residue at position 240 in the amino acid sequence shown in SEQ ID NO:1 or at a position corresponding to this position, is substituted with isoleucine or asparagine; and/or
(3) an amino acid residue at position 390 in the amino acid sequence shown in SEQ ID NO:1 or at a position corresponding to this position, is substituted with serine, aspartic acid, leucine, asparagine or glutamine; and/or
(4) an amino acid residue at position 402 in the amino acid sequence shown in SEQ ID NO:1 or at a position corresponding to this position, is substituted with alanine; and/or
(5) an amino acid residue at position 406 in the amino acid sequence shown in SEQ ID NO:1 or at a position corresponding to this position, is substituted with glycine, alanine or serine; and/or
(6) an amino acid residue at position 408 in the amino acid sequence shown in SEQ ID NO:1 or at a position corresponding to this position, is substituted with isoleucine, alanine, cysteine, leucine, or methionine; and/or
(7) an amino acid residue at position 449 in the amino acid sequence shown in SEQ ID NO:1 or at a position corresponding to this position, is substituted with isoleucine, methionine or valine; and/or
(8) an amino acid residue at position 25 in the amino acid sequence shown in SEQ ID NO:1 or at a position corresponding to this position, is substituted with glutamic acid; and/or
(9) an amino acid residue at position 35 in the amino acid sequence shown in SEQ ID NO:1 or at a position corresponding to this position, is substituted with isoleucine; and/or
(10) an amino acid residue at position 36 in the amino acid sequence shown in SEQ ID NO:1 or at a position corresponding to this position, is substituted with tyrosine; and/or
(11) an amino acid residue at position 74 in the amino acid sequence shown in SEQ ID NO:1 or at a position corresponding to this position, is substituted with valine; and/or
(12) an amino acid residue at position 80 in the amino acid sequence shown in SEQ ID NO:1 or at a position corresponding to this position, is substituted with serine; and/or
(13) an amino acid residue at position 86 in the amino acid sequence shown in SEQ ID NO:1 or at a position corresponding to this position, is substituted with asparagine or tyrosine; and/or
(14) an amino acid residue at position 126 in the amino acid sequence shown in SEQ ID NO:1 or at a position corresponding to this position, is substituted with valine; and/or
(15) an amino acid residue at position 129 in the amino acid sequence shown in SEQ ID NO:1 or at a position corresponding to this position, is substituted with valine; and/or
(16) an amino acid residue at position 166 in the amino acid sequence shown in SEQ ID NO:1 or at a position corresponding to this position, is substituted with aspartic acid; and/or
(17) an amino acid residue at position 196 in the amino acid sequence shown in SEQ ID NO:1 or at a position corresponding to this position, is substituted with isoleucine; and/or
(18) an amino acid residue at position 204 in the amino acid sequence shown in SEQ ID NO:1 or at a position corresponding to this position, is substituted with arginine or serine; and/or
(19) an amino acid residue at position 208 in the amino acid sequence shown in SEQ ID NO:1 or at a position corresponding to this position, is substituted with leucine; and/or
(20) an amino acid residue at position 236 in the amino acid sequence shown in SEQ ID NO:1 or at a position corresponding to this position, is substituted with leucine or valine; and/or
(21) an amino acid residue at position 237 in the amino acid sequence shown in SEQ ID NO:1 or at a position corresponding to this position, is substituted with alanine, aspartic acid, asparagine or proline; and/or
(22) an amino acid residue at position 238 in the amino acid sequence shown in SEQ ID NO:1 or at a position corresponding to this position, is substituted with histidine, arginine, serine or tryptophan; and/or
(23) an amino acid residue at position 298 in the amino acid sequence shown in SEQ ID NO:1 or at a position corresponding to this position, is substituted with serine; and/or
(24) an amino acid residue at position 302 in the amino acid sequence shown in SEQ ID NO:1 or at a position corresponding to this position, is substituted with alanine, cysteine, phenylalanine, lysine, methionine, glutamine, arginine or serine; and/or
(25) an amino acid residue at position 359 in the amino acid sequence shown in SEQ ID NO:1 or at a position corresponding to this position, is substituted with cysteine or valine; and/or
(26) an amino acid residue at position 366 in the amino acid sequence shown in SEQ ID NO:1 or at a position corresponding to this position, is substituted with isoleucine or phenylalanine; and/or
(27) an amino acid residue at position 368 in the amino acid sequence shown in SEQ ID NO:1 or at a position corresponding to this position, is substituted with isoleucine or methionine; and/or
(28) an amino acid residue at position 395 in the amino acid sequence shown in SEQ ID NO:1 or at a position corresponding to this position, is substituted with cysteine, glutamic acid or valine; and/or
(29) an amino acid residue at position 396 in the amino acid sequence shown in SEQ ID NO:1 or at a position corresponding to this position, is substituted with isoleucine; and/or
(30) an amino acid residue at position 407 in the amino acid sequence shown in SEQ ID NO:1 or at a position corresponding to this position, is substituted with alanine, cysteine, isoleucine, lysine, arginine, serine or threonine; and/or
(31) an amino acid residue at position 426 in the amino acid sequence shown in SEQ ID NO:1 or at a position corresponding to this position, is substituted with valine; and/or
(32) an amino acid residue at position 450 in the amino acid sequence shown in SEQ ID NO:1 or at a position corresponding to this position, is substituted with alanine, histidine, methionine, asparagine, glutamine, serine or valine; and/or
(33) an amino acid residue at position 451 in the amino acid sequence shown in SEQ ID NO:1 or at a position corresponding to this position, is substituted with asparagine or serine; and/or
(34) an amino acid residue at position 452 in the amino acid sequence shown in SEQ ID NO:1 or at a position corresponding to this position, is substituted with phenylalanine; and/or
(35) an amino acid residue at position 454 in the amino acid sequence shown in SEQ ID NO:1 or at a position corresponding to this position, is substituted with alanine, aspartic acid, glycine, histidine, lysine, leucine, methionine, glutamine, arginine, serine, threonine, valine or tryptophan; and/or
(36) an amino acid residue at position 455 in the amino acid sequence shown in SEQ ID NO:1 or at a position corresponding to this position, is substituted with alanine, glutamic acid, histidine, lysine, asparagine, serine or valine; and/or
(37) an amino acid residue at position 456 in the amino acid sequence shown in SEQ ID NO:1 or at a position corresponding to this position, is substituted with glutamine; and/or
(38) an amino acid residue at position 457 in the amino acid sequence shown in SEQ ID NO:1 or at a position corresponding to this position, is substituted with lysine; and/or
(39) an amino acid residue at position 458 in the amino acid sequence shown in SEQ ID NO:1 or at a position corresponding to this position, is substituted with glutamic acid, phenylalanine, glycine, leucine, methionine, asparagine, glutamine, arginine, serine or tyrosine; and/or
(40) an amino acid residue at position 462 in the amino acid sequence shown in SEQ ID NO:1 or at a position corresponding to this position, is substituted with cysteine or valine; and/or
(41) an amino acid residue at position 473 in the amino acid sequence shown in SEQ ID NO:1 or at a position corresponding to this position, is substituted with serine.

The invention also relates to variants as defined in (1) to (41) hereinabove, wherein the amino acid residue indicated as substituting the amino acid residue at the position in SEQ ID NO:1 is not that particular amino acid residue but an amino acid residue which is conservative in relation to the indicated substituting amino acid.

Whether an amino acid is conservative with respect to another amino acid can be judged according to means and methods known in the art and as described herein above. One possibility is the PAM 250 matrix; alternatively, the Blosum Family Matrices can be used.

According to another embodiment, the present invention relates to any of the above-described MDC variants, wherein in said variant one or more further amino acid residues at a position selected from the group consisting of 2, 3, 5, 6, 24, 64, 66, 85, 154, 168, 175, 195, 197, 214, 241, 277, 296, 305, 339, 343, 347, 349, 350, 363, 391, 394, 398, 401, 403, 404, 405, 439, 440, 441, 442, 443, 444, 445, 446, 447, 448, 453, 459, 460, 461, 479 and 496 in the amino acid sequence shown in SEQ ID NO:1 or at a position corresponding to any of these positions, are substituted with another amino acid residue at one or more of these positions and wherein said MDC has an improved activity in converting 3-methylcrotonic acid into isobutene over the corresponding MDC from which it is derived.

Thus, the present invention relates in another embodiment to any of the above-described variants wherein said variant is characterized in that it has one or more further amino acid substitutions at a position selected from the group consisting of 2, 3, 5, 6, 24, 64, 66, 85, 154, 168, 175, 195, 197, 214, 241, 277, 296, 305, 339, 343, 347, 349, 350, 363, 391, 394, 398, 401, 403, 404, 405, 439, 440, 441, 442, 443, 444, 445, 446, 447, 448, 453, 459, 460, 461, 479 and 496 in the amino acid sequence shown in SEQ ID NO:1 or at a position corresponding to any of these positions, and wherein said MDC has an improved activity in converting 3-methylcrotonic acid into isobutene over the corresponding MDC from which it is derived.

According to one embodiment, the present invention relates to any of the above-described MDC variants having one or more further amino acid residues at a position selected from the group consisting of 2, 3, 5, 6, 24, 64, 66, 85, 154, 168, 175, 195, 197, 214, 241, 277, 296, 305, 339, 343, 347, 349, 350, 363, 391, 394, 398, 401, 403, 404, 405, 439, 440, 441, 442, 443, 444, 445, 446, 447, 448, 453, 459, 460, 461, 479 and 496 in the amino acid sequence shown in SEQ ID NO:1 or at a position corresponding to any of these positions substituted with another amino acid or having effected an insertion at one or more of these positions and wherein said MDC has an improved activity in converting 3-methylcrotonic acid into isobutene over the corresponding MDC from which it is derived, in which
(42) an amino acid residue at position 85 in the amino acid sequence shown in SEQ ID NO:1 or at a position corresponding to this position, is substituted with leucine; and/or
(43) an amino acid residue at position 241 in the amino acid sequence shown in SEQ ID NO:1 or at a position corresponding to this position, is substituted with aspartic acid, asparagine or valine; and/or
(44) an amino acid residue at position 401 in the amino acid sequence shown in SEQ ID NO:1 or at a position corresponding to this position, is substituted with tyrosine; and/or
(45) an amino acid residue at position 403 in the amino acid sequence shown in SEQ ID NO:1 or at a position corresponding to this position, is substituted with alanine, cysteine, glycine, histidine, isoleucine, asparagine, proline, glutamine, arginine or valine; and/or
(46) an amino acid residue at position 404 in the amino acid sequence shown in SEQ ID NO:1 or at a position corresponding to this position, is substituted with phenylalanine, leucine or methionine; and/or
(47) an amino acid residue at position 405 in the amino acid sequence shown in SEQ ID NO:1 or at a position corresponding to this position, is substituted with histidine, isoleucine, methionine, threonine or valine; and/or
(48) an amino acid residue at position 443 in the amino acid sequence shown in SEQ ID NO:1 or at a position corresponding to this position, is substituted with alanine, aspartic acid, phenylalanine, histidine, leucine, asparagine, arginine, serine, tryptophan or tyrosine; and/or
(49) an amino acid residue at position 444 in the amino acid sequence shown in SEQ ID NO:1 or at a position corresponding to this position, is substituted with alanine, glutamic acid, phenylalanine, histidine, isoleucine, leucine, glutamine or threonine; and/or
(50) an amino acid residue at position 445 in the amino acid sequence shown in SEQ ID NO:1 or at a position corresponding to this position, is substituted with leucine, alanine, cysteine, serine or threonine; and/or
(51) an amino acid residue at position 446 in the amino acid sequence shown in SEQ ID NO:1 or at a position corresponding to this position, is substituted with alanine, cysteine, phenylalanine, isoleucine, methionine, asparagine, serine or valine; and/or
(52) an amino acid residue at position 448 in the amino acid sequence shown in SEQ ID NO:1 or at a position corresponding to this position, substituted with phenylalanine, tryptophan or tyrosine; and/or
(53) an amino acid residue at position 2 in the amino acid sequence shown in SEQ ID NO:1 or at a position corresponding to this position, is substituted with asparagine; and/or
(54) an amino acid residue at position 3 in the amino acid sequence shown in SEQ ID NO:1 or at a position corresponding to this position, is substituted with leucine or methionine; and/or
(55) an amino acid residue at position 5 in the amino acid sequence shown in SEQ ID NO:1 or at a position corresponding to this position, is substituted with isoleucine; and/or
(56) an amino acid residue at position 6 in the amino acid sequence shown in SEQ ID NO:1 or at a position corresponding to this position, is substituted with methionine; and/or
(57) an amino acid residue at position 24 in the amino acid sequence shown in SEQ ID NO:1 or at a position corresponding to this position, is substituted with alanine, aspartic acid, leucine or valine; and/or
(58) an amino acid residue at position 64 in the amino acid sequence shown in SEQ ID NO:1 or at a position corresponding to this position, is substituted with histidine; and/or
(59) an amino acid residue at position 66 in the amino acid sequence shown in SEQ ID NO:1 or at a position corresponding to this position, is substituted with histidine; and/or
(60) an amino acid residue at position 154 in the amino acid sequence shown in SEQ ID NO:1 or at a position corresponding to this position, is substituted with aspartic acid; and/or
(61) an amino acid residue at position 168 in the amino acid sequence shown in SEQ ID NO:1 or at a position corresponding to this position, is substituted with valine; and/or
(62) an amino acid residue at position 175 in the amino acid sequence shown in SEQ ID NO:1 or at a position corresponding to this position, is substituted with glutamic acid; and/or
(63) an amino acid residue at position 195 in the amino acid sequence shown in SEQ ID NO:1 or at a position corresponding to this position, is substituted with isoleucine or valine; and/or
(64) an amino acid residue at position 197 in the amino acid sequence shown in SEQ ID NO:1 or at a position corresponding to this position, is substituted with valine; and/or
(65) an amino acid residue at position 214 in the amino acid sequence shown in SEQ ID NO:1 or at a position corresponding to this position, is substituted with threonine; and/or
(66) an amino acid residue at position 277 in the amino acid sequence shown in SEQ ID NO:1 or at a position corresponding to this position, is substituted with tyrosine; and/or
(67) an amino acid residue at position 296 in the amino acid sequence shown in SEQ ID NO:1 or at a position corresponding to this position, is substituted with glutamine; and/or
(68) an amino acid residue at position 305 in the amino acid sequence shown in SEQ ID NO:1 or at a position corresponding to this position, is substituted with isoleucine or threonine; and/or
(69) an amino acid residue at position 339 in the amino acid sequence shown in SEQ ID NO:1 or at a position corresponding to this position, is substituted with histidine; and/or
(70) an amino acid residue at position 343 in the amino acid sequence shown in SEQ ID NO:1 or at a position corresponding to this position, is substituted with serine; and/or
(70) an amino acid residue at position 347 in the amino acid sequence shown in SEQ ID NO:1 or at a position corresponding to this position, is substituted with cysteine; and/or
(72) an amino acid residue at position 349 in the amino acid sequence shown in SEQ ID NO:1 or at a position corresponding to this position, is substituted with methionine or serine; and/or
(73) an amino acid residue at position 350 in the amino acid sequence shown in SEQ ID NO:1 or at a position corresponding to this position, is substituted with serine; and/or
(74) an amino acid residue at position 363 in the amino acid sequence shown in SEQ ID NO:1 or at a position corresponding to this position, is substituted with glycine; and/or
(75) an amino acid residue at position 391 in the amino acid sequence shown in SEQ ID NO:1 or at a position corresponding to this position, is substituted with aspartic acid or tryptophan; and/or
(76) an amino acid residue at position 394 in the amino acid sequence shown in SEQ ID NO:1 or at a position corresponding to this position, is substituted with cysteine, aspartic acid, glutamine or threonine; and/or
(77) an amino acid residue at position 398 in the amino acid sequence shown in SEQ ID NO:1 or at a position corresponding to this position, is substituted with cysteine or arginine; and/or
(78) an amino acid residue at position 439 in the amino acid sequence shown in SEQ ID NO:1 or at a position corresponding to this position, is substituted with cysteine; and/or
(79) an amino acid residue at position 440 in the amino acid sequence shown in SEQ ID NO:1 or at a position corresponding to this position, is substituted with leucine; and/or
(80) an amino acid residue at position 441 in the amino acid sequence shown in SEQ ID NO:1 or at a position corresponding to this position, is substituted with glycine, histidine, lysine, asparagine, proline, arginine, serine or threonine; and/or
(81) an amino acid residue at position 442 in the amino acid sequence shown in SEQ ID NO:1 or at a position corresponding to this position, is substituted with alanine, cysteine, glutamic acid, glycine, histidine, leucine, methionine, asparagine, glutamine, arginine, serine or threonine; and/or
(82) an amino acid residue at position 447 in the amino acid sequence shown in SEQ ID NO:1 or at a position corresponding to this position, is substituted with glutamine; and/or
(83) an amino acid residue at position 453 in the amino acid sequence shown in SEQ ID NO:1 or at a position corresponding to this position, is substituted with cysteine, glycine, leucine, asparagine, proline, arginine or serine; and/or
(84) an amino acid residue at position 459 in the amino acid sequence shown in SEQ ID NO:1 or at a position corresponding to this position, is substituted with aspartic acid, glutamic acid, glycine, methionine, asparagine, glutamine, arginine, threonine or tyrosine; and/or
(85) an amino acid residue at position 460 in the amino acid sequence shown in SEQ ID NO:1 or at a position corresponding to this position, is substituted with glutamic acid, phenylalanine, glycine, leucine, methionine, asparagine, glutamine or arginine; and/or
(86) an amino acid residue at position 461 in the amino acid sequence shown in SEQ ID NO:1 or at a position corresponding to this position, is substituted with alanine, glycine, leucine, methionine, glutamine, serine or valine; and/or
(87) an amino acid residue at position 479 in the amino acid sequence shown in SEQ ID NO:1 or at a position corresponding to this position, is substituted with cysteine, isoleucine, leucine, methionine, serine, threonine or valine; and/or
(88) an amino acid residue at position 496 in the amino acid sequence shown in SEQ ID NO:1 or at a position corresponding to this position, is substituted with leucine.

The invention also relates to variants as defined in (42) to (88) hereinabove, wherein the amino acid residue indicated as substituting the amino acid residue at the position in SEQ ID NO:1 is not that particular amino acid residue but an amino acid residue which is conservative in relation to the indicated substituting amino acid.

Whether an amino acid is conservative with respect to another amino acid can be judged according to means and methods known in the art and as described herein above. One possibility is the PAM 250 matrix; alternatively, the Blosum Family Matrices can be used.

The present invention relates in another preferred embodiment to a variant of a 3-methylcrotonic acid decarboxylase (MDC) showing an improved activity in converting 3-methylcrotonic acid into isobutene over the MDC of SEQ ID NO:1,
wherein said variant is characterized in that:
it has an amino acid sequence having at least 80 % sequence identity to SEQ ID NO:1; and
it shows one or more amino acid substitutions with an another amino acid residue, deletions or insertions at a position selected from the group consisting of positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462 and 473 in the amino acid sequence shown in SEQ ID NO:1 or at a position corresponding to any of these positions.

As regards the preferred embodiments of the substitutions for these variants, the same applies as described herein above. Thus, in preferred embodiments, the change(s) which are effected in these variants at any of the above position(s) is/are substitution(s) as defined herein above.

The present invention relates in another preferred embodiment to any of the above-described variants having an amino acid sequence having at least 80 % sequence identity to SEQ ID NO:1, wherein said variant is characterized in that it has one or more further amino acid substitutions at a position selected from the group consisting of 2, 3, 5, 6, 24, 64, 66, 85, 154, 168, 175, 195, 197, 214, 241, 277, 296, 305, 339, 343, 347, 349, 350, 363, 391, 394, 398, 401, 403, 404, 405, 439, 440, 441, 442, 443, 444, 445, 446, 447, 448, 453, 459, 460, 461, 479 and 496 in the amino acid sequence shown in SEQ ID NO:1 or at a position corresponding to any of these positions, and wherein said MDC has an improved activity in converting 3-methylcrotonic acid into isobutene over the corresponding MDC from which it is derived.

As regards the preferred embodiments of the substitutions for these variants, the same applies as described herein above. Thus, in preferred embodiments, the change(s) which are effected in these variants at any of the above position(s) is/are substitution(s) as defined herein above.

The present invention relates in another preferred embodiment to a variant of a 3-methylcrotonic acid decarboxylase (MDC) showing an improved activity in converting 3-methylcrotonic acid into isobutene over the MDC of SEQ ID NO:1,
wherein said variant is characterized in that:
it has an amino acid sequence having at least 80 % sequence identity to SEQ ID NO:1; and
it shows one or more amino acid substitutions with an another amino acid residue at a position selected from the group consisting of positions 25, 74, 80, 166, 196, 198, 204, 208, 236, 237, 238, 240, 302, 366, 368, 390, 395, 402, 406, 407, 408, 426, 449, 450, 451, 452, 456, 457, 462, and 473 in the amino acid sequence shown in SEQ ID NO:1 or at a position corresponding to any of these positions.

The present invention relates in another preferred embodiment to any of the above-described variants having an amino acid sequence having at least 80 % sequence identity to SEQ ID NO:1, wherein said variant is characterized in that it has one or more further amino acid substitutions at a position selected from the group consisting of 2, 3, 5, 6, 24, 35, 36, 64, 66, 85, 86, 126, 129, 154, 168, 175, 195, 197, 214, 241, 277, 296, 298, 305, 339, 343, 347, 349, 350, 359, 363, 391, 394, 396, 398, 401, 403, 404, 405, 439, 440, 441, 442, 443, 444, 445, 446, 447, 448, 453, 454, 455, 458, 459, 460, 461, 479, and 496 in the amino acid sequence shown in SEQ ID NO:1 or at a position corresponding to any of these positions, and wherein said MDC has an improved activity in converting 3-methylcrotonic acid into isobutene over the corresponding MDC from which it is derived.

As regards the preferred embodiments of the substitutions for these variants, the same applies as described herein above. Thus, in preferred embodiments, the change(s) which are effected in these variants at any of the above position(s) is/are substitution(s) as defined herein above.

The present invention relates in another preferred embodiment to a variant of a 3-methylcrotonic acid decarboxylase (MDC) showing an improved activity in converting 3-methylcrotonic acid into isobutene over the MDC of SEQ ID NO:1,
wherein said variant is characterized in that:
it has an amino acid sequence having at least 80% sequence identity to SEQ ID NO:1; and
it shows one or more amino acid substitutions with an another amino acid residue at a position selected from the group consisting of positions 25, 74, 80, 166, 196, 198, 204, 208, 236, 237, 238, 366, 368, 390, 395, 402, 406, 408, 426, 449, 450, 451, 452, 456, 457, 462, and 473 in the amino acid sequence shown in SEQ ID NO:1 or at a position corresponding to any of these positions.

The present invention relates in another preferred embodiment to any of the above-described variants having an amino acid sequence having at least 80 % sequence identity to SEQ ID NO:1, wherein said variant is characterized in that it has one or more further amino acid substitutions at a position selected from the group consisting of 2, 3, 5, 6, 24, 35, 36, 64, 66, 85, 86, 126, 129, 154, 168, 175, 195, 197, 214, 240, 241, 277, 296, 298, 302, 305, 339, 343, 347, 349, 350, 359, 363, 391, 394, 396, 398, 401, 403, 404, 405, 407, 439, 440, 441, 442, 443, 444, 445, 446, 447, 448, 453, 454, 455, 458, 459, 460, 461, 479, and 496 in the amino acid sequence shown in SEQ ID NO:1 or at a position corresponding to any of these positions, and wherein said MDC has an improved activity in converting 3-methylcrotonic acid into isobutene over the corresponding MDC from which it is derived.

As regards the preferred embodiments of the substitutions for these variants, the same applies as described herein above. Thus, in preferred embodiments, the change(s) which are effected in these variants at any of the above position(s) is/are substitution(s) as defined herein above.

The present invention relates in another preferred embodiment to a variant of a 3-methylcrotonic acid decarboxylase (MDC) showing an improved activity in converting 3-methylcrotonic acid into isobutene over the MDC of SEQ ID NO:1,
wherein said variant is characterized in that:
it has an amino acid sequence having at least 80 % sequence identity to SEQ ID NO:1; and
it shows one or more amino acid substitutions with an another amino acid residueat a position selected from the group consisting of positions 25, 74, 80, 166, 196, 198, 204, 208, 238, 366, 390, 402, 406, 408, 426, 449, 451, 452, 456, 457, 462, and 473 in the amino acid sequence shown in SEQ ID NO:1 or at a position corresponding to any of these positions.

The present invention relates in another preferred embodiment to any of the above-described variants having an amino acid sequence having at least 80 % sequence identity to SEQ ID NO:1, wherein said variant is characterized in that it has one or more further amino acid substitutions at a position selected from the group consisting of 2, 3, 5, 6, 24, 35, 36, 64, 66, 85, 86, 126, 129, 154, 168, 175, 195, 197, 214, 236, 237, 240, 241, 277, 296, 298, 302, 305, 339, 343, 347, 349, 350, 359, 363, 368, 391, 394, 395, 396, 398, 401, 403, 404, 405, 407, 439, 440, 441, 442, 443, 444, 445, 446, 447, 448, 450, 453, 454, 455, 458, 459, 460, 461, 479, and 496 in the amino acid sequence shown in SEQ ID NO:1 or at a position corresponding to any of these positions, and wherein said MDC has an improved activity in converting 3-methylcrotonic acid into isobutene over the corresponding MDC from which it is derived.

As regards the preferred embodiments of the substitutions for these variants, the same applies as described herein above. Thus, in preferred embodiments, the change(s) which are effected in these variants at any of the above position(s) is/are substitution(s) as defined herein above.

The present invention relates in another preferred embodiment to a variant of a 3-methylcrotonic acid decarboxylase (MDC) showing an improved activity in converting 3-methylcrotonic acid into isobutene over the MDC of SEQ ID NO:1,
wherein said variant is characterized in that:
it has an amino acid sequence having at least 80 % sequence identity to SEQ ID NO:1; and
it shows one or more amino acid substitutions with an another amino acid residue at a position selected from the group consisting of positions 25, 74, 80, 196, 366, 402, 408, 426, 449, 451, 452, 456, 457, and 473 in the amino acid sequence shown in SEQ ID NO:1 or at a position corresponding to any of these positions.

The present invention relates in another preferred embodiment to any of the above-described variants having an amino acid sequence having at least 80 % sequence identity to SEQ ID NO:1, wherein said variant is characterized in that it has one or more further amino acid substitutions at a position selected from the group consisting of 2, 3, 5, 6, 24, 35, 36, 64, 66, 85, 86, 126, 129, 154, 166, 168, 175, 195, 197, 198, 204, 208, 214, 236, 237, 238, 240, 241, 277, 296, 298, 302, 305, 339, 343, 347, 349, 350, 359, 363, 368, 390, 391, 394, 395, 396, 398, 401, 403, 404, 405, 406, 407, 439, 440, 441, 442, 443, 444, 445, 446, 447, 448, 450, 453, 454, 455, 458, 459, 460, 461, 462, 479, and 496 in the amino acid sequence shown in SEQ ID NO:1 or at a position corresponding to any of these positions, and wherein said MDC has an improved activity in converting 3-methylcrotonic acid into isobutene over the corresponding MDC from which it is derived.

As regards the preferred embodiments of the substitutions for these variants, the same applies as described herein above. Thus, in preferred embodiments, the change(s) which are effected in these variants at any of the above position(s) is/are substitution(s) as defined herein above.

In a preferred embodiment, the MDC variant according to the invention showing an improved activity in converting 3-methylcrotonic acid into isobutene is characterized in that contains at least one substitution wherein the substitution is at position 241 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 277, 296, 305, 339, 343, 347, 349, 350, 363, 391, 394, 398, 401, 403, 404, 405, 439, 440, 441, 442, 443, 444, 445, 446, 447, 448, 453, 459, 460, 461, 479 and 496 in the amino acid sequence shown in SEQ ID NO:1.

Preferably, such a variant has the following substitution in the amino acid sequence shown in SEQ ID NO:1 or at positions corresponding to this position: A241D, A241N, or A241V.

In a preferred embodiment, the MDC variant according to the invention showing an improved activity in converting 3-methylcrotonic acid into isobutene is characterized in that contains at least one substitution wherein the substitution is at position 359 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 406, 25, 35, 36, 74, 80, 86, 126, 129, 166, 196, 198, 204, 208, 236, 237, 238, 240, 298, 302, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 241, 277, 296, 305, 339, 343, 347, 349, 350, 363, 391, 394, 398, 401, 403, 404, 405, 439, 440, 441, 442, 443, 444, 445, 446, 447, 448, 453, 459, 460, 461, 479 and 496 in the amino acid sequence shown in SEQ ID NO:1.

Preferably, such a variant has the following substitution in the amino acid sequence shown in SEQ ID NO:1 or at positions corresponding to this position: A359C.

In a preferred embodiment, the MDC variant according to the invention showing an improved activity in converting 3-methylcrotonic acid into isobutene is characterized in that contains at least one substitution wherein the substitution is at position 404 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 241, 277, 296, 305, 339, 343, 347, 349, 350, 363, 391, 394, 398, 401, 403, 405, 439, 440, 441, 442, 443, 444, 445, 446, 447, 448, 453, 459, 460, 461, 479 and 496 in the amino acid sequence shown in SEQ ID NO:1.

Preferably, such a variant has the following substitution in the amino acid sequence shown in SEQ ID NO:1 or at positions corresponding to this position: C404F or C404L. In a preferred embodiment, the MDC variant according to the invention showing an improved activity in converting 3-methylcrotonic acid into isobutene is characterized in that contains at least one substitution wherein the substitution is at position 448 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 241, 277, 296, 305, 339, 343, 347, 349, 350, 363, 391, 394, 398, 401, 403, 404, 405, 439, 440, 441, 442, 443, 444, 445, 446, 447, 453, 459, 460, 461, 479 and 496 in the amino acid sequence shown in SEQ ID NO:1.

Preferably, such a variant has the following substitution in the amino acid sequence shown in SEQ ID NO:1 or at positions corresponding to this position: L448F, L448W or L448Y.

In a preferred embodiment, the MDC variant according to the invention showing an improved activity in converting 3-methylcrotonic acid into isobutene is characterized in that contains at least one substitution wherein the substitution is at position 406 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 241, 277, 296, 305, 339, 343, 347, 349, 350, 363, 391, 394, 398, 401, 403, 404, 405, 439, 440, 441, 442, 443, 444, 445, 446, 447, 448, 453, 459, 460, 461, 479 and 496 in the amino acid sequence shown in SEQ ID NO:1.

Preferably, such a variant has the following substitution in the amino acid sequence shown in SEQ ID NO:1 or at positions corresponding to this position: P406A.

In a preferred embodiment, the MDC variant according to the invention showing an improved activity in converting 3-methylcrotonic acid into isobutene is characterized in that contains at least two substitutions wherein one substitution is at position 390 in the amino acid sequence shown in SEQ ID NO:1 or at a position corresponding to this position and another substitution is at position 448 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 406, 25, 35, 36, 74, 80, 86, 126, 129, 166, 196, 198, 204, 208, 236, 237, 238, 240, 298, 302, 359, 366, 368, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 241, 277, 296, 305, 339, 343, 347, 349, 350, 363, 391, 394, 398, 401, 403, 404, 405, 439, 440, 441, 442, 443, 444, 445, 446, 447, 453, 459, 460, 461, 479 and 496 in the amino acid sequence shown in SEQ ID NO:1.

Preferably, such a variant has the following substitutions in the amino acid sequence shown in SEQ ID NO:1 or at positions corresponding to these positions: R390S-L448W or R390S-L448Y.

In a preferred embodiment, the MDC variant according to the invention showing an improved activity in converting 3-methylcrotonic acid into isobutene is characterized in that contains at least two substitutions wherein one substitution is at position 241 in the amino acid sequence shown in SEQ ID NO:1 or at a position corresponding to this position and another substitution is at position 404 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 277, 296, 305, 339, 343, 347, 349, 350, 363, 391, 394, 398, 401, 403, 405, 439, 440, 441, 442, 443, 444, 445, 446, 447, 448, 453, 459, 460, 461, 479 and 496 in the amino acid sequence shown in SEQ ID NO:1.

Preferably, such a variant has the following substitutions in the amino acid sequence shown in SEQ ID NO:1 or at positions corresponding to these positions: A241D-C404F, A241D-C404L, A241N-C404F, or A241N-C404L.

In a preferred embodiment, the MDC variant according to the invention showing an improved activity in converting 3-methylcrotonic acid into isobutene is characterized in that contains at least two substitutions wherein one substitution is at position 241 in the amino acid sequence shown in SEQ ID NO:1 or at a position corresponding to this position and another substitution is at position 448 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 277, 296, 305, 339, 343, 347, 349, 350, 363, 391, 394, 398, 401, 403, 404, 405, 439, 440, 441, 442, 443, 444, 445, 446, 447, 453, 459, 460, 461, 479 and 496 in the amino acid sequence shown in SEQ ID NO:1.

Preferably, such a variant has the following substitutions in the amino acid sequence shown in SEQ ID NO:1 or at positions corresponding to these positions: A241D-L448F, A241D-L448W, A241D-L448Y, A241N-L448F, A241N-L448W, or A241N-L448Y.

In a preferred embodiment, the MDC variant according to the invention showing an improved activity in converting 3-methylcrotonic acid into isobutene is characterized in that contains at least two substitutions wherein one substitution is at position 241 in the amino acid sequence shown in SEQ ID NO:1 or at a position corresponding to this position and another substitution is at position 406 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 277, 296, 305, 339, 343, 347, 349, 350, 363, 391, 394, 398, 401, 403, 404, 405, 439, 440, 441, 442, 443, 444, 445, 446, 447, 448, 453, 459, 460, 461, 479 and 496 in the amino acid sequence shown in SEQ ID NO:1.

Preferably, such a variant has the following substitutions in the amino acid sequence shown in SEQ ID NO:1 or at positions corresponding to these positions: A241D-P406A or A241N-P406A.

In a preferred embodiment, the MDC variant according to the invention showing an improved activity in converting 3-methylcrotonic acid into isobutene is characterized in that contains at least two deletions wherein one substitution is at position 404 in the amino acid sequence shown in SEQ ID NO:1 or at a position corresponding to this position and another substitution is at position 448 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 241, 277, 296, 305, 339, 343, 347, 349, 350, 363, 391, 394, 398, 401, 403, 405, 439, 440, 441, 442, 443, 444, 445, 446, 447, 453, 459, 460, 461, 479 and 496 in the amino acid sequence shown in SEQ ID NO:1.

Preferably, such a variant has the following substitutions in the amino acid sequence shown in SEQ ID NO:1 or at positions corresponding to these positions: C404L-L448F, C404L-L448W, C404M-L448F, C404M-L448W, C404F-L448F, C404F-L448W, C404F-L448Y or C404L-L448Y.

In a preferred embodiment, the MDC variant according to the invention showing an improved activity in converting 3-methylcrotonic acid into isobutene is characterized in that contains at least two substitutions wherein one substitution is at position 404 in the amino acid sequence shown in SEQ ID NO:1 or at a position corresponding to this position and another substitution is at position 406 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 241, 277, 296, 305, 339, 343, 347, 349, 350, 363, 391, 394, 398, 401, 403, 405, 439, 440, 441, 442, 443, 444, 445, 446, 447, 448, 453, 459, 460, 461, 479 and 496 in the amino acid sequence shown in SEQ ID NO:1.

Preferably, such a variant has the following substitutions in the amino acid sequence shown in SEQ ID NO:1 or at positions corresponding to these positions: C404L-P406A or C404F-P406A.

In a preferred embodiment, the MDC variant according to the invention showing an improved activity in converting 3-methylcrotonic acid into isobutene is characterized in that contains at least two deletions wherein one substitution is at position 406 in the amino acid sequence shown in SEQ ID NO:1 or at a position corresponding to this position and another substitution is at position 448 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 241, 277, 296, 305, 339, 343, 347, 349, 350, 363, 391, 394, 398, 401, 403, 404, 405, 439, 440, 441, 442, 443, 444, 445, 446, 447, 453, 459, 460, 461, 479 and 496 in the amino acid sequence shown in SEQ ID NO:1.

Preferably, such a variant has the following substitutions in the amino acid sequence shown in SEQ ID NO:1 or at positions corresponding to these positions: P406A-L448F, P406A-L448W or P406A-L448Y.

In a preferred embodiment, the MDC variant according to the invention showing an improved activity in converting 3-methylcrotonic acid into isobutene is characterized in that contains at least three substitutions wherein one substitution is at position 241 in the amino acid sequence shown in SEQ ID NO:1 or at a position corresponding to this position, another substitution is at position 404 in the amino acid sequence shown in SEQ ID NO:1 or at a position corresponding to this position and another substitution is at position 448 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 277, 296, 305, 339, 343, 347, 349, 350, 363, 391, 394, 398, 401, 403, 405, 439, 440, 441, 442, 443, 444, 445, 446, 447, 453, 459, 460, 461, 479 and 496 in the amino acid sequence shown in SEQ ID NO:1.

Preferably, such a variant has the following substitutions in the amino acid sequence shown in SEQ ID NO:1 or at positions corresponding to these positions: A241D-C404F-L448F, A241D-C404F-L448Y, A241D-C404L-L448F, A241D-C404L-L448W, A241D-C404L-L448Y, A241N-C404F-L448F, A241N-C404F-L448W, A241N-C404F-L448Y, A241N-C404L-L448F, A241N-C404L-L448W, A241N-C404L-L448Y, A241D-C404F-L448W, A241N-C404M-L448W or A241D-C404M-L448W.

In a preferred embodiment, the MDC variant according to the invention showing an improved activity in converting 3-methylcrotonic acid into isobutene is characterized in that contains at least three substitutions wherein one substitution is at position 241 in the amino acid sequence shown in SEQ ID NO:1 or at a position corresponding to this position, another substitution is at position 404 in the amino acid sequence shown in SEQ ID NO:1 or at a position corresponding to this position and another substitution is at position 406 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 277, 296, 305, 339, 343, 347, 349, 350, 363, 391, 394, 398, 401, 403, 405, 439, 440, 441, 442, 443, 444, 445, 446, 447, 448, 453, 459, 460, 461, 479 and 496 in the amino acid sequence shown in SEQ ID NO:1.

Preferably, such a variant has the following substitutions in the amino acid sequence shown in SEQ ID NO:1 or at positions corresponding to these positions: A241D-C404F-P406A, A241D-C404L-P406A, A241N-C404L-P406A or A241N-C404F-P406A.

In a preferred embodiment, the MDC variant according to the invention showing an improved activity in converting 3-methylcrotonic acid into isobutene is characterized in that contains at least three substitutions wherein one substitution is at position 241 in the amino acid sequence shown in SEQ ID NO:1 or at a position corresponding to this position, another substitution is at position 406 in the amino acid sequence shown in SEQ ID NO:1 or at a position corresponding to this position and another substitution is at position 448 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 277, 296, 305, 339, 343, 347, 349, 350, 363, 391, 394, 398, 401, 403, 404, 405, 439, 440, 441, 442, 443, 444, 445, 446, 447, 453, 459, 460, 461, 479 and 496 in the amino acid sequence shown in SEQ ID NO:1.

Preferably, such a variant has the following substitutions in the amino acid sequence shown in SEQ ID NO:1 or at positions corresponding to these positions: A241D-P406A-L448F, A241D-P406A-L448Y, A241N-P406A-L448F, A241N-P406A-L448W, A241N-P406A-L448Y or A241D-P406A-L448W.

In a preferred embodiment, the MDC variant according to the invention showing an improved activity in converting 3-methylcrotonic acid into isobutene is characterized in that contains at least three substitutions wherein one substitution is at position 404 in the amino acid sequence shown in SEQ ID NO:1 or at a position corresponding to this position, another substitution is at position 406 in the amino acid sequence shown in SEQ ID NO:1 or at a position corresponding to this position and another substitution is at position 448 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 241, 277, 296, 305, 339, 343, 347, 349, 350, 363, 391, 394, 398, 401, 403, 405, 439, 440, 441, 442, 443, 444, 445, 446, 447, 453, 459, 460, 461, 479 and 496 in the amino acid sequence shown in SEQ ID NO:1.

Preferably, such a variant has the following substitutions in the amino acid sequence shown in SEQ ID NO:1 or at positions corresponding to these positions: C404F-P406A-L448F, C404F-P406A-L448W, C404F-P406A-L448Y, C404L-P406A-L448F, C404L-P406A-L448Y, C404M-P406A-L448W, C404M-P406S-L448W or C404L-P406A-L448W.

In a preferred embodiment, the MDC variant according to the invention showing an improved activity in converting 3-methylcrotonic acid into isobutene is characterized in that contains at least three substitutions wherein one substitution is at position 404 in the amino acid sequence shown in SEQ ID NO:1 or at a position corresponding to this position, another substitution is at position 446 in the amino acid sequence shown in SEQ ID NO:1 or at a position corresponding to this position and another substitution is at position 448 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 241, 277, 296, 305, 339, 343, 347, 349, 350, 363, 391, 394, 398, 401, 403, 405, 439, 440, 441, 442, 443, 444, 445, 447, 453, 459, 460, 461, 479 and 496 in the amino acid sequence shown in SEQ ID NO:1.

Preferably, such a variant has the following substitutions in the amino acid sequence shown in SEQ ID NO:1 or at positions corresponding to these positions: C404F-L446I-L448W.

In a preferred embodiment, the MDC variant according to the invention showing an improved activity in converting 3-methylcrotonic acid into isobutene is characterized in that contains at least three substitutions wherein one substitution is at position 404 in the amino acid sequence shown in SEQ ID NO:1 or at a position corresponding to this position, another substitution is at position 446 in the amino acid sequence shown in SEQ ID NO:1 or at a position corresponding to this position and another substitution is at position 448 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 241, 277, 296, 305, 339, 343, 347, 349, 350, 363, 391, 394, 398, 401, 403, 405, 439, 440, 441, 442, 443, 444, 445, 447, 453, 459, 460, 461, 479 and 496 in the amino acid sequence shown in SEQ ID NO:1.

Preferably, such a variant has the following substitutions in the amino acid sequence shown in SEQ ID NO:1 or at positions corresponding to these positions: C404M-L446N-L448W or C404M-L446I-L448W.

In a preferred embodiment, the MDC variant according to the invention showing an improved activity in converting 3-methylcrotonic acid into isobutene is characterized in that contains at least three substitutions wherein one substitution is at position 240 in the amino acid sequence shown in SEQ ID NO:1 or at a position corresponding to this position, another substitution is at position 404 in the amino acid sequence shown in SEQ ID NO:1 or at a position corresponding to this position and another substitution is at position 448 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 406, 25, 35, 36, 74, 80, 86, 126, 129, 166, 196, 198, 204, 208, 236, 237, 238, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 241, 277, 296, 305, 339, 343, 347, 349, 350, 363, 391, 394, 398, 401, 403, 405, 439, 440, 441, 442, 443, 444, 445, 446, 447, 453, 459, 460, 461, 479 and 496 in the amino acid sequence shown in SEQ ID NO:1.

Preferably, such a variant has the following substitutions in the amino acid sequence shown in SEQ ID NO:1 or at positions corresponding to these positions: V240I-C404M-L448F.

In a preferred embodiment, the MDC variant according to the invention showing an improved activity in converting 3-methylcrotonic acid into isobutene is characterized in that contains at least three substitutions wherein one substitution is at position 404 in the amino acid sequence shown in SEQ ID NO:1 or at a position corresponding to this position, another substitution is at position 448 in the amino acid sequence shown in SEQ ID NO:1 or at a position corresponding to this position and another substitution is at position 450 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 451, 452, 454, 455, 456, 457, 458, 462, 473, 2, 3, 5, 6, 24, 64, 66, 85, 154, 168, 175, 195, 197, 214, 241, 277, 296, 305, 339, 343, 347, 349, 350, 363, 391, 394, 398, 401, 403, 405, 439, 440, 441, 442, 443, 444, 445, 446, 447, 453, 459, 460, 461, 479 and 496 in the amino acid sequence shown in SEQ ID NO:1.

Preferably, such a variant has the following substitutions in the amino acid sequence shown in SEQ ID NO:1 or at positions corresponding to these positions: C404M-L448W-T450A or C404M-L448W-T450H.

In a preferred embodiment, the MDC variant according to the invention showing an improved activity in converting 3-methylcrotonic acid into isobutene is characterized in that contains at least three substitutions wherein one substitution is at position 241 in the amino acid sequence shown in SEQ ID NO:1 or at a position corresponding to this position, another substitution is at position 401 in the amino acid sequence shown in SEQ ID NO:1 or at a position corresponding to this position and another substitution is at position 448 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 277, 296, 305, 339, 343, 347, 349, 350, 363, 391, 394, 398, 403, 404, 405, 439, 440, 441, 442, 443, 444, 445, 446, 447, 453, 459, 460, 461, 479 and 496 in the amino acid sequence shown in SEQ ID NO:1.

Preferably, such a variant has the following substitutions in the amino acid sequence shown in SEQ ID NO:1 or at positions corresponding to these positions: A241D-F401Y-L448W or A241N-F401Y-L448W.

In a preferred embodiment, the MDC variant according to the invention showing an improved activity in converting 3-methylcrotonic acid into isobutene is characterized in that contains at least three substitutions wherein one substitution is at position 241 in the amino acid sequence shown in SEQ ID NO:1 or at a position corresponding to this position, another substitution is at position 444 in the amino acid sequence shown in SEQ ID NO:1 or at a position corresponding to this position and another substitution is at position 448 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 277, 296, 305, 339, 343, 347, 349, 350, 363, 391, 394, 398, 401, 403, 404, 405, 439, 440, 441, 442, 443, 445, 446, 447, 453, 459, 460, 461, 479 and 496 in the amino acid sequence shown in SEQ ID NO:1.

Preferably, such a variant has the following substitutions in the amino acid sequence shown in SEQ ID NO:1 or at positions corresponding to these positions: A241D-P444E-L448W.

In a preferred embodiment, the MDC variant according to the invention showing an improved activity in converting 3-methylcrotonic acid into isobutene is characterized in that contains at least three substitutions wherein one substitution is at position 241 in the amino acid sequence shown in SEQ ID NO:1 or at a position corresponding to this position, another substitution is at position 403 in the amino acid sequence shown in SEQ ID NO:1 or at a position corresponding to this position and another substitution is at position 448 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 277, 296, 305, 339, 343, 347, 349, 350, 363, 391, 394, 398, 401, 404, 405, 439, 440, 441, 442, 443, 444, 445, 446, 447, 453, 459, 460, 461, 479 and 496 in the amino acid sequence shown in SEQ ID NO:1.

Preferably, such a variant has the following substitutions in the amino acid sequence shown in SEQ ID NO:1 or at positions corresponding to these positions: A241D-S403G-L448W, A241N-S403P-L448W, A241D-S403P-L448W or A241N-S403G-L448W.

In a preferred embodiment, the MDC variant according to the invention showing an improved activity in converting 3-methylcrotonic acid into isobutene is characterized in that contains at least three substitutions wherein one substitution is at position 241 in the amino acid sequence shown in SEQ ID NO:1 or at a position corresponding to this position, another substitution is at position 446 in the amino acid sequence shown in SEQ ID NO:1 or at a position corresponding to this position and another substitution is at position 448 in the amino acid sequence shown in SEQ ID NO:1 or at a position corresponding to this position. Preferably, such a variant further has one or more substitutions in comparison to the corresponding sequence from which it is derived and wherein these substitutions occur at one or more of the positions corresponding to positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 277, 296, 305, 339, 343, 347, 349, 350, 363, 391, 394, 398, 401, 403, 404, 405, 439, 440, 441, 442, 443, 444, 445, 447, 453, 459, 460, 461, 479 and 496 in the amino acid sequence shown in SEQ ID NO:1.

Preferably, such a variant has the following substitutions in the amino acid sequence shown in SEQ ID NO:1 or at positions corresponding to these positions: A241N-L446V-L448W.

In other preferred embodiments, the MDC variant according to the invention showing an improved activity in converting 3-methylcrotonic acid into isobutene is characterized in that has multiple mutations. As it is exemplified in the examples further below, variants have been found bearing multiple mutations which exhibit an increase in the reaction rate of the conversion of 3-methylcrotonic acid into isobutene. These variants bearing multiple mutations are summarized in the following:
Accordingly, in a preferred embodiment, the MDC variant according to the invention showing an improved activity in converting 3-methylcrotonic acid into isobutene is characterized in that it comprises substitutions wherein the substitutions are at positions 241, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-C404F-P406A-L448F, A241D-C404F-P406A-L448Y, A241D-C404L-P406A-L448F, A241D-C404L-P406A-L448Y, A241N-C404F-P406A-L448F, A241N-C404F-P406A-L448Y, A241N-C404L-P406A-L448F or A241N-C404L-P406A-L448Y.

In a preferred embodiment, the MDC variant according to the invention showing an improved activity in converting 3-methylcrotonic acid into isobutene is characterized in that it comprises substitutions wherein the substitutions are at positions 404, 406, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: C404M-P406A-L446I-L448W or C404M-P406S-L446I-L448W.

In a preferred embodiment, the MDC variant according to the invention showing an improved activity in converting 3-methylcrotonic acid into isobutene is characterized in that it comprises substitutions wherein the substitutions are at positions 403, 404, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403R-C404M-L446I-L448W.

In a preferred embodiment, the MDC variant according to the invention showing an improved activity in converting 3-methylcrotonic acid into isobutene is characterized in that it comprises substitutions wherein the substitutions are at positions 404, 446, 448, and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: C404M-L446I-L448W-T450A, C404M-L446I-L448W-T450H, C404M-L446I-L448W-T450M, C404M-L446I-L448W-T450N, C404M-L446I-L448W-T450S, C404M-L446N-L448W-T450A or C404M-L446N-L448W-T450H.

In a preferred embodiment, the MDC variant according to the invention showing an improved activity in converting 3-methylcrotonic acid into isobutene is characterized in that it comprises substitutions wherein the substitutions are at positions 404, 406, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: C404M-P406A-L446N-L448W, C404M-P406S-L446N-L448W.

In a preferred embodiment, the MDC variant according to the invention showing an improved activity in converting 3-methylcrotonic acid into isobutene is characterized in that it comprises substitutions wherein the substitutions are at positions 404, 406, 446, 448, and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: C404M-P406A-L446I-L448W-T450A, C404M-P406S-L446I-L448W-T450A, C404M-P406S-L446I-L448W-T450H, C404M-P406A-L446N-L448W-T450A, C404M-P406A-L446N-L448W-T450H, C404M-P406S-L446N-L448W-T450A, C404M-P406S-L446N-L448W-T450H, C404M-P406A-L446I-L448W-T450H.

In a preferred embodiment, the MDC variant according to the invention showing an improved activity in converting 3-methylcrotonic acid into isobutene is characterized in that it comprises substitutions wherein the substitutions are at positions 241, 404, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-C404F-L446I-L448W.

In a preferred embodiment, the MDC variant according to the invention showing an improved activity in converting 3-methylcrotonic acid into isobutene is characterized in that it comprises substitutions wherein the substitutions are at positions 241, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-C404F-P406A-L448W, A241D-C404F-P406S-L448W, or A241D-C404L-P406A-L448W.

In a preferred embodiment, the MDC variant according to the invention showing an improved activity in converting 3-methylcrotonic acid into isobutene is characterized in that it comprises substitutions wherein the substitutions are at positions 241, 404, 406, 443 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-C404L-P406A-G443D-L448W.

In a preferred embodiment, the MDC variant according to the invention showing an improved activity in converting 3-methylcrotonic acid into isobutene is characterized in that it comprises substitutions wherein the substitutions are at positions 241, 404, 406, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-C404L-P406A-L446V-L448W.

In a preferred embodiment, the MDC variant according to the invention showing an improved activity in converting 3-methylcrotonic acid into isobutene is characterized in that it comprises substitutions wherein the substitutions are at positions 241, 404, 406, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-C404L-P406A-L446V-L448W-T450M.

In a preferred embodiment, the MDC variant according to the invention showing an improved activity in converting 3-methylcrotonic acid into isobutene is characterized in that it comprises substitutions wherein the substitutions are at positions 241, 404, 406, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-C404L-P406A-L448W-T450M.

In a preferred embodiment, the MDC variant according to the invention showing an improved activity in converting 3-methylcrotonic acid into isobutene is characterized in that it comprises substitutions wherein the substitutions are at positions 241, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-C404M-P406A-L448W.

In a preferred embodiment, the MDC variant according to the invention showing an improved activity in converting 3-methylcrotonic acid into isobutene is characterized in that it comprises substitutions wherein the substitutions are at positions 241, 401, 404, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-F401Y-C404F-L446I-L448W.

In a preferred embodiment, the MDC variant according to the invention showing an improved activity in converting 3-methylcrotonic acid into isobutene is characterized in that it comprises substitutions wherein the substitutions are at positions 241, 401, 404 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-F401Y-C404F-L448W or A241D-F401Y-C404M-L448W.

In a preferred embodiment, the MDC variant according to the invention showing an improved activity in converting 3-methylcrotonic acid into isobutene is characterized in that it comprises substitutions wherein the substitutions are at positions 241, 401, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-F401Y-C404F-P406A-L448W, A241D-F401Y-C404M-P406A-L448W.

In a preferred embodiment, the MDC variant according to the invention showing an improved activity in converting 3-methylcrotonic acid into isobutene is characterized in that it comprises substitutions wherein the substitutions are at positions 241, 401, 403, 404, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-F401Y-S403G-C404F-L446I-L448W.

In a preferred embodiment, the MDC variant according to the invention showing an improved activity in converting 3-methylcrotonic acid into isobutene is characterized in that it comprises substitutions wherein the substitutions are at positions 241, 401, 403, 404, and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-F401Y-S403G-C404F-L448W or A241D-F401Y-S403G-C404M-L448W.

In a preferred embodiment, the MDC variant according to the invention showing an improved activity in converting 3-methylcrotonic acid into isobutene is characterized in that it comprises substitutions wherein the substitutions are at positions 241, 401, 403, 404, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-F401Y-S403P-C404F-L446I-L448W.

In a preferred embodiment, the MDC variant according to the invention showing an improved activity in converting 3-methylcrotonic acid into isobutene is characterized in that it comprises substitutions wherein the substitutions are at positions 241, 401, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-F401Y-S403P-C404M-P406A-L448W.

In a preferred embodiment, the MDC variant according to the invention showing an improved activity in converting 3-methylcrotonic acid into isobutene is characterized in that it comprises substitutions wherein the substitutions are at positions 241, 401, 403, 404 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-F401Y-S403P-C404F-L448W.

In a preferred embodiment, the MDC variant according to the invention showing an improved activity in converting 3-methylcrotonic acid into isobutene is characterized in that it comprises substitutions wherein the substitutions are at positions 241, 402, 404, 406, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-G402A-C404L-P406A-L446V-L448W.

In a preferred embodiment, the MDC variant according to the invention showing an improved activity in converting 3-methylcrotonic acid into isobutene is characterized in that it comprises substitutions wherein the substitutions are at positions 241, 402, 404, 406, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-G402A-C404L-P406A-L446V-L448W-T450M.

In a preferred embodiment, the MDC variant according to the invention showing an improved activity in converting 3-methylcrotonic acid into isobutene is characterized in that it comprises substitutions wherein the substitutions are at positions 241, 402, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-G402A-C404L-P406A-L448W.

In a preferred embodiment, the MDC variant according to the invention showing an improved activity in converting 3-methylcrotonic acid into isobutene is characterized in that it comprises substitutions wherein the substitutions are at positions 241, 402, 404, 406, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-G402A-C404L-P406A-L448W-T450M.

In a preferred embodiment, the MDC variant according to the invention showing an improved activity in converting 3-methylcrotonic acid into isobutene is characterized in that it comprises substitutions wherein the substitutions are at positions 241, 402, 403, 404, 406, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-G402A-S403C-C404L-P406A-L446V-L448W.

In a preferred embodiment, the MDC variant according to the invention showing an improved activity in converting 3-methylcrotonic acid into isobutene is characterized in that it comprises substitutions wherein the substitutions are at positions 241, 402, 403, 404, 406, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-G402A-S403C-C404L-P406A-L446V-L448W-T450M or A241D-G402A-S403V-C404L-P406A-L446V-L448W-T450M.

In a preferred embodiment, the MDC variant according to the invention showing an improved activity in converting 3-methylcrotonic acid into isobutene is characterized in that it comprises substitutions wherein the substitutions are at positions 241, 402, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-G402A-S403C-C404L-P406A-L448W or A241D-G402A-S403V-C404L-P406A-L448W.

In a preferred embodiment, the MDC variant according to the invention showing an improved activity in converting 3-methylcrotonic acid into isobutene is characterized in that it comprises substitutions wherein the substitutions are at positions 241, 402, 403, 404, 406, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-G402A-S403C-C404L-P406A-L448W-T450M or A241D-G402A-S403V-C404L-P406A-L448W-T450M.

In a preferred embodiment, the MDC variant according to the invention showing an improved activity in converting 3-methylcrotonic acid into isobutene is characterized in that it comprises substitutions wherein the substitutions are at positions 241, 402, 403, 404, 406, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A 241D-G402A-S403V-C404L-P406A-L446V-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 406, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-L446V-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 406, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-L446V-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-L448W, A241D-S403G-C404F-P406A-L448W or A241D-S403G-C404M-P406A-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403P-C404F-L446I-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 406, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403G-C404F-L446I-L448W.

In a preferred embodiment, the MDC variant according to the invention showing an improved activity in converting 3-methylcrotonic acid into isobutene is characterized in that it comprises substitutions wherein the substitutions are at positions 241, 403, 404 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403G-C404F-L448W, A241D-S403P-C404F-L448W or A241D-S403P-C404M-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403P-C404F-P406A-L448W, A241D-S403P-C404M-P406A-L448W or A241D-S403V-C404L-P406A-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 406, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403V-C404L-P406A-L446V-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 406, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403V-C404L-P406A-L446V-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403V-C404L-P406A-L448W-T450M.

In a preferred embodiment, the MDC variant according to the invention showing an improved activity in converting 3-methylcrotonic acid into isobutene is characterized in that it comprises substitutions wherein the substitutions are at positions 241, 404, 405, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241N-C404F-L405H-P406A-L448W.

In a preferred embodiment, the MDC variant according to the invention showing an improved activity in converting 3-methylcrotonic acid into isobutene is characterized in that it comprises substitutions wherein the substitutions are at positions 241, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241N-C404F-P406A-L448W.

In a preferred embodiment, the MDC variant according to the invention showing an improved activity in converting 3-methylcrotonic acid into isobutene is characterized in that it comprises substitutions wherein the substitutions are at positions 241, 404, 406, 446, and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241N-C404L-P406A-L446C-L448W or A241N-C404L-P406A-L446V-L448W.

In a preferred embodiment, the MDC variant according to the invention showing an improved activity in converting 3-methylcrotonic acid into isobutene is characterized in that it comprises substitutions wherein the substitutions are at positions 241, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241N-C404L-P406A-L448W or A241N-C404M-P406A-L448W.

In a preferred embodiment, the MDC variant according to the invention showing an improved activity in converting 3-methylcrotonic acid into isobutene is characterized in that it comprises substitutions wherein the substitutions are at positions 241, 404, 406, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241N-C404L-P406A-L448W-L449I.

In a preferred embodiment, the MDC variant according to the invention showing an improved activity in converting 3-methylcrotonic acid into isobutene is characterized in that it comprises substitutions wherein the substitutions are at positions 241, 404, 406, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241N-C404L-P406A-L448W-T450M or A241N-C404L-P406A-L448W-T450Q.

In a preferred embodiment, the MDC variant according to the invention showing an improved activity in converting 3-methylcrotonic acid into isobutene is characterized in that it comprises substitutions wherein the substitutions are at positions 241, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241N-S403C-C404L-P406A-L448W, A241N-S403N-C404L-P406A-L448W or A241N-S403V-C404L-P406A-L448W.

In a preferred embodiment, the MDC variant according to the invention showing an improved activity in converting 3-methylcrotonic acid into isobutene is characterized in that it comprises substitutions wherein the substitutions are at positions 404, 406, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: C404L-P406A-L446F-L448W, C404L-P406A-L446S-L448W or C404L-P406A-L446V-L448W.

In a preferred embodiment, the MDC variant according to the invention showing an improved activity in converting 3-methylcrotonic acid into isobutene is characterized in that it comprises substitutions wherein the substitutions are at positions 404, 406, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: C404L-P406A-L448W-T450M, C404M-P406A-L448W-T450A, C404M-P406S-L448W-T450A, C404M-P406A-L448W-T450H or C404M-P406S-L448W-T450H.

In a preferred embodiment, the MDC variant according to the invention showing an improved activity in converting 3-methylcrotonic acid into isobutene is characterized in that it comprises substitutions wherein the substitutions are at positions 404, 406, 444 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: C404L-P406A-P444H-L448W.

In a preferred embodiment, the MDC variant according to the invention showing an improved activity in converting 3-methylcrotonic acid into isobutene is characterized in that it comprises substitutions wherein the substitutions are at positions 85, 241, 404, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: P85L-A241N-C404M-L448W-T450M.

In a preferred embodiment, the MDC variant according to the invention showing an improved activity in converting 3-methylcrotonic acid into isobutene is characterized in that it comprises substitutions wherein the substitutions are at positions 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403V-C404L-P406A-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-L448W, A241D-S403C-C404L-P406A-G443H-L448W or A241D-S403C-C404L-P406A-G443N-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 406, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-L448W-L449I.

In a preferred embodiment, the MDC variant according to the invention showing an improved activity in converting 3-methylcrotonic acid into isobutene is characterized in that it comprises substitutions wherein the substitutions are at positions 241, 403, 404, 406, 444 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-P444A-L448W, A241D-S403C-C404L-P406A-P444H-L448W or A241D-S403C-C404L-P406A-P444L-L448W.

In a preferred embodiment, the MDC variant according to the invention showing an improved activity in converting 3-methylcrotonic acid into isobutene is characterized in that it comprises substitutions wherein the substitutions are at positions 241, 402, 403, 404, 406, 443 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-G402A-S403C-C404L-P406A-G443H-L448W, A241D-G402A-S403C-C404L-P406A-G443S-L448W.

In a preferred embodiment, the MDC variant according to the invention showing an improved activity in converting 3-methylcrotonic acid into isobutene is characterized in that it comprises substitutions wherein the substitutions are at positions 241, 402, 403, 404, 406, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-G402A-S403C-C404L-P406A-L446M-L448W.

In a preferred embodiment, the MDC variant according to the invention showing an improved activity in converting 3-methylcrotonic acid into isobutene is characterized in that it comprises substitutions wherein the substitutions are at positions 241, 402, 403, 404, 406, 444 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-G402A-S403C-C404L-P406A-P444F-L448W or A241D-G402A-S403C-C404L-P406A-P444H-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 406, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403H-C404L-P406A-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443A-L448W-T450M, A241D-S403C-C404L-P406A-G443F-L448W-T450M, A241D-S403C-C404L-P406A-G443Y-L448W-T450M, A241D-S403V-C404L-P406A-G443A-L448W-T450M, A241D-S403V-C404L-P406A-G443D-L448W-T450M, A241D-S403V-C404L-P406A-G443F-L448W-T450M, A241D-S403V-C404L-P406A-G443N-L448W-T450M or A241D-S403V-C404L-P406A-G443S-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 444, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-P444A-L448W-T450M, A241D-S403C-C404L-P406A-P444F-L448W-T450M and A241D-S403C-C404L-P406A-P444T-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403V-C404L-P406A-G443D-L446V-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403V-C404L-P406A-G443D-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 406, 445, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403V-C404L-P406A-V445L-L448W-T450M.

In a preferred embodiment, the MDC variant according to the invention showing an improved activity in converting 3-methylcrotonic acid into isobutene is characterized in that it comprises substitutions wherein the substitutions are at positions 241, 404, 406, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-C404L-P406A-G443F-L446V-L448W-T450M.

In a preferred embodiment, the MDC variant according to the invention showing an improved activity in converting 3-methylcrotonic acid into isobutene is characterized in that it comprises substitutions wherein the substitutions are at positions 241, 404, 406, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-C404L-P406A-L446V-L448W-T450A.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443A-L446V-L448W-T450M, A241D-S403C-C404L-P406A-G443S-L446V-L448W-T450M, A241D-S403C-C404L-P406A-G443W-L446V-L448W-T450M, A241D-S403C-C404L-P406A-G443Y-L446V-L448W-T450M, A241D-S403V-C404L-P406A-G443D-L446V-L448W-T450M, A241D-S403V-C404L-P406A-G443F-L446V-L448W-T450M or A241D-S403V-C404L-P406A-G443Y-L446V-L448W-T450M.

In a preferred embodiment, the MDC variant according to the invention showing an improved activity in converting 3-methylcrotonic acid into isobutene is characterized in that it comprises substitutions wherein the substitutions are at positions 198, 241, 403, 404, 406, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: H198Q-A241D-S403C-C404L-P406A-G443F-L446V-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 444, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-P444H-L446V-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 408, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-V408I-L446V-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-L446M-L448W-T450M, A241D-S403V-C404L-P406A-L446V-L448W-T450H, A241D-S403A-C404L-P406A-L446V-L448W-T450M, A241D-S403G-C404L-P406A-L446V-L448W-T450M, A241D-S403V-C404L-P406A-L446A-L448W-T450M or A241D-S403V-C404L-P406A-L446M-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 408, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403V-C404L-P406A-V408I-L446V-L448W-T450M.

In a preferred embodiment, the MDC variant according to the invention showing an improved activity in converting 3-methylcrotonic acid into isobutene is characterized in that it comprises substitutions wherein the substitutions are at positions 404, 406, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: C404M-P406A-G443W-L446I-L448W-T450H, C404M-P406A-G443Y-L446I-L448W-T450H.

In a preferred embodiment, the MDC variant according to the invention showing an improved activity in converting 3-methylcrotonic acid into isobutene is characterized in that it comprises substitutions wherein the substitutions are at positions 404, 405, 406, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: C404M-L405I-P406A-L446I-L448W-T450H.

In a preferred embodiment, the MDC variant according to the invention showing an improved activity in converting 3-methylcrotonic acid into isobutene is characterized in that it comprises substitutions wherein the substitutions are at positions 197, 404, 406, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-C404M-P406A-L446I-L448W-T450H.

In a preferred embodiment, the MDC variant according to the invention showing an improved activity in converting 3-methylcrotonic acid into isobutene is characterized in that it comprises substitutions wherein the substitutions are at positions 404, 406, 407, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: C404M-P406A-Q407K-L446I-L448W-T450H.

In a preferred embodiment, the MDC variant according to the invention showing an improved activity in converting 3-methylcrotonic acid into isobutene is characterized in that it comprises substitutions wherein the substitutions are at positions 403, 404, 406, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406A-L446I-L448W-T450H or S403P-C404M-P406A-L446I-L448W-T450H.

In a preferred embodiment, the MDC variant according to the invention showing an improved activity in converting 3-methylcrotonic acid into isobutene is characterized in that it comprises substitutions wherein the substitutions are at positions 404, 405, 406, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: C404M-L405I-P406A-L448W-T450H.

In a preferred embodiment, the MDC variant according to the invention showing an improved activity in converting 3-methylcrotonic acid into isobutene is characterized in that it comprises substitutions wherein the substitutions are at positions 197, 403, 404, 405, 406, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-S403C-C404M-L405I-P406A-G443Y-L448W-T450H.

In a preferred embodiment, the MDC variant according to the invention showing an improved activity in converting 3-methylcrotonic acid into isobutene is characterized in that it comprises substitutions wherein the substitutions are at positions 403, 404, 406, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406A-L448W-T450H.

In a preferred embodiment, the MDC variant according to the invention showing an improved activity in converting 3-methylcrotonic acid into isobutene is characterized in that it comprises substitutions wherein the substitutions are at positions 197, 403, 404, 405, 406, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-S403C-C404M-L405I-P406A-L448W-T450H.

In a preferred embodiment, the MDC variant according to the invention showing an improved activity in converting 3-methylcrotonic acid into isobutene is characterized in that it comprises substitutions wherein the substitutions are at positions 197, 404, 405, 406, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-C404M-L405I-P406A-L446I-L448W-T450H. In a preferred embodiment, the MDC variant according to the invention showing an improved activity in converting 3-methylcrotonic acid into isobutene is characterized in that it comprises substitutions wherein the substitutions are at positions 197, 404, 406, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-C404M-P406A-L448W-T450H.

In a preferred embodiment, the MDC variant according to the invention showing an improved activity in converting 3-methylcrotonic acid into isobutene is characterized in that it comprises substitutions wherein the substitutions are at positions 403, 404, 405, 406, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-L405I-P406A-L448W-T450H.

In a preferred embodiment, the MDC variant according to the invention showing an improved activity in converting 3-methylcrotonic acid into isobutene is characterized in that it comprises substitutions wherein the substitutions are at positions 404, 405, 406, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: C404M-L405I-P406A-G443Y-L446I-L448W-T450H. In a preferred embodiment, the MDC variant according to the invention showing an improved activity in converting 3-methylcrotonic acid into isobutene is characterized in that it comprises substitutions wherein the substitutions are at positions 197, 404, 405, 406, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-C404M-L405I-P406A-L448W-T450H.

In a preferred embodiment, the MDC variant according to the invention showing an improved activity in converting 3-methylcrotonic acid into isobutene is characterized in that it comprises substitutions wherein the substitutions are at positions 197, 403, 404, 406, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-S403C-C404M-P406A-L446I-L448W-T450H.

In a preferred embodiment, the MDC variant according to the invention showing an improved activity in converting 3-methylcrotonic acid into isobutene is characterized in that it comprises substitutions wherein the substitutions are at positions 403, 404, 405, 406, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-L405I-P406A-G443Y-L448W-T450H.

In a preferred embodiment, the MDC variant according to the invention showing an improved activity in converting 3-methylcrotonic acid into isobutene is characterized in that it comprises substitutions wherein the substitutions are at positions 404, 406, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: C404M-P406A-G443Y-L448W-T450H.

In a preferred embodiment, the MDC variant according to the invention showing an improved activity in converting 3-methylcrotonic acid into isobutene is characterized in that it comprises substitutions wherein the substitutions are at positions 403, 404, 406, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406A-G443Y-L446I-L448W-T450H.

In a preferred embodiment, the MDC variant according to the invention showing an improved activity in converting 3-methylcrotonic acid into isobutene is characterized in that it comprises substitutions wherein the substitutions are at positions 403, 404, 406, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406A-G443Y-L448W-T450H.

In a preferred embodiment, the MDC variant according to the invention showing an improved activity in converting 3-methylcrotonic acid into isobutene is characterized in that it comprises substitutions wherein the substitutions are at positions 403, 404, 405, 406, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-L405I-P406A-L446I-L448W-T450H. In a preferred embodiment, the MDC variant according to the invention showing an improved activity in converting 3-methylcrotonic acid into isobutene is characterized in that it comprises substitutions wherein the substitutions are at positions 197, 404, 405, 406, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M 197V -C404M -L 4051-P406A-G443Y -L 4461-L 448W-T450H.

In a preferred embodiment, the MDC variant according to the invention showing an improved activity in converting 3-methylcrotonic acid into isobutene is characterized in that it comprises substitutions wherein the substitutions are at positions 403, 404, 405, 406, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-L405I-P406A-G443Y-L446I-L448W-T450H.

In a preferred embodiment, the MDC variant according to the invention showing an improved activity in converting 3-methylcrotonic acid into isobutene is characterized in that it comprises substitutions wherein the substitutions are at positions 404, 405, 406, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: C404M-L405I-P406A-G443Y-L448W-T450H.

In a preferred embodiment, the MDC variant according to the invention showing an improved activity in converting 3-methylcrotonic acid into isobutene is characterized in that it comprises substitutions wherein the substitutions are at positions 197, 403, 404, 405, 406, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-S403C-C404M-L4051-P406A-L4461-L448W-T450H.

In a preferred embodiment, the MDC variant according to the invention showing an improved activity in converting 3-methylcrotonic acid into isobutene is characterized in that it comprises substitutions wherein the substitutions are at positions 197, 404, 406, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-C404M-P406A-G443Y-L446I-L448W-T450H.

In a preferred embodiment, the MDC variant according to the invention showing an improved activity in converting 3-methylcrotonic acid into isobutene is characterized in that it comprises substitutions wherein the substitutions are at positions 197, 403, 404, 405, 406, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-S403C-C404M-L405I-P406A-G443Y-L446I-L448W-T450H.

In a preferred embodiment, the MDC variant according to the invention showing an improved activity in converting 3-methylcrotonic acid into isobutene is characterized in that it comprises substitutions wherein the substitutions are at positions 197, 403, 404, 406, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-S403C-C404M-P406A-G443Y-L446I-L448W-T450H.

In a preferred embodiment, the MDC variant according to the invention showing an improved activity in converting 3-methylcrotonic acid into isobutene is characterized in that it comprises substitutions wherein the substitutions are at positions 241, 403 404, 406, 443, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-L448W-L449I.

In a preferred embodiment, the MDC variant according to the invention showing an improved activity in converting 3-methylcrotonic acid into isobutene is characterized in that it comprises substitutions wherein the substitutions are at positions 241, 403, 404, 406, 444, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-P444H-L446M-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 444 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-P444A-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 444, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443Y-P444H-L446M-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 408, 444, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-V408I-P444H-L446M-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443Y-L446M-L448W-T450M.

In a preferred embodiment, the MDC variant according to the invention showing an improved activity in converting 3-methylcrotonic acid into isobutene is characterized in that it comprises substitutions wherein the substitutions are at positions 25, 241, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A25E-A241D-S403C-C404L-P406A-L448W.

In a preferred embodiment, the MDC variant according to the invention showing an improved activity in converting 3-methylcrotonic acid into isobutene is characterized in that it comprises substitutions wherein the substitutions are at positions 66, 241, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A66H-A241D-S403C-C404L-P406A-L448W.

In a preferred embodiment, the MDC variant according to the invention showing an improved activity in converting 3-methylcrotonic acid into isobutene is characterized in that it comprises substitutions wherein the substitutions are at positions 80, 241, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A80S-A241D-S403C-C404L-P406A-L448W.

In a preferred embodiment, the MDC variant according to the invention showing an improved activity in converting 3-methylcrotonic acid into isobutene is characterized in that it comprises substitutions wherein the substitutions are at positions 24, 241, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: E24A-A241D-S403C-C404L-P406A-L448W, E24D-A241D-S403C-C404L-P406A-L448W, E24L-A241D-S403C-C404L-P406A-L448W, or E24V-A241D-S403C-C404L-P406A-L448W.

In a preferred embodiment, the MDC variant according to the invention showing an improved activity in converting 3-methylcrotonic acid into isobutene is characterized in that it comprises substitutions wherein the substitutions are at positions 237, 241, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: G237D-A241D-S403C-C404L-P406A-L448W or G237P-A241D-S403C-C404L-P406A-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443A-L448W.

In a preferred embodiment, the MDC variant according to the invention showing an improved activity in converting 3-methylcrotonic acid into isobutene is characterized in that it comprises substitutions wherein the substitutions are at positions 236, 241, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: I236L-A241D-S403C-C404L-P406A-L448W or I236V-A241D-S403C-C404L-P406A-L448W.

In a preferred embodiment, the MDC variant according to the invention showing an improved activity in converting 3-methylcrotonic acid into isobutene is characterized in that it comprises substitutions wherein the substitutions are at positions 241, 296, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-I296Q-S403C-C404L-P406A-L448W.

In a preferred embodiment, the MDC variant according to the invention showing an improved activity in converting 3-methylcrotonic acid into isobutene is characterized in that it comprises substitutions wherein the substitutions are at positions 214, 241, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: K214T-A241D-S403C-C404L-P406A-L448W.

In a preferred embodiment, the MDC variant according to the invention showing an improved activity in converting 3-methylcrotonic acid into isobutene is characterized in that it comprises substitutions wherein the substitutions are at positions 64, 241, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: N64H-A241D-S403C-C404L-P406A-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 406, 448 and 479 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-L448W-P479C or A241D-S403C-C404L-P406A-L448W-P479I or A241D-S403C-C404L-P406A-L448W-P479L or A241D-S403C-C404L-P406A-L448W-P479M or A241D-S403C-C404L-P406A-L448W-P479S or A241D-S403C-C404L-P406A-L448W-P479T or A241D-S403C-C404L-P406A-L448W-P479V.

In a preferred embodiment, the MDC variant according to the invention showing an improved activity in converting 3-methylcrotonic acid into isobutene is characterized in that it comprises substitutions wherein the substitutions are at positions 241, 403, 404, 406, 448 and 496 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-L448W-R496L.

In a preferred embodiment, the MDC variant according to the invention showing an improved activity in converting 3-methylcrotonic acid into isobutene is characterized in that it comprises substitutions wherein the substitutions are at positions 175, 241, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S175E-A241D-S403C-C404L-P406A-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 406, 448 and 473 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-L448W-A473S.

In a preferred embodiment, the MDC variant according to the invention showing an improved activity in converting 3-methylcrotonic acid into isobutene is characterized in that it comprises substitutions wherein the substitutions are at positions 86, 241, 403, 404, 406, and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S86N-A241D-S403C-C404L-P406A-L448W or S86Y-A241D-S403C-C404L-P406A-L448W.

In a preferred embodiment, the MDC variant according to the invention showing an improved activity in converting 3-methylcrotonic acid into isobutene is characterized in that it comprises substitutions wherein the substitutions are at positions 240, 241, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: V240N-A241D-S403C-C404L-P406A-L448W.

In a preferred embodiment, the MDC variant according to the invention showing an improved activity in converting 3-methylcrotonic acid into isobutene is characterized in that it comprises substitutions wherein the substitutions are at positions 240, 241, 403, 404, 406, 448 and 479 in the amino acid sequence shown in SEQ ID NO:1 or at positions corresponding to these positions. Preferably, such a variant has the following substitutions in the amino acid sequence shown in SEQ ID NO:1 or at positions corresponding to these positions: V240N-A241D-S403C-C404L-P406A-L448W-P479V.

In a preferred embodiment, the MDC variant according to the invention showing an improved activity in converting 3-methylcrotonic acid into isobutene is characterized in that it comprises substitutions wherein the substitutions are at positions 66, 214, 237, 241, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A66H-K214T-G237P-A241D-S403C-C404L-P406A-L448W.

In a preferred embodiment, the MDC variant according to the invention showing an improved activity in converting 3-methylcrotonic acid into isobutene is characterized in that it comprises substitutions wherein the substitutions are at positions 214, 237, 241, 296, 403, 404, 406, and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: K214T-G237P-A241D-I296Q-S403C-C404L-P406A-L448W.

In a preferred embodiment, the MDC variant according to the invention showing an improved activity in converting 3-methylcrotonic acid into isobutene is characterized in that it comprises substitutions wherein the substitutions are at positions 214, 237, 241, 296, 403, 404, 406, 448 and 479 in the amino acid sequence shown in SEQ ID NO:1 or at positions corresponding to these positions. Preferably, such a variant has the following substitutions in the amino acid sequence shown in SEQ ID NO:1 or at positions corresponding to these positions: K214T-G237P-A241D-I296Q-S403C-C404L-P406A-L448W-P479V.

In a preferred embodiment, the MDC variant according to the invention showing an improved activity in converting 3-methylcrotonic acid into isobutene is characterized in that it comprises substitutions wherein the substitutions are at positions 214, 241, 403, 404, 406, 448 and 496 in the amino acid sequence shown in SEQ ID NO:1 or at positions corresponding to these positions. Preferably, such a variant has the following substitutions in the amino acid sequence shown in SEQ ID NO:1 or at positions corresponding to these positions: K214T-A241D-S403C-C404L-P406A-L448W-R496L.

In a preferred embodiment, the MDC variant according to the invention showing an improved activity in converting 3-methylcrotonic acid into isobutene is characterized in that it comprises substitutions wherein the substitutions are at positions 240, 241, 403, 404, 406, 448 and 496 in the amino acid sequence shown in SEQ ID NO:1 or at positions corresponding to these positions. Preferably, such a variant has the following substitutions in the amino acid sequence shown in SEQ ID NO:1 or at positions corresponding to these positions: V240N-A241D-S403C-C404L-P406A-L448W-R496L.

In a preferred embodiment, the MDC variant according to the invention showing an improved activity in converting 3-methylcrotonic acid into isobutene is characterized in that it comprises substitutions wherein the substitutions are at positions 214, 237, 240, 241, 296, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: K214T-G237P-V240N-A241D-I296Q-S403C-C404L-P406A-L448W.

In a preferred embodiment, the MDC variant according to the invention showing an improved activity in converting 3-methylcrotonic acid into isobutene is characterized in that it comprises substitutions wherein the substitutions are at positions 66, 241, 403, 404, 406, 448 and 479 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A66H-A241D-S403C-C404L-P406A-L448W-P479V. In a preferred embodiment, the MDC variant according to the invention showing an improved activity in converting 3-methylcrotonic acid into isobutene is characterized in that it comprises substitutions wherein the substitutions are at positions 66, 214, 237, 240, 241, 403, 404, 406, 448 and 479 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A66H-K214T-G237P-V240N-A241D-S403C-C404L-P406A-L448W-P479V.

In a preferred embodiment, the MDC variant according to the invention showing an improved activity in converting 3-methylcrotonic acid into isobutene is characterized in that it comprises substitutions wherein the substitutions are at positions 66, 214, 241, 403, 404, 406, 448 and 479 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A66H-K214T-A241D-S403C-C404L-P406A-L448W-P479V.

In a preferred embodiment, the MDC variant according to the invention showing an improved activity in converting 3-methylcrotonic acid into isobutene is characterized in that it comprises substitutions wherein the substitutions are at positions 237, 241, 296, 403, 404, 406, 448 and 479 in the amino acid sequence shown in SEQ ID NO:1 or at positions corresponding to these positions. Preferably, such a variant has the following substitutions in the amino acid sequence shown in SEQ ID NO:1 or at positions corresponding to these positions: G237P-A241D-I296Q-S403C-C404L-P406A-L448W-P479V.

In a preferred embodiment, the MDC variant according to the invention showing an improved activity in converting 3-methylcrotonic acid into isobutene is characterized in that it comprises substitutions wherein the substitutions are at positions 66, 214, 237, 241, 403, 404, 406, 448 and 479 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A66H-K214T-G237P-A241D-S403C-C404L-P406A-L448W-P479V.

In a preferred embodiment, the MDC variant according to the invention showing an improved activity in converting 3-methylcrotonic acid into isobutene is characterized in that it comprises substitutions wherein the substitutions are at positions 241, 296, 403, 404, 406, 448 and 479 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-I296Q-S403C-C404L-P406A-L448W-P479V.

In a preferred embodiment, the MDC variant according to the invention showing an improved activity in converting 3-methylcrotonic acid into isobutene is characterized in that it comprises substitutions wherein the substitutions are at positions 66, 214, 241, 296, 403, 404, 406, and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A66H-K214T-A241D-I296Q-S403C-C404L-P406A-L448W.

In a preferred embodiment, the MDC variant according to the invention showing an improved activity in converting 3-methylcrotonic acid into isobutene is characterized in that it comprises substitutions wherein the substitutions are at positions 214, 241, 403, 404, 406, 443 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: K214T-A241D-S403C-C404L-P406A-G443N-L448W.

In a preferred embodiment, the MDC variant according to the invention showing an improved activity in converting 3-methylcrotonic acid into isobutene is characterized in that it comprises substitutions wherein the substitutions are at positions 66, 241, 296, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A66H-A241D-I296Q-S403C-C404L-P406A-L448W. In a preferred embodiment, the MDC variant according to the invention showing an improved activity in converting 3-methylcrotonic acid into isobutene is characterized in that it comprises substitutions wherein the substitutions are at positions 214, 241, 296, 403, 404, 406 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: K214T-A241D-I296Q-S403C-C404L-P406A-L448W.

In a preferred embodiment, the MDC variant according to the invention showing an improved activity in converting 3-methylcrotonic acid into isobutene is characterized in that it comprises substitutions wherein the substitutions are at positions 237, 241, 403, 404, 406, 448 and 479 in the amino acid sequence shown in SEQ ID NO:1 or at positions corresponding to these positions. Preferably, such a variant has the following substitutions in the amino acid sequence shown in SEQ ID NO:1 or at positions corresponding to these positions: G237P-A241D-S403C-C404L-P406A-L448W-P479V.

In a preferred embodiment, the MDC variant according to the invention showing an improved activity in converting 3-methylcrotonic acid into isobutene is characterized in that it comprises substitutions wherein the substitutions are at positions 66, 214, 241, 296, 403, 404, 406, 448 and 479 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A66H-K214T-A241D-I296Q-S403C-C404L-P406A-L448W-P479V.

In a preferred embodiment, the MDC variant according to the invention showing an improved activity in converting 3-methylcrotonic acid into isobutene is characterized in that it comprises substitutions wherein the substitutions are at positions 66, 237, 240, 241, 403, 404, 406, 448 and 479 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A66H-G237P-V240N-A241D-S403C-C404L-P406A-L448W-P479V.

In a preferred embodiment, the MDC variant according to the invention showing an improved activity in converting 3-methylcrotonic acid into isobutene is characterized in that it comprises substitutions wherein the substitutions are at positions 66, 237, 241, 403, 404, 406, 448 and 479 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A66H-G237P-A241D-S403C-C404L-P406A-L448W-P479V.

In a preferred embodiment, the MDC variant according to the invention showing an improved activity in converting 3-methylcrotonic acid into isobutene is characterized in that it comprises substitutions wherein the substitutions are at positions 214, 241, 296, 403, 404, 406, 448 and 479 in the amino acid sequence shown in SEQ ID NO:1 or at positions corresponding to these positions. Preferably, such a variant has the following substitutions in the amino acid sequence shown in SEQ ID NO:1 or at positions corresponding to these positions: K214T-A241D-I296Q-S403C-C404L-P406A-L448W-P479V.

In a preferred embodiment, the MDC variant according to the invention showing an improved activity in converting 3-methylcrotonic acid into isobutene is characterized in that it comprises substitutions wherein the substitutions are at positions 66, 241, 296, 403, 404, 406, 448 and 479 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A66H-A241D-I296Q-S403C-C404L-P406A-L448W-P479V.

In a preferred embodiment, the MDC variant according to the invention showing an improved activity in converting 3-methylcrotonic acid into isobutene is characterized in that it comprises substitutions wherein the substitutions are at positions 214, 241, 403, 404, 406, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: K214T-A241D-S403C-C404L-P406A-G443F-L448W-T450M.

In a preferred embodiment, the MDC variant according to the invention showing an improved activity in converting 3-methylcrotonic acid into isobutene is characterized in that it comprises substitutions wherein the substitutions are at positions 214, 237, 241, 403, 404, 406, 448 and 479 in the amino acid sequence shown in SEQ ID NO:1 or at positions corresponding to these positions. Preferably, such a variant has the following substitutions in the amino acid sequence shown in SEQ ID NO:1 or at positions corresponding to these positions: K214T-G237P-A241D-S403C-C404L-P406A-L448W-P479V.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-G443F-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 408, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-V408C-G443F-L448W-T450M or A241D-S403C-C404L-P406A-V408L-G443F-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 445, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443F-V445L-L448W-T450M or A241D-S403C-C404L-P406A-G443F-V445S-L448W-T450M or A241D-S403C-C404L-P406A-G443F-V445T-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 445 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-V445T-L448W.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 443, 444, and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-G443D-P444A-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 405, 406, 443, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-L405I-P406A-G443D-L448W-L449I.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 443 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-G443D-L448W-L449I.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 445, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-V445L-L448W-L449I.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 444, 448, and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443A-P444H-L448W-L449I or A241D-S403C-C404L-P406A-G443F-P444H-L448W-L449I or A241D-S403C-C404L-P406A-G443L-P444H-L448W-L449I or A241D-S403C-C404L-P406A-G443S-P444H-L448W-L449I.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 444, and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-P444H-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-P444H-L448W-L449V.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 241, 404, 405, 406, 443, 446, 448, 449 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-C404L-L405M-P406A-G443F-L446V-L448W-L449M-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403Q-C404L-P406A-G443F-L446V-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 444, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P444H-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 444, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406S-P444H-L448W-T450M.

In a preferred embodiment, the MDC variant according to the invention showing an improved activity in converting 3-methylcrotonic acid into isobutene is characterized in that it comprises substitutions wherein the substitutions are at positions 195, 241, 403, 404, 406, 444, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: L195I-A241D-S403C-C404L-P406A-P444H-L448W-T450M.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 444, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-P444H-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 407, 444, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-Q407R-P444H-L448W-T450M or A241D-S403C-C404L-P406A-Q407S-P444H-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 408, 444, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-V408I-P444H-L448W-T450M.

In a preferred embodiment, the MDC variant according to the invention showing an improved activity in converting 3-methylcrotonic acid into isobutene is characterized in that it comprises substitutions wherein the substitutions are at positions 195, 241, 403, 404, 406, 408, 444, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: L195I-A241D-S403C-C404L-P406A-V408I-P444H-L446M-L448W-T450M.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 408, 444, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-V408I-P444H-L446M-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 408, 444, 445, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-V408I-P444H-V445C-L446M-L448W-T450M.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 443, 444, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-G443Y-P444H-L446M-L448W-T450M.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 443, 444, 448, and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-G443D-P444H-L448W-L449V or M197V-A241D-S403C-C404L-P406A-G443S-P444H-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 197, 403, 404, 406, 443, 446, 448, 450 and 455 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-S403C-C404M-P406A-G443Y-L446I-L448W-T450H-Q455H.

In a preferred embodiment, the MDC variant according to the invention showing an improved activity in converting 3-methylcrotonic acid into isobutene is characterized in that it comprises substitutions wherein the substitutions are at positions 403, 404, 406, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403A-C404M-P406A-G443Y-L446I-L448W-T450H or S403I-C404M-P406A-G443Y-L446I-L448W-T450H or S403Q-C404M-P406A-G443Y-L446I-L448W-T450H or S403V-C404M-P406A-G443Y-L446I-L448W-T450H or S403C-C404M-P406G-G443Y-L446I-L448W-T450H.

In a preferred embodiment, the MDC variant according to the invention showing an improved activity in converting 3-methylcrotonic acid into isobutene is characterized in that it comprises substitutions wherein the substitutions are at positions 403, 404, 406, 442, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406A-D442A-G443Y-L446I-L448W-T450H or S403C-C404M-P406A-D442E-G443Y-L446I-L448W-T450H or S403C-C404M-P406A-D442G-G443Y-L446I-L448W-T450H or S403C-C404M-P406A-D442S-G443Y-L446I-L448W-T450H.

In a preferred embodiment, the MDC variant according to the invention showing an improved activity in converting 3-methylcrotonic acid into isobutene is characterized in that it comprises substitutions wherein the substitutions are at positions 403, 404, 406, 443, 444, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406A-G443Y-P444H-L446I-L448W-T450H.

In a preferred embodiment, the MDC variant according to the invention showing an improved activity in converting 3-methylcrotonic acid into isobutene is characterized in that it comprises substitutions wherein the substitutions are at positions 403, 404, 406, 443, 445, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406A-G443Y-V445A-L446I-L448W-T450H or S403C-C404M-P406A-G443Y-V445C-L446I-L448W-T450H or S403C-C404M-P406A-G443Y-V445L-L446I-L448W-T450H.

In a preferred embodiment, the MDC variant according to the invention showing an improved activity in converting 3-methylcrotonic acid into isobutene is characterized in that it comprises substitutions wherein the substitutions are at positions 403, 404, 406, 443, 446, 448, 450 and 451 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406A-G443Y-L446I-L448W-T450H-C451N or S403C-C404M-P406A-G443Y-L446I-L448W-T450H-C451S.

In a preferred embodiment, the MDC variant according to the invention showing an improved activity in converting 3-methylcrotonic acid into isobutene is characterized in that it comprises substitutions wherein the substitutions are at positions 196, 197, 403, 404, 406, 443, 446, and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: V196I-M197V-S403C-C404M-P406A-G443Y-L446I-L448W.

In a preferred embodiment, the MDC variant according to the invention showing an improved activity in converting 3-methylcrotonic acid into isobutene is characterized in that it comprises substitutions wherein the substitutions are at positions 197, 403, 404, 406, 443, 446, and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-S403C-C404M-P406G-G443Y-L446I-L448W or M197V-S403C-C404M-P406S-G443Y-L446I-L448W.

In a preferred embodiment, the MDC variant according to the invention showing an improved activity in converting 3-methylcrotonic acid into isobutene is characterized in that it comprises substitutions wherein the substitutions are at positions 197, 403, 404, 406, 442, 443, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-S403C-C404M-P406A-D442A-G443Y-L446I-L448W or M197V-S403C-C404M-P406A-D442C-G443Y-L446I-L448W or M 197V-S403C-C404M-P406A-D442G-G443Y-L4461-L448W or M197V-S403C-C404M-P406A-D442H-G443Y-L446I-L448W.

In a preferred embodiment, the MDC variant according to the invention showing an improved activity in converting 3-methylcrotonic acid into isobutene is characterized in that it comprises substitutions wherein the substitutions are at positions 403, 404, 406, 442, 443, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406A-D442H-G443Y-L446I-L448W.

In a preferred embodiment, the MDC variant according to the invention showing an improved activity in converting 3-methylcrotonic acid into isobutene is characterized in that it comprises substitutions wherein the substitutions are at positions 197, 403, 404, 406, 442, 443, 446, and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-S403C-C404M-P406A-D442N-G443Y-L446I-L448W or M197V-S403C-C404M-P406A-D442S-G443Y-L446I-L448W.

In a preferred embodiment, the MDC variant according to the invention showing an improved activity in converting 3-methylcrotonic acid into isobutene is characterized in that it comprises substitutions wherein the substitutions are at positions 197, 403, 404, 406, 443, 445, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-S403C-C404M-P406A-G443Y-V445S-L446I-L448W or M197V-S403C-C404M-P406A-G443Y-V445T-L4461-L448W.

In a preferred embodiment, the MDC variant according to the invention showing an improved activity in converting 3-methylcrotonic acid into isobutene is characterized in that it comprises substitutions wherein the substitutions are at positions 403, 404, 406, 442, 443, 445, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406A-D442A-G443Y-V445L-L446I-L448W-T450H.

In a preferred embodiment, the MDC variant according to the invention showing an improved activity in converting 3-methylcrotonic acid into isobutene is characterized in that it comprises substitutions wherein the substitutions are at positions 197, 403, 404, 406, 442, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-S403C-C404M-P406A-D442A-G443Y-L446I-L448W-T450H or M197V-S403C-C404M-P406A-D442Q-G443Y-L446I-L448W-T450H or M197V-S403C-C404M-P406A-D442N-G443Y-L446I-L448W-T450H or M197V-S403C-C404M-P406A-D442S-G443Y-L446I-L448W-T450H.

In a preferred embodiment, the MDC variant according to the invention showing an improved activity in converting 3-methylcrotonic acid into isobutene is characterized in that it comprises substitutions wherein the substitutions are at positions 241, 403, 404, 406, 441, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-E441R-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 241, 403, 404, 406, 442, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-D442L-G443D-P444H-L448W-L449I or A241D-S403C-C404L-P406A-D442R-G443D-P444H-L448W-L449I or A241D-S403C-C404L-P406A-D442M-G443D-P444H-L448W-L449I or A241D-S403C-C404L-P406A-D442A-G443D-P444H-L448W-L449I or A241D-S403C-C404L-P406A-D442S-G443D-P444H-L448W-L449I or A241D-S403C-C404L-P406A-D442G-G443D-P444H-L448W-L4491.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 444, 445, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-P444H-V445T-L448W-L449I.

In a preferred embodiment, the MDC variant according to the invention showing an improved activity in converting 3-methylcrotonic acid into isobutene is characterized in that it comprises substitutions wherein the substitutions are at positions 241, 403, 404, 406, 407, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-Q407C-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-P444H-L448W-L449I-P454A or A241D-S403C-C404L-P406A-G443D-P444H-L448W-L449I-P454W or A241D-S403C-C404L-P406A-G443D-P444H-L448W-L449I-P454V or A241D-S403C-C404L-P406A-G443D-P444H-L448W-L449I-P454M or A241 0-S403C-C404L-P406A-G4430-P444H-L448W-L4491-P454H or A241D-S403C-C404L-P406A-G443D-P444H-L448W-L449I-P454T or A241D-S403C-C404L-P406A-G443D-P444H-L448W-L449I-P454G or A241D-S403C-C404L-P406A-G443D-P444H-L448W-L449I-P454K or A241D-S403C-C404L-P406A-G443D-P444H-L448W-L449I-P454R or A241D-S403C-C404L-P406A-G443D-P444H-L448W-L449I-P454Q or A241 0-S403C-C404L-P406A-G4430-P444H-L448W-L4491-P454L.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 444, 448, 449 and 459 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-P444H-L448W-L449I-A459Y or A241D-S403C-C404L-P406A-G443D-P444H-L448W-L449I-A459N.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 444, 448, 449 and 460 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-P444H-L448W-L449I-A460N or A241D-S403C-C404L-P406A-G443D-P444H-L448W-L449I-A460L or A241D-S403C-C404L-P406A-G443D-P444H-L448W-L449I-A460M or A241D-S403C-C404L-P406A-G443D-P444H-L448W-L449I-A460R.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 444, 448, 449 and 461 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-P444H-L448W-L449I-R461V or A241D-S403C-C404L-P406A-G443D-P444H-L448W-L449I-R461S.

In a preferred embodiment, the MDC variant according to the invention showing an improved activity in converting 3-methylcrotonic acid into isobutene is characterized in that it comprises substitutions wherein the substitutions are at positions 154, 241, 339, 403, 404, 406, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: E154D-A241D-E339H-S403C-C404L-P406A-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 241, 302, 339, 403, 404, 406, 443, 444, 448, and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-L302R-E339H-S403C-C404L-P406A-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 363, 403, 404, 406, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-A363G-S403C-C404L-P406A-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 390, 403, 404, 406, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-R390D-S403C-C404L-P406A-G443D-P444H-L448W-L449I or A241D-E339H-R390L-S403C-C404L-P406A-G443D-P444H-L448W-L449I or A241D-E339H-R390N-S403C-C404L-P406A-G443D-P444H-L448W-L449I or A241D-E339H-R390Q-S403C-C404L-P406A-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 391, 403, 404, 406, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-R391D-S403C-C404L-P406A-G443D-P444H-L448W-L449I or A241D-E339H-R391W-S403C-C404L-P406A-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 394, 403, 404, 406, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-E394Q-S403C-C404L-P406A-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 3, 241, 339, 394, 403, 404, 406, 443, 444, 448 or 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: V3M-A241D-E339H-E394T-S403C-C404L-P406A-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 398, 403, 404, 406, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-G398R-S403C-C404L-P406A-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 129, 241, 339, 398, 403, 404, 406, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A129V-A241D-E339H-G398C-S403C-C404L-P406A-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 408, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-V408L-G443D-P444H-L448W-L449I or A241D-E339H-S403C-C404L-P406A-V408M-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 441, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-E441S-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 442, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-D442H-G443D-P444H-L448W-L449I or A241D-E339H-S403C-C404L-P406A-D442R-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 168, 241, 339, 403, 404, 406, 443, 444, 445, 448, and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: I168V-A241D-E339H-S403C-C404L-P406A-G443D-P444H-V445L-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 443, 444, 446, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-G443D-P444H-L446M-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 443, 444, 448, 449 and 452 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-G443D-P444H-L448W-L449I-Y452F.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 443, 444, 448, 449 and 453 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-G443D-P444H-L448W-L449I-T453L.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 443, 444, 448, 449, and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-G443D-P444H-L448W-L449I-P454H or A241D-E339H-S403C-C404L-P406A-G443D-P444H-L448W-L449I-P454K or A241D-E339H-S403C-C404L-P406A-G443D-P444H-L448W-L449I-P454R or A241D-E339H-S403C-C404L-P406A-G443D-P444H-L448W-L449I-P454S or A241D-E339H-S403C-C404L-P406A-G443D-P444H-L448W-L449I-P454T or A241D-E339H-S403C-C404L-P406A-G443D-P444H-L448W-L449I-P454W.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 443, 444, 448, 449, 461 and 479 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-G443D-P444H-L448W-L449I-R461V-P479S.

In a preferred embodiment, the MDC variant according to the invention showing an improved activity in converting 3-methylcrotonic acid into isobutene is characterized in that it comprises substitutions wherein the substitutions are at positions 241, 403, 404, 406, 407, 443 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-Q407A-G443N-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 406, 442, 443 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-D442A-G443N-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443N-L448W-T450H.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 448 and 453 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443N-L448W-T453R.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443N-L446M-L448W.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 441, 443 444, 446, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-E441K-G443D-P444H-L446M-L448W-L449I.

In a preferred embodiment, the MDC variant according to the invention showing an improved activity in converting 3-methylcrotonic acid into isobutene is characterized in that it comprises substitutions wherein the substitutions are at positions 197, 237, 241, 403, 404, 406, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-G237A-A241D-S403C-C404L-P406A-G443F-L448W-T450M or M197V-G237D-A241D-S403C-C404L-P406A-G443F-L448W-T450M or M197V-G237N-A241D-S403C-C404L-P406A-G443F-L448W-T450M or M197V-G237P-A241D-S403C-C404L-P406A-G443F-L448W-T450M.

In a preferred embodiment, the MDC variant according to the invention showing an improved activity in converting 3-methylcrotonic acid into isobutene is characterized in that it comprises substitutions wherein the substitutions are at positions 197, 238, 241, 403, 404, 406, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-D238H-A241D-S403C-C404L-P406A-G443F-L448W-T450M or M197V-D238R-A241D-S403C-C404L-P406A-G443F-L448W-T450M or M197V-D238S-A241D-S403C-C404L-P406A-G443F-L448W-T450M or M197V-D238W-A241D-S403C-C404L-P406A-G443F-L448W-T450M.

In a preferred embodiment, the MDC variant according to the invention showing an improved activity in converting 3-methylcrotonic acid into isobutene is characterized in that it comprises substitutions wherein the substitutions are at positions 197, 241, 302, 403, 404, 406, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-L302A-S403C-C404L-P406A-G443F-L448W-T450M or M197V-A241D-L302K-S403C-C404L-P406A-G443F-L448W-T450M or M197V-A241D-L302M-S403C-C404L-P406A-G443F-L448W-T450M or M197V-A241D-L302R-S403C-C404L-P406A-G443F-L448W-T450M.

In a preferred embodiment, the MDC variant according to the invention showing an improved activity in converting 3-methylcrotonic acid into isobutene is characterized in that it comprises substitutions wherein the substitutions are at positions 197, 241, 359, 403, 404, 406, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-A359V-S403C-C404L-P406A-G443F-L448W-T450M.

In a preferred embodiment, the MDC variant according to the invention showing an improved activity in converting 3-methylcrotonic acid into isobutene is characterized in that it comprises substitutions wherein the substitutions are at positions 197, 241, 398, 403, 404, 406, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-G398R-S403C-C404L-P406A-G443F-L448W-T450M.

In a preferred embodiment, the MDC variant according to the invention showing an improved activity in converting 3-methylcrotonic acid into isobutene is characterized in that it comprises substitutions wherein the substitutions are at positions 197, 241, 394, 403, 404, 406, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-E394C-S403C-C404L-P406A-G443F-L448W-T450M or M197V-A241D-E394D-S403C-C404L-P406A-G443F-L448W-T450M.

In a preferred embodiment, the MDC variant according to the invention showing an improved activity in converting 3-methylcrotonic acid into isobutene is characterized in that it comprises substitutions wherein the substitutions are at positions 197, 241, 396, 403, 404, 406, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-V396I-S403C-C404L-P406A-G443F-L448W-T450M.

In a preferred embodiment, the MDC variant according to the invention showing an improved activity in converting 3-methylcrotonic acid into isobutene is characterized in that it comprises substitutions wherein the substitutions are at positions 35, 197, 241, 403, 404, 406, 407, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: V35I-M197V-A241D-S403C-C404L-P406A-Q4071-G443F-L448W-T450M.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 408, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-V408A-G443F-L448W-T450M.

In a preferred embodiment, the MDC variant according to the invention showing an improved activity in converting 3-methylcrotonic acid into isobutene is characterized in that it comprises substitutions wherein the substitutions are at positions 2, 197, 241, 403, 404, 406, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: Y2N-M197V-A241D-S403C-C404L-P406A-G443F-L448W-T450M.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 439, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-R439C-G443F-L448W-T450M.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 440, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-F440L-G443F-L448W-T450M.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 443, 448, 450 and 460 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-G443F-L448W-T450M-A460F or M197V-A241D-S403C-C404L-P406A-G443F-L448W-T450M-A460L or M197V-A241D-S403C-C404L-P406A-G443F-L448W-T450M-A460R.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 443, 448, 450 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-G443F-L448W-T450M-P454T.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 443, 448, 450 and 462 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-G443F-L448W-T450M-A462V.

In a preferred embodiment, the MDC variant according to the invention showing an improved activity in converting 3-methylcrotonic acid into isobutene is characterized in that it comprises substitutions wherein the substitutions are at positions 403, 404, 406, 407, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406G-Q407T-G443Y-L446I-L448W-T450H.

In a preferred embodiment, the MDC variant according to the invention showing an improved activity in converting 3-methylcrotonic acid into isobutene is characterized in that it comprises substitutions wherein the substitutions are at positions 5, 197, 403, 404, 406, 442, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M5I-M197V-S403C-C404M-P406G-D442N-G443Y-L446I-L448W-T450H.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 443, 444, 448, 449 and 459 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-G443D-P444H-L448W-L449V-A459D.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 448, 449 and 459 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-L448W-L449I-A459G.

In a preferred embodiment, the MDC variant according to the invention showing an improved activity in converting 3-methylcrotonic acid into isobutene is characterized in that it comprises substitutions wherein the substitutions are at positions 403, 404, 406, 407, 443, 446, 448, 450 and 459 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406A-Q407T-G443Y-L446I-L448W-T450H-A459G.

In a preferred embodiment, the MDC variant according to the invention showing an improved activity in converting 3-methylcrotonic acid into isobutene is characterized in that it comprises substitutions wherein the substitutions are at positions 403, 404, 406, 443, 446, 448, 450 and 459 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406A-G443Y-L446I-L448W-T450H-A459G.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 448, 449 and 459 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-L448W-L449I-A459R.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 448, 450 and 460 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443Y-L448W-T450M-A460E.

In a preferred embodiment, the MDC variant according to the invention showing an improved activity in converting 3-methylcrotonic acid into isobutene is characterized in that it comprises substitutions wherein the substitutions are at positions 241, 403, 404, 406, 408, 444, 446, 448, 450 and 460 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-V408I-P444H-L446M-L448W-T450M-A460G.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 443, 444, 448, 449, 460 and 462 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-G443D-P444H-L448W-L449V-A460G-A462V.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 448, 450 and 460 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443Y-L448W-T450M-A460L.

In a preferred embodiment, the MDC variant according to the invention showing an improved activity in converting 3-methylcrotonic acid into isobutene is characterized in that it comprises substitutions wherein the substitutions are at positions 403, 404, 406, 407, 443, 446, 448, 450 and 460 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406A-Q407T-G443Y-L446I-L448W-T450H-A460L.

In a preferred embodiment, the MDC variant according to the invention showing an improved activity in converting 3-methylcrotonic acid into isobutene is characterized in that it comprises substitutions wherein the substitutions are at positions 241, 403, 404, 406, 408, 444, 446, 448, 450 and 460 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-V408I-P444H-L446M-L448W-T450M-A460Q.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 448, 450 and 462 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443Y-L448W-T450M-A462C.

In a preferred embodiment, the MDC variant according to the invention showing an improved activity in converting 3-methylcrotonic acid into isobutene is characterized in that it comprises substitutions wherein the substitutions are at positions 197, 403, 404, 406, 442, 443, 446, 448, 450 and 462 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-S403C-C404M-P406A-D442N-G443Y-L446I-L448W-T450H-A462C.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 448, 450 and 462 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443Y-L448W-T450M-A462V.

In a preferred embodiment, the MDC variant according to the invention showing an improved activity in converting 3-methylcrotonic acid into isobutene is characterized in that it comprises substitutions wherein the substitutions are at positions 403, 404, 406, 407, 443, 446, 448, 450 and 462 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406A-Q407T-G443Y-L446I-L448W-T450H-A462V.

In a preferred embodiment, the MDC variant according to the invention showing an improved activity in converting 3-methylcrotonic acid into isobutene is characterized in that it comprises substitutions wherein the substitutions are at positions 403, 404, 406, 407, 443, 446, 448, 450, 454 and 462 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406A-Q407T-G443Y-L446I-L448W-T450H-P454Q-A462V.

In a preferred embodiment, the MDC variant according to the invention showing an improved activity in converting 3-methylcrotonic acid into isobutene is characterized in that it comprises substitutions wherein the substitutions are at positions 198, 403, 404, 406, 407, 443, 446, 448, and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: H198W-S403V-C404M-P406A-Q407T-G443Y-L446I-L448W-T450H.

In a preferred embodiment, the MDC variant according to the invention showing an improved activity in converting 3-methylcrotonic acid into isobutene is characterized in that it comprises substitutions wherein the substitutions are at positions 74, 198, 403, 404, 406, 407, 443, 446, 448, and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: L74V-H198W-S403V-C404M-P406A-Q407T-G443Y-L446I-L448W-T450H.

In a preferred embodiment, the MDC variant according to the invention showing an improved activity in converting 3-methylcrotonic acid into isobutene is characterized in that it comprises substitutions wherein the substitutions are at positions 198, 403, 404, 406, 442, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: H198W-S403C-C404M-P406A-D442Q-G443Y-L446I-L448W-T450H.

In a preferred embodiment, the MDC variant according to the invention showing an improved activity in converting 3-methylcrotonic acid into isobutene is characterized in that it comprises substitutions wherein the substitutions are at positions 241, 403, 404, 406, 441, 443, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-E441G-G443D-L448W-L449I.

In a preferred embodiment, the MDC variant according to the invention showing an improved activity in converting 3-methylcrotonic acid into isobutene is characterized in that it comprises substitutions wherein the substitutions are at positions 208, 241, 403, 404, 406, 408, 443, 444, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: Q208L-A241D-S403C-C404L-P406A-V408I-G443R-P444H-L446M-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 408, 443, 444, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-V408I-G443W-P444H-L446M-L448W-T450M.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 448, 449 and 458 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-L448W-L449I-H458G.

In a preferred embodiment, the MDC variant according to the invention showing an improved activity in converting 3-methylcrotonic acid into isobutene is characterized in that it comprises substitutions wherein the substitutions are at positions 195, 403, 404, 406, 407, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: L195V-S403C-C404M-P406A-Q407T-G443Y-L446I-L448W-T450H.

In a preferred embodiment, the MDC variant according to the invention showing an improved activity in converting 3-methylcrotonic acid into isobutene is characterized in that it comprises substitutions wherein the substitutions are at positions 195, 403, 404, 406, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: L195V-S403C-C404M-P406A-G443Y-L446I-L448W-T450H.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-G443Y-L448W-T450M.

In a preferred embodiment, the MDC variant according to the invention showing an improved activity in converting 3-methylcrotonic acid into isobutene is characterized in that it comprises substitutions wherein the substitutions are at positions 197, 403, 404, 406, 407, 443, 446, 448, and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-S403C-C404M-P406A-Q407T-G443Y-L446I-L448W-T450H.

In a preferred embodiment, the MDC variant according to the invention showing an improved activity in converting 3-methylcrotonic acid into isobutene is characterized in that it comprises substitutions wherein the substitutions are at positions 197, 403, 404, 406, 442, 443, 446, 448, 450 and 456 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-S403C-C404M-P406A-D442N-G443Y-L446I-L448W-T450H-E456Q.

In a preferred embodiment, the MDC variant according to the invention showing an improved activity in converting 3-methylcrotonic acid into isobutene is characterized in that it comprises substitutions wherein the substitutions are at positions 403, 404, 406, 407, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404F-P406A-Q407T-G443Y-L446I-L448W-T450H.

In a preferred embodiment, the MDC variant according to the invention showing an improved activity in converting 3-methylcrotonic acid into isobutene is characterized in that it comprises substitutions wherein the substitutions are at positions 403, 404, 406, 407, 443, 444, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406A-Q407T-G443Y-P444F-L446I-L448W-T450H.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-L448W-L449I-P454K.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-G443D-P444H-L448W-L449V-P454K.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-L448W-L449I-P454R.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-G443D-P444H-L448W-L449V-P454R.

In a preferred embodiment, the MDC variant according to the invention showing an improved activity in converting 3-methylcrotonic acid into isobutene is characterized in that it comprises substitutions wherein the substitutions are at positions 241, 403 404, 406, 443, 448, 449 and 455 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-L448W-L449I-Q455S.

In a preferred embodiment, the MDC variant according to the invention showing an improved activity in converting 3-methylcrotonic acid into isobutene is characterized in that it comprises substitutions wherein the substitutions are at positions 197, 403, 404, 406, 442, 443, 446, 448, 450 and 455 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-S403C-C404M-P406A-D442N-G443Y-L446I-L448W-T450H-Q455V.

In a preferred embodiment, the MDC variant according to the invention showing an improved activity in converting 3-methylcrotonic acid into isobutene is characterized in that it comprises substitutions wherein the substitutions are at positions 403, 404, 406, 407, 443, 446, 448, 450 and 457 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406A-Q407T-G443Y-L446I-L448W-T450H-R457K.

In a preferred embodiment, the MDC variant according to the invention showing an improved activity in converting 3-methylcrotonic acid into isobutene is characterized in that it comprises substitutions wherein the substitutions are at positions 197, 403, 404, 406, 442, 443, 446, 448, 450, and 461 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-S403C-C404M-P406A-D442N-G443Y-L446I-L448W-T450H-R461G.

In a preferred embodiment, the MDC variant according to the invention showing an improved activity in converting 3-methylcrotonic acid into isobutene is characterized in that it comprises substitutions wherein the substitutions are at positions 241, 403, 404, 406, 408, 444, 446, 448, 450, and 461 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-V408I-P444H-L446M-L448W-T450M-R461S.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 443, 444, 448, 449 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-G443D-P444H-L448W-L449V-T450H.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 448, 449 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-L448W-L449I-T450V.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 443, 444, 448, 449 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-G443D-P444H-L448W-L449V-T450V.

In a preferred embodiment, the MDC variant according to the invention showing an improved activity in converting 3-methylcrotonic acid into isobutene is characterized in that it comprises substitutions wherein the substitutions are at positions 36, 197, 403, 404, 406, 442, 443, 446, 448, 450 and 453 in the amino acid sequence shown in SEQ ID NO:1 or at positions corresponding to these positions. Preferably, such a variant has the following substitutions in the amino acid sequence shown in SEQ ID NO:1 or at positions corresponding to these positions: H36Y-M197V-S403C-C404M-P406A-D442N-G443Y-L446I-L448W-T450H-T453G.

In a preferred embodiment, the MDC variant according to the invention showing an improved activity in converting 3-methylcrotonic acid into isobutene is characterized in that it comprises substitutions wherein the substitutions are at positions 403, 404, 406, 407, 443, 446, 448, 450 and 453 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406A-Q407T-G443Y-L446I-L448W-T450H-T453N.

In a preferred embodiment, the MDC variant according to the invention showing an improved activity in converting 3-methylcrotonic acid into isobutene is characterized in that it comprises substitutions wherein the substitutions are at positions 198, 403, 404, 406, 443, 446, 448, 450 and 453 in the amino acid sequence shown in SEQ ID NO:1 or at positions corresponding to these positions. Preferably, such a variant has the following substitutions in the amino acid sequence shown in SEQ ID NO:1 or at positions corresponding to these positions: H198W-S403C-C404M-P406A-G443Y-L446I-L448W-T450H-T453P.

In a preferred embodiment, the MDC variant according to the invention showing an improved activity in converting 3-methylcrotonic acid into isobutene is characterized in that it comprises substitutions wherein the substitutions are at positions 241, 403, 404, 406, 408, 444, 446, 448, 450 and 453 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-V408I-P444H-L446M-L448W-T450M-T453R or A241D-S403C-C404L-P406A-V408I-P444H-L446M-L448W-T450M-T453S.

In a preferred embodiment, the MDC variant according to the invention showing an improved activity in converting 3-methylcrotonic acid into isobutene is characterized in that it comprises substitutions wherein the substitutions are at positions 403, 404, 406, 407, 443, 445, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404M-P406A-Q407T-G443Y-V445L-L446I-L448W-T450H or S403C-C404M-P406A-Q407T-G443Y-V445S-L446I-L448W-T450H.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 443, 444 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-G443D-P444H-L448W.

In a preferred embodiment, the MDC variant according to the invention showing an improved activity in converting 3-methylcrotonic acid into isobutene is characterized in that it comprises substitutions wherein the substitutions are at positions 241, 403, 404, 406, 408, 444, 446, 448, 450 and 452 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-V408I-P444H-L446M-L448W-T450M-Y452F.

In a preferred embodiment, the MDC variant according to the invention showing an improved activity in converting 3-methylcrotonic acid into isobutene is characterized in that it comprises substitutions wherein the substitutions are at positions 5, 241, 403, 404, 406, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M5I-A241D-S403C-C404L-P406A-G443Y-L448W-T450M.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-G443F-L446M-L448W-T450M.

In a preferred embodiment, the MDC variant according to the invention showing an improved activity in converting 3-methylcrotonic acid into isobutene is characterized in that it comprises substitutions wherein the substitutions are at positions 197, 403, 404, 406, 442, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-S403C-C404L-P406A-D442N-G443Y-L446M-L448W-T450H.

In a preferred embodiment, the MDC variant according to the invention showing an improved activity in converting 3-methylcrotonic acid into isobutene is characterized in that it comprises substitutions wherein the substitutions are at positions 197, 403, 404, 406, 442, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-S403C-C404L-P406A-D442N-G443Y-L448W-T450M or M197V-S403C-C404L-P406A-D442N-G443Y-L448W-T450H.

In a preferred embodiment, the MDC variant according to the invention showing an improved activity in converting 3-methylcrotonic acid into isobutene is characterized in that it comprises substitutions wherein the substitutions are at positions 403, 404, 406, 407, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404L-P406A-Q407T-G443Y-L446M-L448W-T450H or S403C-C404L-P406A-Q407T-G443Y-L446F-L448W-T450H.

In a preferred embodiment, the MDC variant according to the invention showing an improved activity in converting 3-methylcrotonic acid into isobutene is characterized in that it comprises substitutions wherein the substitutions are at positions 403, 404, 406, 407, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404L-P406A-Q407T-G443Y-L448W-T450H.

In a preferred embodiment, the MDC variant according to the invention showing an improved activity in converting 3-methylcrotonic acid into isobutene is characterized in that it comprises substitutions wherein the substitutions are at positions 241, 339, 343, 403, 404, 406, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-A343S-S403C-C404L-P406A-G443D-P444H-L448W-L449I-P454R.

In a preferred embodiment, the MDC variant according to the invention showing an improved activity in converting 3-methylcrotonic acid into isobutene is characterized in that it comprises substitutions wherein the substitutions are at positions 241, 339, 350, 403, 404, 406, 443, 444, 446, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-A350S-S403C-C404L-P406A-G443D-P444H-L446M-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 395, 403, 404, 406, 426, 443, 444, 446, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-A395E-S403C-C404L-P406A-A426V-G443D-P444H-L446M-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 442, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-D442N-G443D-P444H-L448W-L449I-P454R or A241D-E339H-S403C-C404L-P406A-D442S-G443D-P444H-L448W-L449I-P454R.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 442, 443, 444, 446, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-D442T-G443D-P444H-L446M-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-G443H-P444H-L448W-L449I-P454R.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 441, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-E441R-G443D-P444H-L448W-L449I-P454R.

In a preferred embodiment, the MDC variant according to the invention showing an improved activity in converting 3-methylcrotonic acid into isobutene is characterized in that it comprises substitutions wherein the substitutions are at positions 241, 339, 347, 403, 404, 406, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-I347C-S403C-C404L-P406A-G443D-P444H-L448W-L449I-P454R.

In a preferred embodiment, the MDC variant according to the invention showing an improved activity in converting 3-methylcrotonic acid into isobutene is characterized in that it comprises substitutions wherein the substitutions are at positions 3, 241, 302, 339, 403, 404, 406, 443, 444, 446, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: V3L-A241D-L302Q-E339H-S403C-C404L-P406A-G443D-P444H-L446M-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 403, 404, 405, 406, 443, 444, 446, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-L405T-P406A-G443D-P444H-L446M-L448W-L449I.

In a preferred embodiment, the MDC variant according to the invention showing an improved activity in converting 3-methylcrotonic acid into isobutene is characterized in that it comprises substitutions wherein the substitutions are at positions 5, 241, 339, 403, 404, 406, 443, 444, 446, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M5I-A241D-E339H-S403C-C404L-P406A-G443D-P444H-L446M-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 443, 444, 448, 449, 454 and 455 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-G443D-P444H-L448W-L4491-P454R-Q455A or A241D-E339H-S403C-C404L-P406A-G443D-P444H-L448W-L449I-P454R-Q455N.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 444, 448, 449, 454 and 459 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-P444H-L448W-L449I-P454R-A459E or A241D-S403C-C404L-P406A-G443D-P444H-L448W-L449I-P454R-A459M or A241D-S403C-C404L-P406A-G443D-P444H-L448W-L449I-P454R-A459Q or A241D-S403C-C404L-P406A-G443D-P444H-L448W-L449I-P454R-A459T.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443N-P444H-L448W-L449I-P454R.

In a preferred embodiment, the MDC variant according to the invention showing an improved activity in converting 3-methylcrotonic acid into isobutene is characterized in that it comprises substitutions wherein the substitutions are at positions 241, 403, 404, 406, 441, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-E441G-G443D-P444H-L448W-L449I-P454R.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-P444F-L448W-L4491-P454R.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 444, 448, 449, 454 and 458 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-P444H-L448W-L449I-P454R-H458F or A241D-S403C-C404L-P406A-G443D-P444H-L448W-L449I-P454R-H458N.

In a preferred embodiment, the MDC variant according to the invention showing an improved activity in converting 3-methylcrotonic acid into isobutene is characterized in that it comprises substitutions wherein the substitutions are at positions 241, 296, 403, 404, 406, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-I296Q-S403C-C404L-P406A-G443D-P444H-L448W-L449I-P454R.

In a preferred embodiment, the MDC variant according to the invention showing an improved activity in converting 3-methylcrotonic acid into isobutene is characterized in that it comprises substitutions wherein the substitutions are at positions 241, 302, 403, 404, 406, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-L302A-S403C-C404L-P406A-G443D-P444H-L448W-L449I-P454R.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 444, 448, 449, 453 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-G443D-P444H-L448W-L449I-T453S-P454R.

In a preferred embodiment, the MDC variant according to the invention showing an improved activity in converting 3-methylcrotonic acid into isobutene is characterized in that it comprises substitutions wherein the substitutions are at positions 196, 241, 403, 404, 406, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: V196I-A241D-S403C-C404L-P406A-G443D-P444H-L448W-L449I-P454R.

In a preferred embodiment, the MDC variant according to the invention showing an improved activity in converting 3-methylcrotonic acid into isobutene is characterized in that it comprises substitutions wherein the substitutions are at positions 241, 305, 403, 404, 406, 443, 444, 448, 449, and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-V305I-S403C-C404L-P406A-G443D-P444H-L448W-L449I-P454R or A241D-V305T-S403C-C404L-P406A-G443D-P444H-L448W-L449I-P454R.

In a preferred embodiment, the MDC variant according to the invention showing an improved activity in converting 3-methylcrotonic acid into isobutene is characterized in that it comprises substitutions wherein the substitutions are at positions 197, 237, 241, 403, 404, 406, 441, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-G237A-A241D-S403C-C404L-P406A-E441G-G443F-L448W-T450M or M197V-G237A-A241D-S403C-C404L-P406A-E441H-G443F-L448W-T450M or M197V-G237A-A241D-S403C-C404L-P406A-E441T-G443F-L448W-T450M.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 440, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-F440L-G443F-L446M-L448W-T450M.

In a preferred embodiment, the MDC variant according to the invention showing an improved activity in converting 3-methylcrotonic acid into isobutene is characterized in that it comprises substitutions wherein the substitutions are at positions 197, 241, 277, 403, 404, 406, 440, 443, 448, 449 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-H277Y-S403C-C404L-P406A-F440L-G443F-L448W-L449I-T450M.

In a preferred embodiment, the MDC variant according to the invention showing an improved activity in converting 3-methylcrotonic acid into isobutene is characterized in that it comprises substitutions wherein the substitutions are at positions 197, 237, 241, 403, 404, 406, 443, 444, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-G237A-A241D-S403C-C404L-P406A-G443F-P444H-L448W-T450M or M197V-G237A-A241D-S403C-C404L-P406A-G443F-P4441-L448W-T450M.

In a preferred embodiment, the MDC variant according to the invention showing an improved activity in converting 3-methylcrotonic acid into isobutene is characterized in that it comprises substitutions wherein the substitutions are at positions 197, 241, 403, 404, 406, 407, 440, 443, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-A241D-S403C-C404L-P406A-Q407R-F440L-G443F-L448W-T450M.

In a preferred embodiment, the MDC variant according to the invention showing an improved activity in converting 3-methylcrotonic acid into isobutene is characterized in that it comprises substitutions wherein the substitutions are at positions 6, 197, 238, 241, 403, 404, 406, 443, 448, 450 and 453 in the amino acid sequence shown in SEQ ID NO:1 or at positions corresponding to these positions. Preferably, such a variant has the following substitutions in the amino acid sequence shown in SEQ ID NO:1 or at positions corresponding to these positions: V6M-M197V-D238H-A241D-S403C-C404L-P406A-G443F-L448W-T450M-T453C.

In a preferred embodiment, the MDC variant according to the invention showing an improved activity in converting 3-methylcrotonic acid into isobutene is characterized in that it comprises substitutions wherein the substitutions are at positions 197, 238, 241, 403, 404, 406, 443, 448, 450 and 453 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-D238H-A241D-S403C-C404L-P406A-G443F-L448W-T450M-T453R.

In a preferred embodiment, the MDC variant according to the invention showing an improved activity in converting 3-methylcrotonic acid into isobutene is characterized in that it comprises substitutions wherein the substitutions are at positions 197, 238, 241, 403, 404, 406, 443, 448, 450 and 452 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M197V-D238H-A241D-S403C-C404L-P406A-G443F-L448W-T450M-Y452F.

In a preferred embodiment, the MDC variant according to the invention showing an improved activity in converting 3-methylcrotonic acid into isobutene is characterized in that it comprises substitutions wherein the substitutions are at positions 241, 403, 404, 406, 444, 446, 448, 450 and 452 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-P444H-L446M-L448W-T450M-Y452F.

In a preferred embodiment, the MDC variant according to the invention showing an improved activity in converting 3-methylcrotonic acid into isobutene is characterized in that it comprises substitutions wherein the substitutions are at positions 241, 403, 404, 406, 408, 444, 446, 448, 450, 452 and 455 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-V408I-P444H-L446M-L448W-T450M-Y452F-Q455H.

In a preferred embodiment, the MDC variant according to the invention showing an improved activity in converting 3-methylcrotonic acid into isobutene is characterized in that it comprises substitutions wherein the substitutions are at positions 241, 403, 404, 406, 408, 444, 446, 447, 448, 450 and 460 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-V408I-P444H-L446M-P447Q-L448W-T450M-A460G.

In a preferred embodiment, the MDC variant according to the invention showing an improved activity in converting 3-methylcrotonic acid into isobutene is characterized in that it comprises substitutions wherein the substitutions are at positions 241, 403, 404, 406, 408, 444, 446, 448, 450, 455 and 461 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406A-V408I-P444H-L446M-L448W-T450M-Q455K-R461S or A241D-S403C-C404L-P406A-V408I-P444H-L446M-L448W-T450M-Q455V-R461S.

In a preferred embodiment, the MDC variant according to the invention showing an improved activity in converting 3-methylcrotonic acid into isobutene is characterized in that it comprises substitutions wherein the substitutions are at positions 204, 403, 404, 406, 407, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: Q204R-S403C-C404L-P406A-Q407T-G443Y-L446F-L448W-T450H or Q204S-S403C-C404L-P406A-Q407T-G443Y-L446F-L448W-T450H.

In a preferred embodiment, the MDC variant according to the invention showing an improved activity in converting 3-methylcrotonic acid into isobutene is characterized in that it comprises substitutions wherein the substitutions are at positions 302, 403, 404, 406, 407, 443, 446, 448, and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: L302F-S403C-C404L-P406A-Q407T-G443Y-L446F-L448W-T450H or L302S-S403C-C404L-P406A-Q407T-G443Y-L446F-L448W-T450H.

In a preferred embodiment, the MDC variant according to the invention showing an improved activity in converting 3-methylcrotonic acid into isobutene is characterized in that it comprises substitutions wherein the substitutions are at positions 349, 403, 404, 406, 407, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: E349S-S403C-C404L-P406A-Q407T-G443Y-L446F-L448W-T450H.

In a preferred embodiment, the MDC variant according to the invention showing an improved activity in converting 3-methylcrotonic acid into isobutene is characterized in that it comprises substitutions wherein the substitutions are at positions 395, 403, 404, 406, 407, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A395C-S403C-C404L-P406A-Q407T-G443Y-L446F-L448W-T450H.

In a preferred embodiment, the MDC variant according to the invention showing an improved activity in converting 3-methylcrotonic acid into isobutene is characterized in that it comprises substitutions wherein the substitutions are at positions 403, 404, 406, 407, 441, 443, 446, 448, and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404L-P406A-Q407T-E441P-G443Y-L446F-L448W-T450H.

In a preferred embodiment, the MDC variant according to the invention showing an improved activity in converting 3-methylcrotonic acid into isobutene is characterized in that it comprises substitutions wherein the substitutions are at positions 403, 404, 406, 407, 443, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404L-P406A-Q407T-G443A-L446F-L448W-T450H.

In a preferred embodiment, the MDC variant according to the invention showing an improved activity in converting 3-methylcrotonic acid into isobutene is characterized in that it comprises substitutions wherein the substitutions are at positions 403, 404, 406, 407, 443, 444, 446, 448 and 450 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404L-P406A-Q407T-G443Y-P444Q-L446F-L448W-T450H.

In a preferred embodiment, the MDC variant according to the invention showing an improved activity in converting 3-methylcrotonic acid into isobutene is characterized in that it comprises substitutions wherein the substitutions are at positions 403, 404, 406, 407, 443, 446, 448, 450 and 458 in the amino acid sequence shown in SEQ ID NO:1 or at positions corresponding to these positions. Preferably, such a variant has the following substitutions in the amino acid sequence shown in SEQ ID NO:1 or at positions corresponding to these positions: S403C-C404L-P406A-Q407T-G443Y-L446F-L448W-T450H-H458L or S403C-C404L-P406A-Q407T-G443Y-L446F-L448W-T450H-H458M.

In a preferred embodiment, the MDC variant according to the invention showing an improved activity in converting 3-methylcrotonic acid into isobutene is characterized in that it comprises substitutions wherein the substitutions are at positions 241, 339, 366, 403, 404, 406, 443, 444, 446, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-H366F-S403C-C404L-P406A-G443D-P444H-L446M-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 366, 403, 404, 406, 443, 444, 446 and 448 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-H366F-S403C-C404L-P406A-G443D-P444H-L446M-L448W.

In a preferred embodiment, the MDC variant according to the invention showing an improved activity in converting 3-methylcrotonic acid into isobutene is characterized in that it comprises substitutions wherein the substitutions are at positions 241, 339, 366, 403, 404, 406, 443, 444, 446, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-H366I-S403C-C404L-P406A-G443D-P444H-L446M-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 368, 403, 404, 406, 443, 444, 446, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-L368I-S403C-C404L-P406A-G443D-P444H-L446M-L448W-L449I or A241D-E339H-L368M-S403C-C404L-P406A-G443D-P444H-L446M-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 368, 403, 404, 405, 406, 443, 444, 446, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-L368M-S403C-C404L-L405M-P406A-G443D-P444H-L446M-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 395, 403, 404, 406, 442, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-A395V-S403C-C404L-P406A-D442N-G443D-P444H-L448W-L449I-P454R.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 442, 443, 444, 448, 449, 454 and 460 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-D442N-G443D-P444H-L448W-L449I-P454R-A460F.

In a preferred embodiment, the MDC variant according to the invention showing an improved activity in converting 3-methylcrotonic acid into isobutene is characterized in that it comprises substitutions wherein the substitutions are at positions 241, 339, 349, 403, 404, 406, 442, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-E349M-S403C-C404L-P406A-D442N-G443D-P444H-L448W-L449I-P454R.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 441, 442, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-E441H-D442N-G443D-P444H-L448W-L4491-P454R or A241D-E339H-S403C-C404L-P406A-E441N-D442N-G443D-P444H-L448W-L4491-P454R or A241D-E339H-S403C-C404L-P406A-E441R-D442N-G443D-P444H-L448W-L4491-P454R.

In a preferred embodiment, the MDC variant according to the invention showing an improved activity in converting 3-methylcrotonic acid into isobutene is characterized in that it comprises substitutions wherein the substitutions are at positions 126, 241, 339, 403, 404, 406, 442, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A126V-A241D-E339H-S403C-C404L-P406A-D442N-G443D-P444H-L448W-L449I-P454R.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 442, 443, 444, 448, 449, 454 and 458 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-D442N-G443D-P444H-L448W-L449I-P454R-H458E or A241D-E339H-S403C-C404L-P406A-D442N-G443D-P444H-L448W-L449I-P454R-H458G or A241D-E339H-S403C-C404L-P406A-D442N-G443D-P444H-L448W-L449I-P454R-H458L or A241D-E339H-S403C-C404L-P406A-D442N-G443D-P444H-L448W-L449I-P454R-H458Q or A241D-E339H-S403C-C404L-P406A-D442N-G443D-P444H-L448W-L449I-P454R-H458R or A241D-E339H-S403C-C404L-P406A-D442N-G443D-P444H-L448W-L449I-P454R-H458S or A241D-E339H-S403C-C404L-P406A-D442N-G443D-P444H-L448W-L449I-P454R-H458Y.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 442, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-D442N-G443D-P444H-L448W-L449V-P454R.

In a preferred embodiment, the MDC variant according to the invention showing an improved activity in converting 3-methylcrotonic acid into isobutene is characterized in that it comprises substitutions wherein the substitutions are at positions 241, 302, 339, 403, 404, 406, 442, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-L302C-E339H-S403C-C404L-P406A-D442N-G443D-P444H-L448W-L449I-P454R.

In a preferred embodiment, the MDC variant according to the invention showing an improved activity in converting 3-methylcrotonic acid into isobutene is characterized in that it comprises substitutions wherein the substitutions are at positions 241, 339, 403, 404, 405, 406, 442, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-L405V-P406A-D442N-G443D-P444H-L448W-L449I-P454R.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 407, 442, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-Q407R-D442N-G443D-P444H-L448W-L4491-P454R or A241D-E339H-S403C-C404L-P406A-Q407S-D442N-G443D-P444H-L448W-L449I-P454R.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 442, 443, 444, 448, 449, 454 and 455 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-D442N-G443D-P444H-L448W-L449I-P454R-Q455E.

In a preferred embodiment, the MDC variant according to the invention showing an improved activity in converting 3-methylcrotonic acid into isobutene is characterized in that it comprises substitutions wherein the substitutions are at positions 36, 241, 298, 339, 403, 404, 406, 442, 443, 444, 448, 449 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: H36Y-A241D-R298S-E339H-S403C-C404L-P406A-D442N-G443D-P444H-L448W-L449I-P454R.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 442, 443, 444, 448, 449, and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-D442N-G443D-P444H-L448W-L449I-P454A or A241D-E339H-S403C-C404L-P406A-D442N-G443D-P444H-L448W-L449I-P454D or A241D-E339H-S403C-C404L-P406A-D442N-G443D-P444H-L448W-L449I-P454L.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 442, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-D442N-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 442, 443, 444, 448, 449, and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-D442N-G443D-P444H-L448W-L449I-P454S.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 442, 443, 444, 448, 449, 454 and 461 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-D442N-G443D-P444H-L448W-L449I-P454R-R461A or A241D-E339H-S403C-C404L-P406A-D442N-G443D-P444H-L448W-L4491-P454R-R461L or A241D-E339H-S403C-C404L-P406A-D442N-G443D-P444H-L448W-L449I-P454R-R461M or A241D-E339H-S403C-C404L-P406A-D442N-G443D-P444H-L448W-L449I-P454R-R461Q or A241D-E339H-S403C-C404L-P406A-D442N-G443D-P444H-L448W-L449I-P454R-R461S or A241D-E339H-S403C-C404L-P406A-D442N-G443D-P444H-L448W-L449I-P454R-R461V.

In a preferred embodiment, the MDC variant according to the invention showing an improved activity in converting 3-methylcrotonic acid into isobutene is characterized in that it comprises substitutions wherein the substitutions are at positions 241, 339, 403, 404, 406, 442, 443, 444, 448, 449, 450 and 454 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-E339H-S403C-C404L-P406A-D442N-G443D-P444H-L448W-L449I-T450S-P454R.

In a preferred embodiment, the MDC variant according to the invention showing an improved activity in converting 3-methylcrotonic acid into isobutene is characterized in that it comprises substitutions wherein the substitutions are at positions 241, 403, 404, 406, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: A241D-S403C-C404L-P406S-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 5, 241, 403, 404, 406, 443, 444, 448 and 449 in the amino acid sequence shown in SEQ ID NO:1 or at positions corresponding to these positions. Preferably, such a variant has the following substitutions in the amino acid sequence shown in SEQ ID NO:1 or at positions corresponding to these positions: M51-A241D-S403C-C404L-P406A-G443D-P444H-L448W-L449I.

In a preferred embodiment, the MDC variant according to the invention showing an improved activity in converting 3-methylcrotonic acid into isobutene is characterized in that it comprises substitutions wherein the substitutions are at positions 166, 241, 403, 404, 406, 443, 444, 448, 449 and 452 in the amino acid sequence shown in SEQ ID NO:1 or at positions corresponding to these positions. Preferably, such a variant has the following substitutions in the amino acid sequence shown in SEQ ID NO:1 or at positions corresponding to these positions: V166D-A241D-S403C-C404L-P406A-G443D-P444H-L448W-L449I-Y452F.

Preferably, any of the above described variants having multiple mutations further has one or more substitutions in comparison to the corresponding sequence from which it is derived and wherein these substitutions occur at one or more of the positions corresponding to positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 241, 277, 296, 305, 339, 343, 347, 349, 350, 363, 391, 394, 398, 401, 403, 404, 405, 439, 440, 441, 442, 443, 444, 445, 446, 447, 448, 453, 459, 460, 461, 479 and 496 in the amino acid sequence shown in SEQ ID NO:1.

The present invention also relates to a method for providing a variant of an MDC wherein said variant shows an improved activity of converting 3-methylcrotonic acid into isobutene, said method comprising the step of effecting one or more changes in the sequence of the MDC wherein said change(s) is/are effected at one or more amino acid positions selected from the group consisting of the amino acid positions corresponding to positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462, 473, 2, 3, 5, 6, 24, 64, 66, 85, 154, 168, 175, 195, 197, 214, 241, 277, 296, 305, 339, 343, 347, 349, 350, 363, 391, 394, 398, 401, 403, 404, 405, 439, 440, 441, 442, 443, 444, 445, 446, 447, 448, 453, 459, 460, 461, 479 and 496 in the amino acid sequence shown in SEQ ID NO:1. "Corresponding to" means corresponding to any of these positions in a related sequence.

As regards the preferred embodiments of an MDC to be mutated according to such a method, the same applies as has been set forth herein-above.

In one preferred embodiment the MDC from which the MDC variant is derived is an MDC which shows the amino acid sequence as shown in SEQ ID NO:1 or an amino acid sequence having at least 80%, 90%, 94% or 97% sequence identity to SEQ ID NO:1 or any of the preferred degrees of sequence identity as specified herein above. Moreover, as regards preferred embodiments of the degree of improvement in activity and the changes to be effected, the same applies as described herein above.

The change(s) which is/are effected at any of the above position(s) is/are substitution(s) as defined herein above.

An MDC variant of the present invention can be fused to a homologous or heterologous polypeptide or protein, an enzyme, a substrate or a tag to form a fusion protein. Fusion proteins in accordance with the present invention will have the same improved activity as the MDC variant of the present invention. Polypeptides, enzymes, substrates or tags that can be added to another protein are known in the art. They may useful for purifying or detecting the proteins of the invention. For instance, tags that can be used for detection and/or purification are e.g. FLAG-tag, His6-tag or a Strep-tag. Alternatively, the protein of the invention can be fused to an enzyme e.g. luciferase, for the detection or localisation of said protein. Other fusion partners include, but are not limited to, bacterial β-galactosidase, trpE, Protein A, β-lactamase, alpha amylase, alcohol dehydrogenase or yeast alpha mating factor. It is also conceivable that the polypeptide, enzyme, substrate or tag is removed from the protein of the invention after e.g. purification. Fusion proteins can typically be made by either recombinant nucleic acid methods or by synthetic polypeptide methods known in art.

The present invention further relates to a nucleic acid molecule encoding an MDC variant of the present invention and to a vector comprising said nucleic acid molecules. Vectors that can be used in accordance with the present invention are known in the art. The vectors can further comprise expression control sequences operably linked to the nucleic acid molecules of the present invention contained in the vectors. These expression control sequences may be suited to ensure transcription and synthesis of a translatable RNA in bacteria or fungi. Expression control sequences can for instance be promoters. Promoters for use in connection with the nucleic acid molecules of the present invention may be homologous or heterologous with regard to its origin and/or with regard to the gene to be expressed. Suitable promoters are for instance promoters which lend themselves to constitutive expression. However, promoters which are only activated at a point in time determined by external influences can also be used. Artificial and/or chemically inducible promoters may be used in this context.

Preferably, the vector of the present invention is an expression vector. Expression vectors have been widely described in the literature. As a rule, they contain not only a selection marker gene and a replication-origin ensuring replication in the host selected, but also a bacterial or viral promoter, and in most cases a termination signal for transcription. Between the promoter and the termination signal there is in general at least one restriction site or a polylinker which enables the insertion of a coding DNA sequence. The DNA sequence naturally controlling the transcription of the corresponding gene can be used as the promoter sequence, if it is active in the selected host organism. However, this sequence can also be exchanged for other promoter sequences. It is possible to use promoters ensuring constitutive expression of the gene and inducible promoters which permit a deliberate control of the expression of the gene. Bacterial and viral promoter sequences possessing these properties are described in detail in the literature. Regulatory sequences for the expression in microorganisms (for instance *E*. *coli, S*. *cerevisiae)* are sufficiently described in the literature. Promoters permitting a particularly high expression of a downstream sequence are for instance the T7 promoter (Studier et al., Methods in Enzymology 185 (1990), 60-89), lacUV5, trp, trp-lacUV5 (DeBoer et al., in Rodriguez and Chamberlin (Eds), Promoters, Structure and Function; Praeger, New York, (1982), 462-481; DeBoer et al., Proc. Natl. Acad. Sci. USA (1983), 21-25), Ip1, rac (Boros et al., Gene 42 (1986), 97-100). Inducible promoters are preferably used for the synthesis of polypeptides. These promoters often lead to higher polypeptide yields than do constitutive promoters. In order to obtain an optimum amount of polypeptide, a two-stage process is often used. First, the host cells are cultured under optimum conditions up to a relatively high cell density. In the second step, transcription is induced depending on the type of promoter used. In this regard, a tac promoter is particularly suitable which can be induced by lactose or IPTG (=isopropyl-β-D-thiogalactopyranoside) (deBoer et al., Proc. Natl. Acad. Sci. USA 80 (1983), 21-25). Termination signals for transcription are also described in the literature.

In addition, the present invention relates to a host cell comprising the nucleic acid molecule or the vector of the present invention.

In a preferred embodiment, the host cell according to the presenting invention is a microorganism, in particular a bacterium or a fungus. In a more preferred embodiment, the host cell of the present invention is E. coli, a bacterium of the genus Clostridium or a yeast cell, such as S. cerevisiae. In another preferred embodiment the host cell is a plant cell or a non-human animal cell.

The transformation of the host cell with a vector according to the invention can be carried out by standard methods, as for instance described in Sambrook and Russell (2001), Molecular Cloning: A Laboratory Manual, CSH Press, Cold Spring Harbor, NY, USA; Methods in Yeast Genetics, A Laboratory Course Manual, Cold Spring Harbor Laboratory Press, 1990. The host cell is cultured in nutrient media meeting the requirements of the particular host cell used, in particular in respect of the pH value, temperature, salt concentration, aeration, antibiotics, vitamins, trace elements etc.

As mentioned above, the enzymatic conversion of 3-methylcrotonic acid into isobutene utilizing an MDC is preferably performed in the presence of an FMN prenyl transferase and relies on a reaction of two consecutive steps catalyzed by the two enzymes, i.e., the MDC (catalyzing the actual decarboxylation of 3-methylcrotonic acid into isobutene) with an FMN prenyl transferase which provides the modified flavin cofactor. The flavin cofactor may preferably be FMN or FAD. FMN (flavin mononucleotide; also termed riboflavin-5'-phosphate) is a biomolecule produced from riboflavin (vitamin B2) by the enzyme riboflavin kinase and functions as prosthetic group of various reactions. FAD (flavin adenine dinucleotide) is a redox cofactor, more specifically a prosthetic group, involved in several important reactions in metabolism. Thus, in a preferred embodiment, when producing isobutene from 3-methylcrotonic acid comprising the step of incubating an MDC variant of the invention with 3-methylcrotonic acid an FMN prenyl transferase is present which, in a first step, modifies a flavin cofactor (FMN or FAD) into a (modified) flavin-derived cofactor. FMN prenyl transferase prenylates the flavin ring of the flavin cofactor (FMN or FAD) into a (modified) prenylated flavin cofactor. This reaction is schematically illustrated in **Figure 1A****.**

In a second step, the actual conversion of 3-methylcrotonic acid into isobutene is catalyzed by said MDC variant via a 1,3-dipolar cycloaddition based mechanism wherein said FMN-dependent decarboxylase uses the prenylated flavin cofactor (FMN or FAD) provided by the associated FMN prenyl transferase. This reaction is schematically illustrated in **Figure 1B****.**

Thus, preferably, the host cell of the present invention is a cell which expresses an FMN prenyl transferase capable of modifying a flavin cofactor (FMN or FAD) into a (modified) flavin-derived cofactor. In a preferred embodiment, the host cell is a cell which naturally (endogenously) expresses an FMN prenyl transferase. In another preferred embodiment, the host cell is a cell which recombinantly expresses an FMN prenyl transferase by, e.g., introducing a nucleic acid molecule encoding an FMN prenyl transferase or a vector comprising such a nucleic acid molecule.

In a preferred embodiment, said FMN prenyl transferase which modifies the flavin cofactor (FMN or FAD) into a (modified) flavin-derived cofactor is a phenylacrylic acid decarboxylase (PAD)-type protein, or the closely related prokaryotic enzyme UbiX, an enzyme which is involved in ubiquinone biosynthesis in prokaryotes.

In Escherichia coli, the protein UbiX (also termed 3-octaprenyl-4-hydroxybenzoate carboxy-lyase) has been shown to be involved in the third step of ubiquinone biosynthesis.

It catalyses the reaction 3-octaprenyl-4-hydroxybenzoate ⇄ 2-octaprenylphenol + CO₂.

Thus, in a preferred embodiment, the modification of a flavin cofactor (FMN or FAD) into the corresponding (modified) flavin-derived cofactor is catalyzed by the FMN-containing protein phenylacrylic acid decarboxylase (PAD). The enzymes involved in the modification of the flavin cofactor (FMN or FAD) into the corresponding modified flavin-derived cofactor were initially annotated as decarboxylases ( EC 4.1.1.-). Some phenylacrylic acid decarboxylases (PAD) are now annotated as flavin prenyl transferases as EC 2.5.1.-.

In a more preferred embodiment, the conversion of 3-methylcrotonic acid into isobutene makes use of a phenylacrylic acid decarboxylase (PAD)-type protein as the FMN prenyl transferase which modifies a flavin cofactor (FMN or FAD) into the corresponding (modified) flavin-derived cofactor wherein said phenylacrylic acid decarboxylase (PAD)-type protein is derived from Candida albicans (Uniprot accession number Q5A8L8), Aspergillus niger (Uniprot accession number A3F715), Saccharomyces cerevisiae (Uniprot accession number P33751) or Cryptococcus gattii (Uniprot accession number E6R9Z0).

In a preferred embodiment, the phenylacrylic acid decarboxylase (PAD)-type protein employed in the method of the present invention is a phenylacrylic acid decarboxylase (PAD)-type protein derived from Candida albicans (Uniprot accession number Q5A8L8; SEQ ID NO:3), Aspergillus niger (Uniprot accession number A3F715; SEQ ID NO:4), Saccharomyces cerevisiae (Uniprot accession number P33751; SEQ ID NO:5) or Cryptococcus gattii (Uniprot accession number E6R9Z0; SEQ ID NO:6) having the amino acid sequence as shown in SEQ ID NO:3, SEQ ID NO:4, SEQ ID NO:5 and SEQ ID NO:6, respectively.

The phenylacrylic acid decarboxylase (PAD)-type protein may preferably be an enzyme comprising an amino acid sequence selected from the group consisting of SEQ ID NOs: 3 to 6 or a sequence which is at least n % identical to any of SEQ ID NOs: 3 to 6 with n being an integer between 10 and 100, preferably 10, 15, 20, 25, 30, 35, 40, 45, 50, 55, 60, 65, 70, 75, 80, 85, 90, 91, 92, 93, 94, 95, 96, 97, 98 or 99 and wherein the enzyme has the enzymatic activity of modifying a flavin cofactor (FMN or FAD) into the corresponding (modified) flavin-derived cofactor.

As regards the determination of sequence identity, the same applies as has been set forth above.

In another preferred embodiment, the modification of a flavin cofactor (FMN or FAD) into the corresponding (modified) flavin-derived cofactor is catalyzed by the FMN-containing protein 3-octaprenyl-4-hydroxybenzoate carboxy-lyase also termed UbiX (initially annotated EC 4.1.1.-). As mentioned above, the enzymes involved in the modification of the flavin cofactor (FMN or FAD) into the corresponding modified flavin-derived cofactor were initially annotated as decarboxylases. Some phenylacrylic acid decarboxylases (PAD) are now annotated as flavin prenyl transferases as EC 2.5.1.-. In a more preferred embodiment, the conversion of 3-methylcrotonic acid into isobutene makes use of a 3-octaprenyl-4-hydroxybenzoate carboxy-lyase (also termed UbiX) as the FMN prenyl transferase which modifies the flavin cofactor (FMN or FAD) into the corresponding (modified) flavin-derived cofactor wherein said 3-octaprenyl-4-hydroxybenzoate carboxy-lyase (also termed UbiX) is derived from Escherichia coli (Uniprot accession number P0AG03), Bacillus subtilis (Uniprot accession, number A0A086WXG4), Pseudomonas aeruginosa (Uniprot accession number A0A072ZCW8) or Enterobacter sp. DC4 (Uniprot accession number W7P6B1).

In an even more preferred embodiment, the 3-octaprenyl-4-hydroxybenzoate carboxy-lyase (also termed UbiX) employed in the method of the present invention is a 3-octaprenyl-4-hydroxybenzoate carboxy-lyase (also termed UbiX) derived from Escherichia coli (Uniprot accession number P0AG03; SEQ ID NO:2), Bacillus subtilis (Uniprot accession, number A0A086WXG4; SEQ ID NO:7), Pseudomonas aeruginosa (Uniprot accession number A0A072ZCW8; SEQ ID NO:8) or Enterobacter sp. DC4 (Uniprot accession number W7P6B1; SEQ ID NO:9) having the amino acid sequence as shown in SEQ ID NO:2, SEQ ID NO:7, SEQ ID NO:8 and SEQ ID NO:9, respectively.

The 3-octaprenyl-4-hydroxybenzoate carboxy-lyase may preferably be an enzyme comprising an amino acid sequence selected from the group consisting of SEQ ID NOs: 2 and 7 to 9 or a sequence which is at least n % identical to any of SEQ ID NOs: 2 and 7 to 9 with n being an integer between 10 and 100, preferably 10, 15, 20, 25, 30, 35, 40, 45, 50, 55, 60, 65, 70, 75, 80, 85, 90, 91, 92, 93, 94, 95, 96, 97, 98 or 99 and wherein the enzyme has the enzymatic activity of modifying a flavin cofactor (FMN or FAD) into the corresponding (modified) flavin-derived cofactor. As regards the determination of the sequence identity, the same applies as has been set forth above.

The present invention also relates to a method for producing isobutene from 3-methylcrotonic acid comprising the step of incubating an MDC variant of the invention with 3-methylcrotonic acid under conditions allowing said conversion (preferably further in the presence of an FMN prenyl transferase as described above) or comprising the step of culturing a host cell of the present invention expressing an MDC variant (and preferably further expressing an FMN prenyl transferase as described above) in a suitable medium and recovering the produced isobutene.

It is also conceivable in this context that in such a method not only one enzyme according to the present invention is employed but a combination of two or more enzymes.

The present invention also relates to the use of an MDC variant or a host cell of the present invention as described above for the conversion of 3-methylcrotonic acid into isobutene, preferably in the presence of an FMN prenyl transferase or in the presence of a host co-expressing an FMN prenyl transferase as described herein above. Moreover, in a further embodiment, the present invention relates to a method for producing isobutene from 3-methylcrotonic acid by bringing 3-methylcrotonic acid into contact with the MDC variant of the present invention, preferably in the presence of an FMN prenyl transferase, or with a host cell comprising a nucleic acid molecule encoding the MDC variant of the present invention, wherein said host cell preferably expresses an FMN prenyl transferase. Thus, in a preferred embodiment, the present invention relates to a method for converting 3-methylcrotonic acid into isobutene comprising the steps of: (i) culturing the above-described host cell of the invention in a suitable medium; and (ii) achieving the production of isobutene from 3-methylcrotonic acid.

Thus, in a preferred embodiment, the present invention relates to methods and uses utilizing a host cell of the present invention which expresses an MDC variant of the present invention and, preferably, further expressing an FMN prenyl transferase as described herein above.

In another preferred embodiment, such a host cell is an organism which is capable of producing 3-methylcrotonic acid.

In another preferred embodiment, the method according to the invention is carried out in culture, in the presence of an organism, preferably a microorganism, producing an enzyme variant of the present invention and, preferably, also producing an FMN prenyl transferase. In such an embodiment of the invention, an organism, preferably a microorganism, that produces an enzyme of the present invention and, preferably, also producing an FMN prenyl transferase, is used. In a preferred embodiment, the (micro)organism is recombinant in that the enzyme produced by the host is heterologous relative to the production host. The method can thus be carried out directly in the culture medium, without the need to separate or purify the enzymes. In an especially advantageous manner, a (micro)organism is used having the natural or artificial property of endogenously producing 3-methylcrotonic acid so as to produce isobutene directly from the substrate already present in the culture in solution.

In connection with the above described methods and uses, the microorganisms are cultivated under suitable culture conditions allowing the occurrence of the enzymatic reaction of the MDC variants of the present invention (and, preferably, also the FMN prenyl transferases as described above). The specific culture conditions depend on the specific microorganism employed but are well known to the person skilled in the art. The culture conditions are generally chosen in such a manner that they allow the expression of the genes encoding the MDC variant of the present invention (and, preferably, also an FMN prenyl transferase as described above). Various methods are known to the person skilled in the art in order to improve and fine-tune the expression of certain genes at certain stages of the culture such as induction of gene expression by chemical inducers or by a temperature shift.

In another embodiment, the above described methods of the invention comprise the step of providing the organism, preferably the microorganism carrying the respective enzyme activity or activities in the form of a (cell) culture, preferably in the form of a liquid cell culture, a subsequent step of cultivating the organism, preferably the microorganism in a fermenter (often also referred to a bioreactor) under suitable conditions allowing the expression of the respective enzyme and further comprising the step of effecting an enzymatic conversion of a method of the invention as described herein above. Suitable fermenter or bioreactor devices and fermentation conditions are known to the person skilled in the art. A bioreactor or a fermenter refers to any manufactured or engineered device or system known in the art that supports a biologically active environment. Thus, a bioreactor or a fermenter may be a vessel in which a chemical/biochemical process like the method of the present invention is carried out which involves organisms, preferably microorganisms and/or biochemically active substances, i.e., the enzyme(s) described above derived from such organisms or organisms harbouring the above described enzyme(s). In a bioreactor or a fermenter, this process can either be aerobic or anaerobic. These bioreactors are commonly cylindrical, and may range in size from litres to hundreds of cubic meters, and are often made of stainless steel. In this respect, without being bound by theory, the fermenter or bioreactor may be designed in a way that it is suitable to cultivate the organisms, preferably microorganisms, in, e.g., a batch-culture, feed-batch-culture, perfusion culture or chemostate-culture, all of which are generally known in the art. The culture medium can be any culture medium suitable for cultivating the respective organism or microorganism.

In yet a further embodiment, the method according to the invention can be carried out *in vitro,* e.g. in the presence of isolated enzyme or of cell lysates comprising the enzyme or partially purified enzyme preparations comprising the MDC variant of the present invention (and, preferably, also an FMN prenyl transferase as described above). In vitro preferably means in a cell-free system.

In one embodiment, the enzyme(s) employed in the method is (are) used in purified form. However, such a method may be costly, since enzyme and substrate production and purification costs are high.

Thus, in another preferred embodiment, the enzymes employed in the method are present in the reaction as a non-purified extract, or else in the form of non-lysed bacteria, so as to economize on protein purification costs. However, the costs associated with such a method may still be quite high due to the costs of producing and purifying the substrates.

In an *in vitro* reaction the enzymes, native or recombinant, purified or not, are incubated in the presence of the substrate in physicochemical conditions allowing the enzymes to be active, and the incubation is allowed to proceed for a sufficient period of time allowing production of the desired product as described above. At the end of the incubation, one optionally measures the presence of isobutene by using any detection system known to one of skill in the art such as gas chromatography or colorimetric tests for measuring the formation of isobutene.

In a particularly preferred embodiment of the invention the method is carried out *in vitro* and the enzyme is immobilized. Means and methods for immobilizing enzymes on different supports are well-known to the person skilled in the art.

The method according to the invention furthermore comprises the step of collecting gaseous products, i.e. isobutene, degassing out of the reaction, i.e. recovering the product which degasses, e.g., out of the culture. Thus, in a preferred embodiment, the method is carried out in the presence of a system for collecting isobutene under gaseous form during the reaction.

As a matter of fact, isobutene adopts the gaseous state at room temperature and atmospheric pressure. Moreover, isobutene also adopts the gaseous state under culture conditions at 37 °C. The method according to the invention therefore does not require extraction of isobutene from the liquid culture medium, a step which is always very costly when performed at industrial scale. The evacuation and storage of gaseous hydrocarbons, in particular of isobutene, and their possible subsequent physical separation and chemical conversion can be performed according to any method known to one of skill in the art.

Finally, the present invention relates to a composition comprising a variant of an MDC of the present invention, a nucleic acid molecule of the present invention, a vector of the present invention or a host cell of the present invention. As regards the variant of an MDC, the nucleic acid molecule, the vector or the host cell, the same applies as has been set forth above in connection with the methods according to the present invention.
- **Figure 1A:**: shows a schematic reaction of the enzymatic prenylation of a flavin mononucleotide (FMN) into the corresponding modified (prenylated) flavin cofactor.
- **Figure 1B:**: Schematic reaction of the enzymatic conversion of 3-methylcrotonic acid into isobutene.
- **Figure 2:**: Isobutene (IBN) production from 30 mM 3-methylcrotonic acid (3MC) after two hours of incubation with two 3-methylcrotonic acid decarboxylase (3-MDC) enzymes. Ss5: enzyme from *Streptomyces sp.* 769 (Uniprot Accession Number A0A0A8EV26); Ha: enzyme from *Hypocrea atroviridis* (Uniprot Accession Number G9NLP8). Tests are conducted in 384 or 96 microplates (DW384 and DW96), *in vitro* and *in vivo.*
- **Figure 3:**: Isobutene (IBN) production from 30 mM 3-methylcrotonic acid (3MC) after two hours of incubation with two 3-methylcrotonic acid decarboxylase (3MDC) enzymes (Ss5 and Ha) fused with a 6-His-tag. Ss5: enzyme from *Streptomyces sp. 769* (Uniprot Accession Number A0A0A8EV26); Ha: enzyme from *Hypocrea atroviridis* (Uniprot Accession Number G9NLP8). Tests are conducted in 384 or 96 microplates (DW384 and DW96), *in vitro* and *in vivo.*

The invention will now be described by reference to the following examples which are merely illustrative and are not to be construed as a limitation of the scope of the present invention.

### Examples

### Material and Methods: Methods used to assess isobutene production activities

### a) Cloning of 3-methylcrotonic acid decarboxylase (3-MDC) enzymes

All the 3-methylcrotonic acid decarboxylase (3-MDC) polynucleotide sequences were codon-optimized for the expression in *Escherichia coli* and subsequently chem ically synthesized. They were then cloned in a pET25 (Novagen) expression vector or fused with a polynucleotide tag in 5' coding for a 6-His purification tag before being cloned in a pET25 expression vector, resulting in 2 expression vectors for each 3-MDC sequence.

### b) Construction of 3-methylcrotonic acid decarboxylase (3-MDC) mutants

The polynucleotide sequences coding for the different mutants identified during the evolution of the selected 3-MDC enzymes were generated using a range of standard molecular biology techniques.

Different PCR-based techniques known in the art were used for the construction of single-point mutants. For the generation of enzyme variants bearing multiple mutations (at least two mutations), either PCR-based techniques or other methods known in the art were used to introduce these mutations.

Following mutagenesis, the mutated polynucleotide sequence was inserted into a pET25 expression vector with or without being fused to a tag as described above either using standard ligase-based subcloning techniques, whole plasmid extension by PCR or ligase-independent cloning techniques.

### c) Screening of 3-methylcrotonic acid decarboxylase (3-MDC) enzymes activity

A total of four different screening methods were developed and used during the evolution of the *Streptomyes sp. 769* MDC enzyme.

### c1) In vivo assay in 384-well microplates based on exogenous 3-methylcrotonic acid (3MC) (VIVO384)

This assay is based on the use of a bacterial strain (BL21(DE3), Novagen) transformed with two expression vectors leading to the production of the last two enzymes involved in the metabolic pathway converting 3MC to isobutene; namely the prenylated FMN Synthase UbiX protein from *E. coli* cloned in a pRSFDuet^{™} (Novagen) expression vector and the respective 3-MDC enzyme cloned in one of the above expression vectors. This strain is first plated out onto LB-agar plates supplemented with the appropriate antibiotic. Cells were grown overnight at 32°C until individual colonies reach the desired size. Single colonies were then picked and individually transferred into 50µL of liquid LB medium supplemented with the appropriate antibiotics. Cell growth is carried out with shaking for 20 hours at 32°C. The LB cultures were used to inoculate 300 µL in 384 deep well microplates of auto-induction medium (Studier FW, Prat.Exp.Pur. 41, (2005), 207-234) supplemented with the appropriate antibiotics and grown in a shaking incubator set at 700rpm and 85% humidity for 24h at 32°C in order to produce the two types of recombinant enzymes. The cell pellet containing these two overexpressed recombinant enzymes is then resuspended in 40 µL of minimum medium (pH 7.5, Phosphate 100 mM, Glucose 10g.L⁻¹, MgSO₄ 1mM) supplemented with 3, 10 or 30 mM 3MC and incubated for a further 2 or 4 hours in a shaking incubator at 36°C, 700 rpm. During this step, the 3-MDC enzyme catalyzes the decarboxylation of 3MC into IBN. After 5 min inactivation at 80°C, the IBN produced is quantified by gas chromatography as followed. 100 µL of headspace gases from each enzymatic reaction are injected in a Brucker GC-450 system equipped with a Flame Ionization Detector (FID). Compounds present in samples were separated by chromatography using a RTX-1 columns at 100°C with a 1 mL.min⁻¹ constant flow of nitrogen as carrier gas. Upon injection, peak areas of isobutene were calculated.

### c2) In vivo assay in 96-well microplates based on exogenous 3-methylcrotonic acid (3MC) (VIVO96)

This assay is based on the use of a bacterial strain (BL21(DE3), Novagen) transformed with two expression vectors leading to the production of the last two enzymes involved in the metabolic pathway converting 3MC to isobutene; namely the prenylated FMN Synthase UbiX protein from *E. coli* cloned in a pRSFDuet^{™} (Novagen) expression vector and the respective 3-MDC enzyme cloned in one of the above expression vectors. This strain is first plated out onto LB-agar plates supplemented with the appropriate antibiotics. Cells were grown overnight at 32°C until individual colonies reach the desired size. Single colonies were then picked and individually transferred into 50µL of liquid LB medium supplemented with the appropriate antibiotics. Cell growth is carried out with shaking for 20 hours at 32°C. The LB cultures were used to inoculate 1 mL in 96 deep well microplates of auto-induction medium (Studier FW, Prat.Exp.Pur. 41, (2005), 207-234) supplemented with the appropriate antibiotics and grown in a shaking incubator set at 700rpm and 85% humidity for 24h at 32°C in order to produce the two types of recombinant enzymes. The cell pellet containing these two overexpressed recombinant enzymes is then resuspended in 400 µL of minimum medium (pH 7.5, Phosphate 100 mM, Glucose 10g.L⁻¹, MgSO₄ 1mM) supplemented with 3, 10 or 30 mM 3MC and incubated for a further 2 or 4 hours in a shaking incubator at 36°C, 700 rpm. During this step, the 3-MDC enzyme catalyzes the decarboxylation of 3MC into IBN. After 5 min inactivation at 80°C, the IBN produced is quantified by gas chromatography as followed. 100 µL of headspace gases from each enzymatic reaction are injected in a Brucker GC-450 system equipped with a Flame Ionization Detector (FID). Compounds present in samples were separated by chromatography using a RTX-1 columns at 100°C with a 1 mL.min⁻¹ constant flow of nitrogen as carrier gas. Upon injection, peak areas of isobutene were calculated.

### c3) In vitro assay in 384-well microplates based on exogenous 3-methylcrotonic acid (3MC) (VITRO384)

This assay is based on the use of a bacterial strain (BL21(DE3), Novagen) transformed with two expression vectors leading to the production of the last two enzymes involved in the metabolic pathway converting 3MC to isobutene; namely the prenylated FMN Synthase UbiX protein from *E. coli* cloned in a pRSFDuet^{™} (Novagen) expression vector and the respective 3-MDC enzyme cloned in one of the above expression vectors. This strain is first plated out onto LB-agar plates supplemented with the appropriate antibiotics. Cells were grown overnight at 32°C until individual colonies reach the desired size. Single colonies were then picked and individually transferred into 50µL of liquid LB medium supplemented with the appropriate antibiotic. Cell growth is carried out with shaking for 20 hours at 32°C. The LB cultures were used to inoculate 300 µL in 384 deep well microplates of auto-induction medium (Studier FW, Prat.Exp.Pur. 41, (2005), 207-234) supplemented with the appropriate antibiotic and grown in a shaking incubator set at 700rpm and 85% humidity for 24h at 32°C in order to produce the two types of recombinant enzymes. The cell pellet containing these two overexpressed recombinant enzymes is then resuspended in 30 µL of lysis mix (pH 7.5, Phosphate 50 mM, NaCl 20 mM, MgCl₂ 2 mM, Lysozyme 1 mg/mL, DNAse 0.03 mg/mL) and incubated for 1 hour in a shaking incubator at 36°C, 700 rpm. The mix is then supplemented with 10µL of reaction mix (final composition: pH 7.5, Phosphate 50 mM, NaCl 20 mM, MgCl₂ 2 mM, Lysozyme 0.75 mg/mL, DNAse 0.0225 mg/mL, KCl 100mM) supplemented with 3, 10 or 30 mM (final) 3MC and incubated for a further 2 or 4 hours in a shaking incubator at 36°C, 700 rpm. During this step, the 3-MDC enzyme catalyzes the decarboxylation of 3MC into IBN. After 5 min inactivation at 80°C, the IBN produced is quantified by gas chromatography as followed. 100 µL of headspace gases from each enzymatic reaction are injected in a Brucker GC-450 system equipped with a Flame Ionization Detector (FID). Compounds present in samples were separated by chromatography using a RTX-1 columns at 100°C with a 1 mL.min⁻¹ constant flow of nitrogen as carrier gas. Upon injection, peak areas of isobutene were calculated.

### c4) In vitro assay in 96-well microplates based on exogenous 3-methylcrotonic acid (3MC) (VITRO96)

This assay is based on the use of a bacterial strain (BL21(DE3), Novagen) transformed with two expression vectors leading to the production of the last two enzymes involved in the metabolic pathway converting 3MC to isobutene; namely the prenylated FMN Synthase UbiX protein from *E. coli* cloned in a pRSFDuet^{™} (Novagen) expression vector and the respective 3-MDC enzyme cloned in one of the above expression vectors. This strain is first plated out onto LB-agar plates supplemented with the appropriate antibiotics. Cells were grown overnight at 32°C until individual colonies reach the desired size. Single colonies were then picked and individually transferred into 50µL of liquid LB medium supplemented with the appropriate antibiotic. Cell growth is carried out with shaking for 20 hours at 32°C. The LB cultures were used to inoculate 1 mL in 96 deep well microplates of auto-induction medium (Studier FW, Prat.Exp.Pur. 41, (2005), 207-234) supplemented with the appropriate antibiotic and grown in a shaking incubator set at 700rpm and 85% humidity for 24h at 32°C in order to produce the two types of recombinant enzymes. The cell pellet containing these two overexpressed recombinant enzymes is then resuspended in 150 µL of lysis mix (pH 7.5, Phosphate 50 mM, NaCl 20 mM, MgCl₂ 2 mM, Lysozyme 1 mg/mL, DNAse 0.03 mg/mL) and incubated for 1 hour in a shaking incubator at 36°C, 700 rpm. The mix is then supplemented with 50µL of reaction mix (final composition: pH 7.5, Phosphate 50 mM, NaCl 20 mM, MgCl₂ 2 mM, Lysozyme 0.75 mg/mL, DNAse 0.0225 mg/mL, KCl 100mM) supplemented with 3, 10 or 30 mM (final) 3MC and incubated for a further 2 or 4 hours in a shaking incubator at 36°C, 700 rpm. During this step, the 3-MDC enzyme catalyzes the decarboxylation of 3MC into IBN. After 5 min inactivation at 80°C, the IBN produced is quantified by gas chromatography as followed. 100 µL of headspace gases from each enzymatic reaction are injected in a Brucker GC-450 system equipped with a Flame Ionization Detector (FID). Compounds present in samples were separated by chromatography using a RTX-1 columns at 100°C with a 1 mL.min⁻¹ constant flow of nitrogen as carrier gas. Upon injection, peak areas of isobutene were calculated.

### Example 1: Identification of a Streptomyces sp. 769 UbiD-like decarboxylase enzyme presenting an activity for the reaction of conversion of 3-methylcrotonic acid (3MC) into isobutene (IBN)

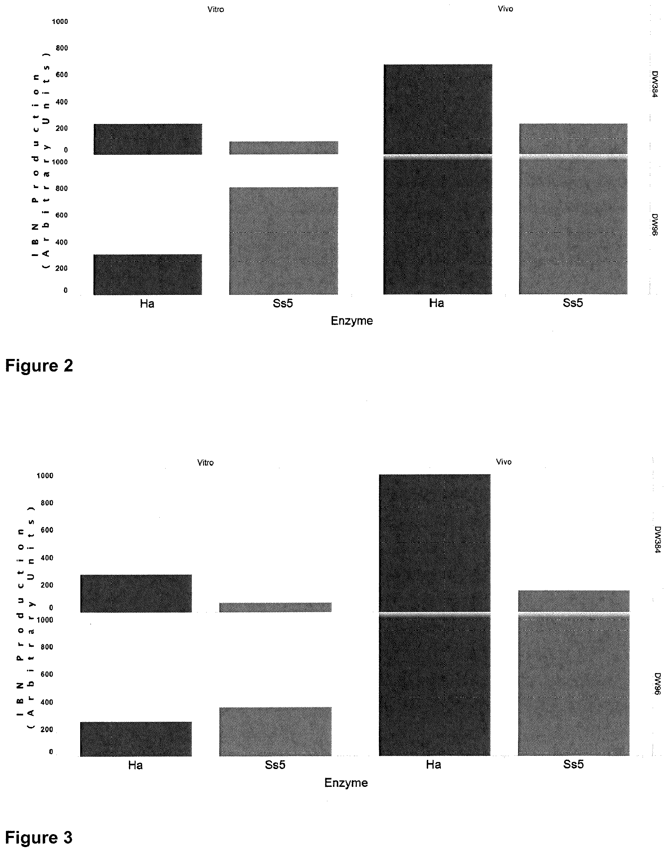

We tested a UbiD-like decarboxylase enzyme from *Streptomyces sp. 769* (UniProt ID A0A0A8EV26, gene GZL_07100, herein called Ss5; SEQ ID NO:1) for its capacity to catalyze the reaction of conversion of 3MC into IBN. The gene was codon-optimized for expression in *Escherichia Coli,* synthesized, fused to a 6-His-tag or not fused to a 6-His-tag, and then subcloned into a pET25 expression vector, as described above. The expression vectors were then screened for the production of isobutene (IBN) from 3-methylcrotonic acid (3MC) in microplate 96 and 384-wells, using both an *in vivo* and an *in vitro* assay, as described above. An 3-methylcrotonic acid decarboxylase (3-MDC) enzyme from *Hypocrea atroviridis* (herein called Ha, Uniprot Accession Number G9NLP8) previously described in WO2017085167 (see Example 8, Table G; example 12 Table J therein) and in WO2017191239 (SEQ ID NO:1 therein) was added as a control **(****Figure 2** and **Figure 3**).

### Example 2: Identification of variants of a 3-methylcrotonic acid decarboxylase (3-MDC) enzyme from Streptomyces sp. 769 with increased activity for the reaction of conversion of 3-methylcrotonic acid (3MC) into isobutene (IBN)

The above 3-methylcrotonic acid decarboxylase (3-MDC) gene presenting a capacity to catalyze the reaction of conversion of 3MC into IBN (UniProt ID A0A0A8EV26, gene GZL_07100 from *Streptomyces sp. 769*) was submitted to directed mutagenesis in order to create single point mutations or multiple mutations variants. Each of these variants was subsequently tested for their increased activity to convert 3MC into IBN, using one or more of the previously described assays. 230 variants presented an increased capacity in converting 3MC into IBN.

The list of improved variants is presented in the following **Table 1.** The increase in activity is described relative to the wild-type enzyme (with "+" representing a low increase in activity and "+++" representing a high increase in activity).

The list of mutations involved in the variants of the *Streptomyces sp. 769* 3-MethylCrotonate Decarboxylase with increased activity is described in **Table 2.**

**Table 1.**

| **List of *Streptomyces sp.* 769 3-MethylCrotonate Decarboxylase variants presenting an increase in isobutene production from 3-methylcrotonic acid.** | |
|---|---|
| **Mutants** | **Activity relative to WT** |
| A241D | + |
| A241N | + |
| A241V | + |
| A359C | + |
| C404F | + |
| C404L | + |
| L448F | + |
| L448W | + |
| L448Y | + |
| P406A | + |
| R390S-L448W | + |
| R390S-L448Y | + |
| A241D-C404F | ++ |
| A241D-C404F-L448F | ++ |
| A241D-C404F-L448Y | ++ |
| A241D-C404F-P406A | ++ |
| A241D-C404F-P406A-L448F | ++ |
| A241D-C404F-P406A-L448Y | ++ |
| A241D-C404L | ++ |
| A241D-C404L-L448F | ++ |
| A241D-C404L-L448W | ++ |
| A241D-C404L-L448Y | ++ |
| A241D-C404L-P406A | ++ |
| A241D-C404L-P406A-L448F | ++ |
| A241D-C404L-P406A-L448Y | ++ |
| A241D-L448F | ++ |
| A241D-L448W | ++ |
| A241D-L448Y | ++ |
| A241D-P406A | ++ |
| A241D-P406A-L448F | ++ |
| A241D-P406A-L448Y | ++ |
| A241N-C404F | ++ |
| A241N-C404F-L448F | ++ |
| A241N-C404F-L448W | ++ |
| A241N-C404F-L448Y | ++ |
| A241N-C404F-P406A | ++ |
| A241N-C404F-P406A-L448F | ++ |
| A241N-C404F-P406A-L448Y | ++ |
| A241N-C404L | ++ |
| A241N-C404L-L448F | ++ |
| A241N-C404L-L448W | ++ |
| A241N-C404L-L448Y | ++ |
| A241N-C404L-P406A | ++ |
| A241N-C404L-P406A-L448F | ++ |
| A241N-C404L-P406A-L448Y | ++ |
| A241N-L448F | ++ |
| A241N-L448W | ++ |
| A241N-L448Y | ++ |
| A241N-P406A | ++ |
| A241N-P406A-L448F | ++ |
| A241N-P406A-L448W | ++ |
| A241N-P406A-L448Y | ++ |
| C404F-L4461-L448W | ++ |
| C404F-L448F | ++ |
| C404F-L448W | ++ |
| C404F-L448Y | ++ |
| C404F-P406A | ++ |
| C404F-P406A-L448F | ++ |
| C404F-P406A-L448W | ++ |
| C404F-P406A-L448Y | ++ |
| C404L-L448F | ++ |
| C404L-L448W | ++ |
| C404L-L448Y | ++ |
| C404L-P406A | ++ |
| C404L-P406A-L448F | ++ |
| C404L-P406A-L448Y | ++ |
| C404M-L446I-L448W | ++ |
| C404M-L448F | ++ |
| C404M-L448W | ++ |
| P406A-L448F | ++ |
| P406A-L448W | ++ |
| P406A-L448Y | ++ |
| V240I-C404M-L448F | ++ |
| C404M-L446N-L448W | ++ |
| C404M-P406A-L446I-L448W | ++ |
| C404M-P406S-L446I-L448W | ++ |
| S403R-C404M-L446I-L448W | ++ |
| C404M-L446I-L448W-T450A | ++ |
| C404M-L446I-L448W-T450H | ++ |
| C404M-L446I-L448W-T450M | ++ |
| C404M-L446I-L448W-T450N | ++ |
| C404M-L446I-L448W-T450S | ++ |
| C404M-P406A-L446N-L448W | ++ |
| C404M-P406A-L448W | ++ |
| C404M-P406A-L446I-L448W-T450A | ++ |
| C404M-P406S-L446N-L448W | ++ |
| C404M-P406S-L448W | ++ |
| C404M-P406S-L446I-L448W-T450A | ++ |
| C404M-P406S-L446I-L448W-T450H | ++ |
| C404M-L446N-L448W-T450A | ++ |
| C404M-L446N-L448W-T450H | ++ |
| C404M-L448W-T450A | ++ |
| C404M-L448W-T450H | ++ |
| C404M-P406A-L446N-L448W-T450A | ++ |
| C404M-P406A-L448W-T450A | ++ |
| C404M-P406A-L446N-L448W-T450H | ++ |
| C404M-P406S-L446N-L448W-T450A | ++ |
| C404M-P406S-L448W-T450A | ++ |
| C404M-P406S-L446N-L448W-T450H | ++ |
| C404M-P406A-L4461-L448W-T450H | +++ |
| C404M-P406A-L448W-T450H | +++ |
| C404M-P406S-L448W-T450H | +++ |
| A241D-C404F-L446I-L448W | +++ |
| A241D-C404F-L448W | +++ |
| A241D-C404F-P406A-L448W | +++ |
| A241D-C404F-P406S-L448W | +++ |
| A241D-C404L-P406A-G443D-L448W | +++ |
| A241D-C404L-P406A-L446V-L448W | +++ |
| A241D-C404L-P406A-L446V-L448W-T450M | +++ |
| A241D-C404L-P406A-L448W | +++ |
| A241D-C404L-P406A-L448W-T450M | +++ |
| A241D-C404M-L448W | +++ |
| A241D-C404M-P406A-L448W | +++ |
| A241D-F401Y-C404F-L446I-L448W | +++ |
| A241D-F401Y-C404F-L448W | +++ |
| A241D-F401Y-C404F-P406A-L448W | +++ |
| A241D-F401Y-C404M-L448W | +++ |
| A241D-F401Y-C404M-P406A-L448W | +++ |
| A241D-F401Y-L448W | +++ |
| A241D-F401Y-S403G-C404F-L446I-L448W | +++ |
| A241D-F401Y-S403G-C404F-L448W | +++ |
| A241D-F401Y-S403G-C404M-L448W | +++ |
| A241D-F401Y-S403P-C404F-L446I-L448W | +++ |
| A241D-F401Y-S403P-C404F-L448W | +++ |
| A241D-F401Y-S403P-C404M-P406A-L448W | +++ |
| A241D-G402A-C404L-P406A-L446V-L448W | +++ |
| A241D-G402A-C404L-P406A-L446V-L448W-T450M | +++ |
| A241D-G402A-C404L-P406A-L448W | +++ |
| A241D-G402A-C404L-P406A-L448W-T450M | +++ |
| A241D-G402A-S403C-C404L-P406A-L446V-L448W | +++ |
| A241D-G402A-S403C-C404L-P406A-L446V-L448W-T450M | +++ |
| A241D-G402A-S403C-C404L-P406A-L448W | +++ |
| A241D-G402A-S403C-C404L-P406A-L448W-T450M | +++ |
| A241D-G402A-S403V-C404L-P406A-L446V-L448W | +++ |
| A241D-G402A-S403V-C404L-P406A-L446V-L448W-T450M | +++ |
| A241D-G402A-S403V-C404L-P406A-L448W | +++ |
| A241D-G402A-S403V-C404L-P406A-L448W-T450M | +++ |
| A241D-P406A-L448W | +++ |
| A241D-P444E-L448W | +++ |
| A241D-S403C-C404L-P406A-L446V-L448W | +++ |
| A241D-S403C-C404L-P406A-L446V-L448W-T450M | +++ |
| A241D-S403C-C404L-P406A-L448W | +++ |
| A241D-S403C-C404L-P406A-L448W-T450M | +++ |
| A241D-S403G-C404F-L446I-L448W | +++ |
| A241D-S403G-C404F-L448W | +++ |
| A241D-S403G-C404F-P406A-L448W | +++ |
| A241D-S403G-C404M-P406A-L448W | +++ |
| A241D-S403G-L448W | +++ |
| A241D-S403P-C404F-L446I-L448W | +++ |
| A241D-S403P-C404F-L448W | +++ |
| A241D-S403P-C404F-P406A-L448W | +++ |
| A241D-S403P-C404M-L448W | +++ |
| A241D-S403P-C404M-P406A-L448W | +++ |
| A241D-S403P-L448W | +++ |
| A241D-S403V-C404L-P406A-L446V-L448W | +++ |
| A241D-S403V-C404L-P406A-L446V-L448W-T450M | +++ |
| A241D-S403V-C404L-P406A-L448W | +++ |
| A241D-S403V-C404L-P406A-L448W-T450M | +++ |
| A241N-C404F-L405H-P406A-L448W | +++ |
| A241N-C404F-P406A-L448W | +++ |
| A241N-C404L-P406A-L446C-L448W | +++ |
| A241N-C404L-P406A-L446V-L448W | +++ |
| A241N-C404L-P406A-L448W | +++ |
| A241N-C404L-P406A-L448W-L449I | +++ |
| A241N-C404L-P406A-L448W-T450M | +++ |
| A241N-C404L-P406A-L448W-T450Q | +++ |
| A241N-C404M-L448W | +++ |
| A241N-C404M-P406A-L448W | +++ |
| A241N-F401Y-L448W | +++ |
| A241N-L446V-L448W | +++ |
| A241N-S403C-C404L-P406A-L448W | +++ |
| A241N-S403G-L448W | +++ |
| A241N-S403N-C404L-P406A-L448W | +++ |
| A241N-S403P-L448W | +++ |
| A241N-S403V-C404L-P406A-L448W | +++ |
| C404L-P406A-L446F-L448W | +++ |
| C404L-P406A-L446S-L448W | +++ |
| C404L-P406A-L446V-L448W | +++ |
| C404L-P406A-L448W | +++ |
| C404L-P406A-L448W-T450M | +++ |
| C404L-P406A-P444H-L448W | +++ |
| P85L-A241N-C404M-L448W-T450M | +++ |
| S403V-C404L-P406A-L448W | +++ |
| A241D-S403C-C404L-P406A-G443D-L448W | +++ |
| A241D-S403C-C404L-P406A-G443H-L448W | +++ |
| A241D-S403C-C404L-P406A-G443N-L448W | +++ |
| A241D-S403C-C404L-P406A-L448W-L449I | +++ |
| A241D-S403C-C404L-P406A-P444A-L448W | +++ |
| A241D-S403C-C404L-P406A-P444H-L448W | +++ |
| A241D-S403C-C404L-P406A-P444L-L448W | +++ |
| A241D-G402A-S403C-C404L-P406A-G443H-L448W | +++ |
| A241D-G402A-S403C-C404L-P406A-G443S-L448W | +++ |
| A241D-G402A-S403C-C404L-P406A-L446M-L448W | +++ |
| A241D-G402A-S403C-C404L-P406A-P444F-L448W | +++ |
| A241D-G402A-S403C-C404L-P406A-P444H-L448W | +++ |
| A241D-S403H-C404L-P406A-L448W-T450M | +++ |
| A241D-S403C-C404L-P406A-G443A-L448W-T450M | +++ |
| A241D-S403C-C404L-P406A-G443F-L448W-T450M | +++ |
| A241D-S403C-C404L-P406A-G443Y-L448W-T450M | +++ |
| A241D-S403C-C404L-P406A-P444A-L448W-T450M | +++ |
| A241D-S403C-C404L-P406A-P444F-L448W-T450M | +++ |
| A241D-S403C-C404L-P406A-P444T-L448W-T450M | +++ |
| A241D-S403V-C404L-P406A-G443D-L446V-L448W | +++ |
| A241D-S403V-C404L-P406A-G443D-L448W | +++ |
| A241D-S403V-C404L-P406A-G443A-L448W-T450M | +++ |
| A241D-S403V-C404L-P406A-G443D-L448W-T450M | +++ |
| A241D-S403V-C404L-P406A-G443F-L448W-T450M | +++ |
| A241D-S403V-C404L-P406A-G443N-L448W-T450M | +++ |
| A241D-S403V-C404L-P406A-G443S-L448W-T450M | +++ |
| A241D-S403V-C404L-P406A-V445L-L448W-T450M | +++ |
| A241D-C404L-P406A-G443F-L446V-L448W-T450M | +++ |
| A241D-C404L-P406A-L446V-L448W-T450A | +++ |
| A241D-S403C-C404L-P406A-G443A-L446V-L448W-T450M | +++ |
| A241D-S403C-C404L-P406A-G443S-L446V-L448W-T450M | +++ |
| A241D-S403C-C404L-P406A-G443W-L446V-L448W- T450M | +++ |
| A241D-S403C-C404L-P406A-G443Y-L446V-L448W-T450M | +++ |
| H198Q-A241D-S403C-C404L-P406A-G443F-L446V- L448W-T450M | +++ |
| A241D-S403C-C404L-P406A-P444H-L446V-L448W- T450M | +++ |
| A241D-S403C-C404L-P406A-V408I-L446V-L448W-T450M | +++ |
| A241D-S403C-C404L-P406A-L446M-L448W-T450M | +++ |
| A241D-S403V-C404L-P406A-G443D-L446V-L448W-T450M | +++ |
| A241D-S403V-C404L-P406A-G443F-L446V-L448W-T450M | +++ |
| A241D-S403V-C404L-P406A-G443Y-L446V-L448W-T450M | +++ |
| A241D-S403V-C404L-P406A-L446V-L448W-T450H | +++ |
| A241D-S403A-C404L-P406A-L446V-L448W-T450M | +++ |
| A241D-S403G-C404L-P406A-L446V-L448W-T450M | +++ |
| A241D-S403V-C404L-P406A-V408I-L446V-L448W-T450M | +++ |
| A241D-S403V-C404L-P406A-L446A-L448W-T450M | +++ |
| A241D-S403V-C404L-P406A-L446M-L448W-T450M | +++ |
| C404M-P406A-G443W-L446I-L448W-T450H | +++ |
| C404M-P406A-G443Y-L446I-L448W-T450H | +++ |
| C404M-L405I-P406A-L446I-L448W-T450H | +++ |
| M197V-C404M-P406A-L446I-L448W- T450H | +++ |
| C404M-P406A-Q407K-L446I-L448W-T450H | +++ |
| S403C-C404M-P406A-L446I-L448W-T450H | +++ |
| S403P-C404M-P406A-L446I-L448W-T450H | +++ |
| C404M-L405I-P406A-L448W-T450H | +++ |
| M197V-S403C-C404M-L405I-P406A-G443Y-L448W-T450H | +++ |
| S403C-C404M-P406A-L448W-T450H | +++ |
| M197V-S403C-C404M-L405I-P406A-L448W-T450H | +++ |
| M197V-C404M-L405I-P406A-L446I-L448W-T450H | +++ |
| M 1 97V-C404M-P406A-L448W-T450H | +++ |
| S403C-C404M-L405I-P406A-L448W-T450H | +++ |
| C404M-L405I-P406A-G443Y-L446I-L448W-T450H | +++ |
| M197V-C404M-L405I-P406A-L448W-T450H | +++ |
| M197V-S403C-C404M-P406A-L4461-L448W-T450H | +++ |
| S403C-C404M-L405I-P406A-G443Y-L448W-T450H | +++ |
| C404M-P406A-G443Y-L448W-T450H | +++ |
| S403C-C404M-P406A-G443Y-L446I-L448W-T450H | +++ |
| S403C-C404M-P406A-G443Y-L448W-T450H | +++ |
| S403C-C404M-L405I-P406A-L446I-L448W-T450H | +++ |
| M 197V-C404M-L4051-P406A-G443Y-L4461-L448W-T450H | +++ |
| S403C-C404M-L405I-P406A-G443Y-L446I-L448W-T450H | +++ |
| C404M-L405I-P406A-G443Y-L448W-T450H | +++ |
| M197V-S403C-C404M-L405I-P406A-L446I-L448W-T450H | +++ |
| M197V-C404M-P406A-G443Y-L4461-L448W-T450H | +++ |
| M197V-S403C-C404M-L405I-P406A-G443Y-L446I-L448W- T450H | +++ |
| M 197V-S403C-C404M-P406A-G443Y-L4461-L448W-T450H | +++ |
| A241D-S403C-C404L-P406A-G443D-L448W-L449I | +++ |
| A241D-S403C-C404L-P406A-P444H-L446M-L448W- T450M | +++ |
| A241D-S403C-C404L-P406A-G443D-P444H-L448W-L449I | +++ |
| A241D-S403C-C404L-P406A-G443D-P444A-L448W | +++ |
| A241D-S403C-C404L-P406A-G443Y-P444H-L446M- L448W-T450M | +++ |
| A241D-S403C-C404L-P406A-V408I-P444H-L446M-L448W- T450M | +++ |
| A241D-S403C-C404L-P406A-G443Y-L446M-L448W- T450M | +++ |
| A25E-A241D-S403C-C404L-P406A-L448W | +++ |
| A66H-A241D-S403C-C404L-P406A-L448W | +++ |
| A80S-A241D-S403C-C404L-P406A-L448W | +++ |
| E24A-A241D-S403C-C404L-P406A-L448W | +++ |
| E24D-A241D-S403C-C404L-P406A-L448W | +++ |
| E24L-A241D-S403C-C404L-P406A-L448W | +++ |
| E24V-A241D-S403C-C404L-P406A-L448W | +++ |
| G237D-A241D-S403C-C404L-P406A-L448W | +++ |
| G237P-A241D-S403C-C404L-P406A-L448W | +++ |
| A241D-S403C-C404L-P406A-G443A-L448W | +++ |
| I236L-A241D-S403C-C404L-P406A-L448W | +++ |
| I236V-A241D-S403C-C404L-P406A-L448W | +++ |
| A241D-I296Q-S403C-C404L-P406A-L448W | +++ |
| K214T-A241D-S403C-C404L-P406A-L448W | +++ |
| N64H-A241D-S403C-C404L-P406A-L448W | +++ |
| A241D-S403C-C404L-P406A-L448W-P479C | +++ |
| A241D-S403C-C404L-P406A-L448W-P479I | +++ |
| A241D-S403C-C404L-P406A-L448W-P479L | +++ |
| A241D-S403C-C404L-P406A-L448W-P479M | +++ |
| A241D-S403C-C404L-P406A-L448W-P479S | +++ |
| A241D-S403C-C404L-P406A-L448W-P479T | +++ |
| A241D-S403C-C404L-P406A-L448W-P479V | +++ |
| A241D-S403C-C404L-P406A-L448W-R496L | +++ |
| S175E-A241D-S403C-C404L-P406A-L448W | +++ |
| A241D-S403C-C404L-P406A-L448W-A473S | +++ |
| S86N-A241D-S403C-C404L-P406A-L448W | +++ |
| S86Y-A241D-S403C-C404L-P406A-L448W | +++ |
| V240N-A241D-S403C-C404L-P406A-L448W | +++ |
| V240N-A241D-S403C-C404L-P406A-L448W-P479V | +++ |
| A66H-K214T-G237P-A241D-S403C-C404L-P406A-L448W | +++ |
| K214T-G237P-A241D-I296Q-S403C-C404L-P406A-L448W | +++ |
| K214T-G237P-A241D-I296Q-S403C-C404L-P406A-L448W- P479V | +++ |
| K214T-A241D-S403C-C404L-P406A-L448W-R496L | +++ |
| V240N-A241D-S403C-C404L-P406A-L448W-R496L | +++ |
| K214T-G237P-V240N-A241D-I296Q-S403C-C404L-P406A- L448W | +++ |
| A66H-A241D-S403C-C404L-P406A-L448W-P479V | +++ |
| A66H-K214T-G237P-V240N-A241D-S403C-C404L-P406A- L448W-P479V | +++ |
| A66H-K214T-A241D-S403C-C404L-P406A-L448W-P479V | +++ |
| G237P-A241D-I296Q-S403C-C404L-P406A-L448W-P479V | +++ |
| A66H-K214T-G237P-A241D-S403C-C404L-P406A-L448W- P479V | +++ |
| A241D-1296Q-S403C-C404L-P406A-L448W-P479V | +++ |
| A66H-K214T-A241D-I296Q-S403C-C404L-P406A-L448W | +++ |
| K214T-A241D-S403C-C404L-P406A-G443N-L448W | +++ |
| A66H-A241D-I296Q-S403C-C404L-P406A-L448W | +++ |
| K214T-A241D-I296Q-S403C-C404L-P406A-L448W | +++ |
| G237P-A241D-S403C-C404L-P406A-L448W-P479V | +++ |
| A66H-K214T-A241D-I296Q-S403C-C404L-P406A-L448W-P479V | +++ |
| A66H-G237P-V240N-A241D-S403C-C404L-P406A-L448W- P479V | +++ |
| A66H-G237P-A241D-S403C-C404L-P406A-L448W-P479V | +++ |
| K214T-A241D-1296Q-S403C-C404L-P406A-L448W-P479V | +++ |
| A66H-A241D-1296Q-S403C-C404L-P406A-L448W-P479V | +++ |
| K214T-A241D-S403C-C404L-P406A-G443F-L448W-T450M | +++ |
| K214T-G237P-A241D-S403C-C404L-P406A-L448W-P479V | +++ |
| M197V-A241D-S403C-C404L-P406A-G443F-L448W- T450M | +++ |
| A241D-S403C-C404L-P406A-V408C-G443F-L448W- T450M | +++ |
| A241D-S403C-C404L-P406A-V408L-G443F-L448W-T450M | +++ |
| A241D-S403C-C404L-P406A-G443F-V445L-L448W-T450M | +++ |
| A241D-S403C-C404L-P406A-G443F-V445S-L448W-T450M | +++ |
| A241D-S403C-C404L-P406A-G443F-V445T-L448W-T450M | +++ |
| A241D-S403C-C404L-P406A-G443D-V445T-L448W | +++ |
| M197V-A241D-S403C-C404L-P406A-G443D-P444A- L448W | +++ |
| A241D-S403C-C404L-L405I-P406A-G443D-L448W-L449I | +++ |
| M197V-A241D-S403C-C404L-P406A-G443D-L448W-L449I | +++ |
| A241D-S403C-C404L-P406A-G443D-V445L-L448W-L449I | +++ |
| A241D-S403C-C404L-P406A-G443A-P444H-L448W-L449I | +++ |
| A241D-S403C-C404L-P406A-G443F-P444H-L448W-L449I | +++ |
| A241D-S403C-C404L-P406A-G443L-P444H-L448W-L449I | +++ |
| A241D-S403C-C404L-P406A-G443S-P444H-L448W-L449I | +++ |
| A241D-S403C-C404L-P406A-G443D-P444H-L448W | +++ |
| A241D-S403C-C404L-P406A-G443D-P444H-L448W-L449V | +++ |
| M197V-A241D-S403C-C404L-P406A-G443D-P444H- L448W-L449I | +++ |
| A241D-C404L-L405M-P406A-G443F-L446V-L448W- L449M-T450M | +++ |
| A241D-S403Q-C404L-P406A-G443F-L446V-L448W-T450M | +++ |
| A241D-S403C-C404L-P444H-L448W-T450M | +++ |
| A241D-S403C-C404L-P406S-P444H-L448W-T450M | +++ |
| L195I-A241D-S403C-C404L-P406A-P444H-L448W-T450M | +++ |
| M197V-A241D-S403C-C404L-P406A-P444H-L448W- T450M | +++ |
| A241D-S403C-C404L-P406A-Q407R-P444H-L448W- T450M | +++ |
| A241D-S403C-C404L-P406A-Q407S-P444H-L448W- T450M | +++ |
| A241D-S403C-C404L-P406A-V408I-P444H-L448W-T450M | +++ |
| L195I-A241D-S403C-C404L-P406A-V408I-P444H-L446M- L448W-T450M | +++ |
| M197V-A241D-S403C-C404L-P406A-V408I-P444H-L446M- L448W-T450M | +++ |
| A241D-S403C-C404L-P406A-V408I-P444H-V445C-L446M- L448W-T450M | +++ |
| M197V-A241D-S403C-C404L-P406A-G443Y-P444H- L446M-L448W-T450M | +++ |
| M197V-A241D-S403C-C404L-P406A-G443D-P444H- L448W-L449V | +++ |
| M197V-A241D-S403C-C404L-P406A-G443S-P444H- L448W-L449I | +++ |
| A241D-E339H-S403C-C404L-P406A-G443D-P444H- L448W-L449I | +++ |
| M197V-S403C-C404M-P406A-G443Y-L446I-L448W- T450H-Q455H | +++ |
| S403A-C404M-P406A-G443Y-L446I-L448W-T450H | +++ |
| S403I-C404M-P406A-G443Y-L446I-L448W-T450H | +++ |
| S403Q-C404M-P406A-G443Y-L446I-L448W-T450H | +++ |
| S403V-C404M-P406A-G443Y-L446I-L448W-T450H | +++ |
| S403C-C404M-P406G-G443Y-L446I-L448W-T450H | +++ |
| S403C-C404M-P406A-D442A-G443Y-L446I-L448W-T450H | +++ |
| S403C-C404M-P406A-D442E-G443Y-L446I-L448W-T450H | +++ |
| S403C-C404M-P406A-D442G-G443Y-L446I-L448W-T450H | +++ |
| S403C-C404M-P406A-D442S-G443Y-L446I-L448W-T450H | +++ |
| S403C-C404M-P406A-G443Y-P444H-L446I-L448W-T450H | +++ |
| S403C-C404M-P406A-G443Y-V445A-L446I-L448W-T450H | +++ |
| S403C-C404M-P406A-G443Y-V445C-L446I-L448W-T450H | +++ |
| S403C-C404M-P406A-G443Y-V445L-L446I-L448W-T450H | +++ |
| S403C-C404M-P406A-G443Y-L446I-L448W-T450H-C451N | +++ |
| S403C-C404M-P406A-G443Y-L446I-L448W-T450H-C451S | +++ |
| V196I-M197V-S403C-C404M-P406A-G443Y-L446I-L448W | +++ |
| M197V-S403C-C404M-P406G-G443Y-L446I-L448W | +++ |
| M 197V-S403C-C404M-P406S-G443Y-L4461-L448W | +++ |
| M197V-S403C-C404M-P406A-D442A-G443Y-L446I-L448W | +++ |
| M197V-S403C-C404M-P406A-D442C-G443Y-L446I-L448W | +++ |
| M197V-S403C-C404M-P406A-D442G-G443Y-L446I-L448W | +++ |
| M197V-S403C-C404M-P406A-D442H-G443Y-L446I-L448W | +++ |
| S403C-C404M-P406A-D442H-G443Y-L446I-L448W | +++ |
| M197V-S403C-C404M-P406A-D442N-G443Y-L446I-L448W | +++ |
| M197V-S403C-C404M-P406A-D442S-G443Y-L446I-L448W | +++ |
| M197V-S403C-C404M-P406A-G443Y-V445S-L446I-L448W | +++ |
| M197V-S403C-C404M-P406A-G443Y-V445T-L446I-L448W | +++ |
| S403C-C404M-P406A-D442A-G443Y-V445L-L446I-L448W- T450H | +++ |
| M197V-S403C-C404M-P406A-D442A-G443Y-L446I- L448W-T450H | +++ |
| M197V-S403C-C404M-P406A-D442Q-G443Y-L446I- L448W-T450H | +++ |
| M197V-S403C-C404M-P406A-D442N-G443Y-L446I- L448W-T450H | +++ |
| M197V-S403C-C404M-P406A-D442S-G443Y-L446I- L448W-T450H | +++ |
| A241D-S403C-C404L-P406A-E441R-G443D-P444H- L448W-L449I | +++ |
| A241D-S403C-C404L-P406A-D442L-G443D-P444H- L448W-L449I | +++ |
| A241D-S403C-C404L-P406A-D442R-G443D-P444H- L448W-L449I | +++ |
| A241D-S403C-C404L-P406A-D442M-G443D-P444H- L448W-L449I | +++ |
| A241D-S403C-C404L-P406A-D442A-G443D-P444H- L448W-L449I | +++ |
| A241D-S403C-C404L-P406A-D442S-G443D-P444H- L448W-L449I | +++ |
| A241D-S403C-C404L-P406A-D442G-G443D-P444H- L448W-L449I | +++ |
| A241D-S403C-C404L-P406A-G443D-P444H-V445T- L448W-L449I | +++ |
| A241D-S403C-C404L-P406A-Q407C-G443D-P444H- L448W-L449I | +++ |
| A241D-S403C-C404L-P406A-G443D-P444H-L448W-L449I- P454A | +++ |
| A241D-S403C-C404L-P406A-G443D-P444H-L448W-L449I- P454W | +++ |
| A241D-S403C-C404L-P406A-G443D-P444H-L448W-L449I- P454V | +++ |
| A241D-S403C-C404L-P406A-G443D-P444H-L448W-L449I- P454M | +++ |
| A241D-S403C-C404L-P406A-G443D-P444H-L448W-L449I- P454H | +++ |
| A241D-S403C-C404L-P406A-G443D-P444H-L448W-L449I- P454T | +++ |
| A241D-S403C-C404L-P406A-G443D-P444H-L448W-L449I- P454G | +++ |
| A241D-S403C-C404L-P406A-G443D-P444H-L448W-L449I- P454K | +++ |
| A241D-S403C-C404L-P406A-G443D-P444H-L448W-L449I- P454R | +++ |
| A241D-S403C-C404L-P406A-G443D-P444H-L448W-L449I- P454Q | +++ |
| A241D-S403C-C404L-P406A-G443D-P444H-L448W-L449I- P454L | +++ |
| A241D-S403C-C404L-P406A-G443D-P444H-L448W-L449I- A459Y | +++ |
| A241D-S403C-C404L-P406A-G443D-P444H-L448W-L449I- A459N | +++ |
| A241D-S403C-C404L-P406A-G443D-P444H-L448W-L449I- A460N | +++ |
| A241D-S403C-C404L-P406A-G443D-P444H-L448W-L449I- A460L | +++ |
| A241D-S403C-C404L-P406A-G443D-P444H-L448W-L449I- A460M | +++ |
| A241D-S403C-C404L-P406A-G443D-P444H-L448W-L449I- A460R | +++ |
| A241D-S403C-C404L-P406A-G443D-P444H-L448W-L449I- R461V | +++ |
| A241D-S403C-C404L-P406A-G443D-P444H-L448W-L449I- R461S | +++ |
| E154D-A241D-E339H-S403C-C404L-P406A-G443D- P444H-L448W-L449I | +++ |
| A241D-L302R-E339H-S403C-C404L-P406A-G443D- P444H-L448W-L449I | +++ |
| A241D-E339H-A363G-S403C-C404L-P406A-G443D- P444H-L448W-L449I | +++ |
| A241D-E339H-R390D-S403C-C404L-P406A-G443D- P444H-L448W-L449I | +++ |
| A241D-E339H-R390L-S403C-C404L-P406A-G443D- P444H-L448W-L449I | +++ |
| A241D-E339H-R390N-S403C-C404L-P406A-G443D- P444H-L448W-L449I | +++ |
| A241D-E339H-R390Q-S403C-C404L-P406A-G443D- P444H-L448W-L449I | +++ |
| A241D-E339H-R391D-S403C-C404L-P406A-G443D- P444H-L448W-L449I | +++ |
| A241D-E339H-R391W-S403C-C404L-P406A-G443D- P444H-L448W-L449I | +++ |
| A241D-E339H-E394Q-S403C-C404L-P406A-G443D- P444H-L448W-L449I | +++ |
| V3M-A241D-E339H-E394T-S403C-C404L-P406A-G443D- P444H-L448W-L449I | +++ |
| A241D-E339H-G398R-S403C-C404L-P406A-G443D- P444H-L448W-L449I | +++ |
| A129V-A241D-E339H-G398C-S403C-C404L-P406A- G443D-P444H-L448W-L449I | +++ |
| A241D-E339H-S403C-C404L-P406A-V408L-G443D- P444H-L448W-L449I | +++ |
| A241D-E339H-S403C-C404L-P406A-V408M-G443D- P444H-L448W-L449I | +++ |
| A241D-E339H-S403C-C404L-P406A-E441S-G443D- P444H-L448W-L449I | +++ |
| A241D-E339H-S403C-C404L-P406A-D442H-G443D- P444H-L448W-L449I | +++ |
| A241D-E339H-S403C-C404L-P406A-D442R-G443D- P444H-L448W-L449I | +++ |
| I168V-A241D-E339H-S403C-C404L-P406A-G443D-P444H- V445L-L448W-L449I | +++ |
| A241D-E339H-S403C-C404L-P406A-G443D-P444H- L446M-L448W-L449I | +++ |
| A241D-E339H-S403C-C404L-P406A-G443D-P444H- L448W-L449I-Y452F | +++ |
| A241D-E339H-S403C-C404L-P406A-G443D-P444H- L448W-L449I-T453L | +++ |
| A241D-E339H-S403C-C404L-P406A-G443D-P444H- L448W-L449I-P454H | +++ |
| A241D-E339H-S403C-C404L-P406A-G443D-P444H- L448W-L449I-P454K | +++ |
| A241D-E339H-S403C-C404L-P406A-G443D-P444H- L448W-L449I-P454R | +++ |
| A241D-E339H-S403C-C404L-P406A-G443D-P444H- L448W-L449I-P454S | +++ |
| A241D-E339H-S403C-C404L-P406A-G443D-P444H- L448W-L449I-P454T | +++ |
| A241D-E339H-S403C-C404L-P406A-G443D-P444H- L448W-L449I-P454W | +++ |
| A241D-E339H-S403C-C404L-P406A-G443D-P444H- L448W-L449I-R461V-P479S | +++ |
| A241D-S403C-C404L-P406A-Q407A-G443N-L448W | +++ |
| A241D-S403C-C404L-P406A-D442A-G443N-L448W | +++ |
| A241D-S403C-C404L-P406A-G443N-L448W-T450H | +++ |
| A241D-S403C-C404L-P406A-G443N-L448W-T453R | +++ |
| A241D-S403C-C404L-P406A-G443N-L446M-L448W | +++ |
| A241D-E339H-S403C-C404L-P406A-E441K-G443D- P444H-L446M-L448W-L449I | +++ |
| M197V-G237A-A241D-S403C-C404L-P406A-G443F- L448W-T450M | +++ |
| M197V-G237D-A241D-S403C-C404L-P406A-G443F- L448W-T450M | +++ |
| M197V-G237N-A241D-S403C-C404L-P406A-G443F- L448W-T450M | +++ |
| M197V-G237P-A241D-S403C-C404L-P406A-G443F- L448W-T450M | +++ |
| M197V-D238H-A241D-S403C-C404L-P406A-G443F- L448W-T450M | +++ |
| M197V-D238R-A241D-S403C-C404L-P406A-G443F- L448W-T450M | +++ |
| M197V-D238S-A241D-S403C-C404L-P406A-G443F- L448W-T450M | +++ |
| M197V-D238W-A241D-S403C-C404L-P406A-G443F- L448W-T450M | +++ |
| M197V-A241D-L302A-S403C-C404L-P406A-G443F- L448W-T450M | +++ |
| M197V-A241D-L302K-S403C-C404L-P406A-G443F- L448W-T450M | +++ |
| M197V-A241D-L302M-S403C-C404L-P406A-G443F- L448W-T450M | +++ |
| M197V-A241D-L302R-S403C-C404L-P406A-G443F- L448W-T450M | +++ |
| M197V-A241D-A359V-S403C-C404L-P406A-G443F- L448W-T450M | +++ |
| M197V-A241D-G398R-S403C-C404L-P406A-G443F- L448W-T450M | +++ |
| M197V-A241D-E394C-S403C-C404L-P406A-G443F- L448W-T450M | +++ |
| M197V-A241D-E394D-S403C-C404L-P406A-G443F- L448W-T450M | +++ |
| M197V-A241D-V396I-S403C-C404L-P406A-G443F-L448W- T450M | +++ |
| V35I-M197V-A241D-S403C-C404L-P406A-Q407I-G443F- L448W-T450M | +++ |
| M197V-A241D-S403C-C404L-P406A-V408A-G443F- L448W-T450M | +++ |
| Y2N-M197V-A241D-S403C-C404L-P406A-G443F-L448W- T450M | +++ |
| M197V-A241D-S403C-C404L-P406A-R439C-G443F- L448W-T450M | +++ |
| M197V-A241D-S403C-C404L-P406A-F440L-G443F- L448W-T450M | +++ |
| M197V-A241D-S403C-C404L-P406A-G443F-L448W- T450M-A460F | +++ |
| M197V-A241D-S403C-C404L-P406A-G443F-L448W- T450M-A460L | +++ |
| M197V-A241D-S403C-C404L-P406A-G443F-L448W- T450M-A460R | +++ |
| M197V-A241D-S403C-C404L-P406A-G443F-L448W- T450M-P454T | +++ |
| M197V-A241D-S403C-C404L-P406A-G443F-L448W- T450M-A462V | +++ |
| S403C-C404M-P406G-Q407T-G443Y-L446I-L448W-T450H | +++ |
| M5I-M197V-S403C-C404M-P406G-D442N-G443Y-L446I- L448W-T450H | +++ |
| M197V-A241D-S403C-C404L-P406A-G443D-P444H- L448W-L449V-A459D | +++ |
| A241D-S403C-C404L-P406A-G443D-L448W-L449I-A459G | +++ |
| S403C-C404M-P406A-Q407T-G443Y-L446I-L448W- T450H-A459G | +++ |
| S403C-C404M-P406A-G443Y-L446I-L448W-T450H-A459G | +++ |
| A241D-S403C-C404L-P406A-G443D-L448W-L449I-A459R | +++ |
| A241D-S403C-C404L-P406A-G443Y-L448W-T450M- A460E | +++ |
| A241D-S403C-C404L-P406A-V408I-P444H-L446M-L448W- T450M-A460G | +++ |
| M197V-A241D-S403C-C404L-P406A-G443D-P444H- L448W-L449V-A460G-A462V | +++ |
| A241D-S403C-C404L-P406A-G443Y-L448W-T450M-A460L | +++ |
| S403C-C404M-P406A-Q407T-G443Y-L446I-L448W- T450H-A460L | +++ |
| A241D-S403C-C404L-P406A-V408I-P444H-L446M-L448W- T450M-A460Q | +++ |
| A241D-S403C-C404L-P406A-G443Y-L448W-T450M- A462C | +++ |
| M197V-S403C-C404M-P406A-D442N-G443Y-L446I- L448W-T450H-A462C | +++ |
| A241D-S403C-C404L-P406A-G443Y-L448W-T450M- A462V | +++ |
| S403C-C404M-P406A-Q407T-G443Y-L446I-L448W- T450H-A462V | +++ |
| S403C-C404M-P406A-Q407T-G443Y-L446I-L448W- T450H-P454Q-A462V | +++ |
| H198W-S403V-C404M-P406A-Q407T-G443Y-L446I- L448W-T450H | +++ |
| L74V-H198W-S403V-C404M-P406A-Q407T-G443Y-L446I- L448W-T450H | +++ |
| H198W-S403C-C404M-P406A-D442Q-G443Y-L446I- L448W-T450H | +++ |
| A241D-S403C-C404L-P406A-E441G-G443D-L448W-L449I | +++ |
| Q208L-A241D-S403C-C404L-P406A-V408I-G443R-P444H- L446M-L448W-T450M | +++ |
| A241D-S403C-C404L-P406A-V408I-G443W-P444H- L446M-L448W-T450M | +++ |
| A241D-S403C-C404L-P406A-G443D-L448W-L449I-H458G | +++ |
| L195V-S403C-C404M-P406A-Q407T-G443Y-L446I-L448W- T450H | +++ |
| L195V-S403C-C404M-P406A-G443Y-L446I-L448W-T450H | +++ |
| M197V-A241D-S403C-C404L-P406A-G443Y-L448W- T450M | +++ |
| M197V-S403C-C404M-P406A-Q407T-G443Y-L446I- L448W-T450H | +++ |
| M197V-S403C-C404M-P406A-D442N-G443Y-L446I- L448W-T450H-E456Q | +++ |
| S403C-C404F-P406A-Q407T-G443Y-L446I-L448W-T450H | +++ |
| S403C-C404M-P406A-Q407T-G443Y-P444F-L446I-L448W- T450H | +++ |
| A241D-S403C-C404L-P406A-G443D-L448W-L449I-P454K | +++ |
| M197V-A241D-S403C-C404L-P406A-G443D-P444H- L448W-L449V-P454K | +++ |
| A241D-S403C-C404L-P406A-G443D-L448W-L449I-P454R | +++ |
| M197V-A241D-S403C-C404L-P406A-G443D-P444H- L448W-L449V-P454R | +++ |
| A241D-S403C-C404L-P406A-G443D-L448W-L449I-Q455S | +++ |
| M197V-S403C-C404M-P406A-D442N-G443Y-L446I- L448W-T450H-Q455V | +++ |
| S403C-C404M-P406A-Q407T-G443Y-L446I-L448W- T450H-R457K | +++ |
| M197V-S403C-C404M-P406A-D442N-G443Y-L446I- L448W-T450H-R461G | +++ |
| A241D-S403C-C404L-P406A-V408I-P444H-L446M-L448W- T450M-R461S | +++ |
| M197V-A241D-S403C-C404L-P406A-G443D-P444H- L448W-L449V-T450H | +++ |
| A241D-S403C-C404L-P406A-G443D-L448W-L449I-T450V | +++ |
| M197V-A241D-S403C-C404L-P406A-G443D-P444H- L448W-L449V-T450V | +++ |
| H36Y-M197V-S403C-C404M-P406A-D442N-G443Y-L446I- L448W-T450H-T453G | +++ |
| S403C-C404M-P406A-Q407T-G443Y-L446I-L448W- T450H-T453N | +++ |
| H198W-S403C-C404M-P406A-G443Y-L446I-L448W- T450H-T453P | +++ |
| A241D-S403C-C404L-P406A-V408I-P444H-L446M-L448W- T450M-T453R | +++ |
| A241D-S403C-C404L-P406A-V408I-P444H-L446M-L448W- T450M-T453S | +++ |
| S403C-C404M-P406A-Q407T-G443Y-V445L-L446I-L448W- T450H | +++ |
| S403C-C404M-P406A-Q407T-G443Y-V445S-L446I- L448W-T450H | +++ |
| M197V-A241D-S403C-C404L-P406A-G443D-P444H- L448W | +++ |
| A241D-S403C-C404L-P406A-V408I-P444H-L446M-L448W- T450M-Y452F | +++ |
| M5I-A241D-S403C-C404L-P406A-G443Y-L448W-T450M | +++ |
| M197V-A241D-S403C-C404L-P406A-G443F-L446M- L448W-T450M | +++ |
| M197V-S403C-C404L-P406A-D442N-G443Y-L446M- L448W-T450H | +++ |
| M197V-S403C-C404L-P406A-D442N-G443Y-L448W- T450M | +++ |
| M197V-S403C-C404L-P406A-D442N-G443Y-L448W- T450H | +++ |
| S403C-C404L-P406A-Q407T-G443Y-L446M-L448W-T450H | +++ |
| S403C-C404L-P406A-Q407T-G443Y-L446F-L448W-T450H | +++ |
| S403C-C404L-P406A-Q407T-G443Y-L448W-T450H | +++ |
| A241D-E339H-A343S-S403C-C404L-P406A-G443D- P444H-L448W-L449I-P454R | +++ |
| A241D-E339H-A350S-S403C-C404L-P406A-G443D- P444H-L446M-L448W-L449I | +++ |
| A241D-E339H-A395E-S403C-C404L-P406A-A426V- G443D-P444H-L446M-L448W-L449I | +++ |
| A241D-E339H-S403C-C404L-P406A-D442N-G443D- P444H-L448W-L449I-P454R | +++ |
| A241D-E339H-S403C-C404L-P406A-D442S-G443D- P444H-L448W-L449I-P454R | +++ |
| A241D-E339H-S403C-C404L-P406A-D442T-G443D- P444H-L446M-L448W-L449I | +++ |
| A241D-E339H-S403C-C404L-P406A-G443H-P444H- L448W-L449I-P454R | +++ |
| A241D-E339H-S403C-C404L-P406A-E441R-G443D- P444H-L448W-L449I-P454R | +++ |
| A241D-E339H-I347C-S403C-C404L-P406A-G443D-P444H- L448W-L449I-P454R | +++ |
| V3L-A241D-L302Q-E339H-S403C-C404L-P406A-G443D- P444H-L446M-L448W-L449I | +++ |
| A241D-E339H-S403C-C404L-L405T-P406A-G443D- P444H-L446M-L448W-L449I | +++ |
| M5I-A241D-E339H-S403C-C404L-P406A-G443D-P444H- L446M-L448W-L449I | +++ |
| A241D-E339H-S403C-C404L-P406A-G443D-P444H- L448W-L449I-P454R-Q455A | +++ |
| A241D-E339H-S403C-C404L-P406A-G443D-P444H- L448W-L449I-P454R-Q455N | +++ |
| A241D-S403C-C404L-P406A-G443D-P444H-L448W-L449I- P454R-A459E | +++ |
| A241D-S403C-C404L-P406A-G443D-P444H-L448W-L449I- P454R-A459M | +++ |
| A241D-S403C-C404L-P406A-G443D-P444H-L448W-L449I- P454R-A459Q | +++ |
| A241D-S403C-C404L-P406A-G443D-P444H-L448W-L449I- P454R-A459T | +++ |
| A241D-S403C-C404L-P406A-G443N-P444H-L448W-L449I- P454R | +++ |
| A241D-S403C-C404L-P406A-E441G-G443D-P444H- L448W-L449I-P454R | +++ |
| A241D-S403C-C404L-P406A-G443D-P444F-L448W-L449I- P454R | +++ |
| A241D-S403C-C404L-P406A-G443D-P444H-L448W-L449I- P454R-H458F | +++ |
| A241D-S403C-C404L-P406A-G443D-P444H-L448W-L449I- P454R-H458N | +++ |
| A241D-I296Q-S403C-C404L-P406A-G443D-P444H- L448W-L449I-P454R | +++ |
| A241D-L302A-S403C-C404L-P406A-G443D-P444H- L448W-L449I-P454R | +++ |
| A241D-S403C-C404L-P406A-G443D-P444H-L448W-L449I- T453S-P454R | +++ |
| V196I-A241D-S403C-C404L-P406A-G443D-P444H-L448W- L449I-P454R | +++ |
| A241D-V305I-S403C-C404L-P406A-G443D-P444H-L448W- L449I-P454R | +++ |
| A241D-V305T-S403C-C404L-P406A-G443D-P444H- L448W-L449I-P454R | +++ |
| M197V-G237A-A241D-S403C-C404L-P406A-E441G- G443F-L448W-T450M | +++ |
| M197V-G237A-A241D-S403C-C404L-P406A-E441H- G443F-L448W-T450M | +++ |
| M197V-G237A-A241D-S403C-C404L-P406A-E441T- G443F-L448W-T450M | +++ |
| M197V-A241D-S403C-C404L-P406A-F440L-G443F- L446M-L448W-T450M | +++ |
| M197V-A241D-H277Y-S403C-C404L-P406A-F440L- G443F-L448W-L449I-T450M | +++ |
| M197V-G237A-A241D-S403C-C404L-P406A-G443F- P444H-L448W-T450M | +++ |
| M197V-G237A-A241D-S403C-C404L-P406A-G443F-P444I- L448W-T450M | +++ |
| M197V-A241D-S403C-C404L-P406A-Q407R-F440L- G443F-L448W-T450M | +++ |
| V6M-M197V-D238H-A241D-S403C-C404L-P406A-G443F- L448W-T450M-T453C | +++ |
| M197V-D238H-A241D-S403C-C404L-P406A-G443F- L448W-T450M-T453R | +++ |
| M197V-D238H-A241D-S403C-C404L-P406A-G443F- L448W-T450M-Y452F | +++ |
| A241D-S403C-C404L-P406A-P444H-L446M-L448W- T450M-Y452F | +++ |
| A241D-S403C-C404L-P406A-V408I-P444H-L446M-L448W- T450M-Y452F-Q455H | +++ |
| A241D-S403C-C404L-P406A-V408I-P444H-L446M-P447Q- L448W-T450M-A460G | +++ |
| A241D-S403C-C404L-P406A-V408I-P444H-L446M-L448W- T450M-Q455K-R461S | +++ |
| A241D-S403C-C404L-P406A-V408I-P444H-L446M-L448W- T450M-Q455V-R461S | +++ |
| Q204R-S403C-C404L-P406A-Q407T-G443Y-L446F- L448W-T450H | +++ |
| Q204S-S403C-C404L-P406A-Q407T-G443Y-L446F- L448W-T450H | +++ |
| L302F-S403C-C404L-P406A-Q407T-G443Y-L446F-L448W- T450H | +++ |
| L302S-S403C-C404L-P406A-Q407T-G443Y-L446F-L448W- T450H | +++ |
| E349S-S403C-C404L-P406A-Q407T-G443Y-L446F- L448W-T450H | +++ |
| A395C-S403C-C404L-P406A-Q407T-G443Y-L446F- L448W-T450H | +++ |
| S403C-C404L-P406A-Q407T-E441P-G443Y-L446F- L448W-T450H | +++ |
| S403C-C404L-P406A-Q407T-G443A-L446F-L448W-T450H | +++ |
| S403C-C404L-P406A-Q407T-G443Y-P444Q-L446F- L448W-T450H | +++ |
| S403C-C404L-P406A-Q407T-G443Y-L446F-L448W- T450H-H458L | +++ |
| S403C-C404L-P406A-Q407T-G443Y-L446F-L448W- T450H-H458M | +++ |
| A241D-E339H-H366F-S403C-C404L-P406A-G443D- P444H-L446M-L448W-L449I | +++ |
| A241D-E339H-H366F-S403C-C404L-P406A-G443D- P444H-L446M-L448W | +++ |
| A241D-E339H-H366I-S403C-C404L-P406A-G443D-P444H- L446M-L448W-L449I | +++ |
| A241D-E339H-L368I-S403C-C404L-P406A-G443D-P444H- L446M-L448W-L449I | +++ |
| A241D-E339H-L368M-S403C-C404L-P406A-G443D- P444H-L446M-L448W-L449I | +++ |
| A241D-E339H-L368M-S403C-C404L-L405M-P406A- G443D-P444H-L446M-L448W-L449I | +++ |
| A241D-E339H-A395V-S403C-C404L-P406A-D442N- G443D-P444H-L448W-L449I-P454R | +++ |
| A241D-E339H-S403C-C404L-P406A-D442N-G443D- P444H-L448W-L449I-P454R-A460F | +++ |
| A241D-E339H-E349M-S403C-C404L-P406A-D442N- G443D-P444H-L448W-L449I-P454R | +++ |
| A241D-E339H-S403C-C404L-P406A-E441H-D442N- G443D-P444H-L448W-L449I-P454R | +++ |
| A241D-E339H-S403C-C404L-P406A-E441N-D442N- G443D-P444H-L448W-L449I-P454R | +++ |
| A241D-E339H-S403C-C404L-P406A-E441R-D442N- G443D-P444H-L448W-L449I-P454R | +++ |
| A126V-A241D-E339H-S403C-C404L-P406A-D442N- G443D-P444H-L448W-L449I-P454R | +++ |
| A241D-E339H-S403C-C404L-P406A-D442N-G443D- P444H-L448W-L449I-P454R-H458E | +++ |
| A241D-E339H-S403C-C404L-P406A-D442N-G443D- P444H-L448W-L449I-P454R-H458G | +++ |
| A241D-E339H-S403C-C404L-P406A-D442N-G443D- P444H-L448W-L449I-P454R-H458L | +++ |
| A241D-E339H-S403C-C404L-P406A-D442N-G443D- P444H-L448W-L449I-P454R-H458Q | +++ |
| A241D-E339H-S403C-C404L-P406A-D442N-G443D- P444H-L448W-L449I-P454R-H458R | +++ |
| A241D-E339H-S403C-C404L-P406A-D442N-G443D- P444H-L448W-L449I-P454R-H458S | +++ |
| A241D-E339H-S403C-C404L-P406A-D442N-G443D- P444H-L448W-L449I-P454R-H458Y | +++ |
| A241D-E339H-S403C-C404L-P406A-D442N-G443D- P444H-L448W-L449V-P454R | +++ |
| A241D-L302C-E339H-S403C-C404L-P406A-D442N- G443D-P444H-L448W-L449I-P454R | +++ |
| A241D-E339H-S403C-C404L-L405V-P406A-D442N- G443D-P444H-L448W-L449I-P454R | +++ |
| A241D-E339H-S403C-C404L-P406A-Q407R-D442N- G443D-P444H-L448W-L449I-P454R | +++ |
| A241D-E339H-S403C-C404L-P406A-Q407S-D442N- G443D-P444H-L448W-L449I-P454R | +++ |
| A241D-E339H-S403C-C404L-P406A-D442N-G443D- P444H-L448W-L449I-P454R-Q455E | +++ |
| H36Y-A241D-R298S-E339H-S403C-C404L-P406A-D442N- G443D-P444H-L448W-L449I-P454R | +++ |
| A241D-E339H-S403C-C404L-P406A-D442N-G443D- P444H-L448W-L449I-P454A | +++ |
| A241D-E339H-S403C-C404L-P406A-D442N-G443D- P444H-L448W-L449I-P454D | +++ |
| A241D-E339H-S403C-C404L-P406A-D442N-G443D- P444H-L448W-L449I-P454L | +++ |
| A241D-E339H-S403C-C404L-P406A-D442N-G443D- P444H-L448W-L449I | +++ |
| A241D-E339H-S403C-C404L-P406A-D442N-G443D- P444H-L448W-L449I-P454S | +++ |
| A241D-E339H-S403C-C404L-P406A-D442N-G443D- P444H-L448W-L449I-P454R-R461A | +++ |
| A241D-E339H-S403C-C404L-P406A-D442N-G443D- P444H-L448W-L449I-P454R-R461L | +++ |
| A241D-E339H-S403C-C404L-P406A-D442N-G443D- P444H-L448W-L449I-P454R-R461M | +++ |
| A241D-E339H-S403C-C404L-P406A-D442N-G443D- P444H-L448W-L449I-P454R-R461Q | +++ |
| A241D-E339H-S403C-C404L-P406A-D442N-G443D- P444H-L448W-L449I-P454R-R461S | +++ |
| A241D-E339H-S403C-C404L-P406A-D442N-G443D- P444H-L448W-L449I-P454R-R461V | +++ |
| A241D-E339H-S403C-C404L-P406A-D442N-G443D- P444H-L448W-L449I-T450S-P454R | +++ |
| A241D-S403C-C404L-P406S-G443D-P444H-L448W-L449I | +++ |
| M5I-A241D-S403C-C404L-P406A-G443D-P444H-L448W- L449I | +++ |
| V166D-A241D-S403C-C404L-P406A-G443D-P444H- L448W-L449I-Y452F | +++ |

**Table 2.**

| **List of the positions modified in the variants of *Streptomyces sp.* 769 3-MethylCrotonate Decarboxylase with increased activity** | | |
|---|---|---|
| **Position** | **Wild-Type amino Acid** | **Mutations** |
| 2 | Y | N |
| 3 | V | L,M |
| 5 | M | I |
| 6 | V | M |
| 24 | E | A,D,L,V |
| 25 | A | E |
| 35 | V | I |
| 36 | H | Y |
| 64 | N | H |
| 66 | A | H |
| 74 | L | V |
| 80 | A | S |
| 85 | P | L |
| 86 | S | N,Y |
| 126 | A | V |
| 129 | A | V |
| 154 | E | D |
| 166 | V | D |
| 168 | I | V |
| 175 | S | E |
| 195 | L | I,V |
| 196 | V | I |
| 197 | M | V |
| 198 | H | Q,W |
| 204 | Q | R,S |
| 208 | Q | L |
| 214 | K | T |
| 236 | I | L,V |
| 237 | G | A,D,N,P |
| 238 | D | H,R,S,W |
| 240 | V | I,N |
| 241 | A | D,N,V |
| 277 | H | Y |
| 296 | I | Q |
| 298 | R | S |
| 302 | L | A,C,F,K,M,Q,R,S |
| 305 | V | I,T |
| 339 | E | H |
| 343 | A | S |
| 347 | I | C |
| 349 | E | M,S |
| 350 | A | S |
| 359 | A | C,V |
| 363 | A | G |
| 366 | H | F, I |
| 368 | L | I,M |
| 390 | R | D,L,N,Q,S |
| 391 | R | D,W |
| 394 | E | C,D,Q,T |
| 395 | A | C,E,V |
| 396 | V | I |
| 398 | G | C,R |
| 401 | F | Y |
| 402 | G | A |
| 403 | S | A,C,G,H,I,N,P,Q,R,V |
| 404 | C | F,L,M |
| 405 | L | H,I,M,T,V |
| 406 | P | A,G,S |
| 407 | Q | A,C,I,K,R,S,T |
| 408 | V | A,C,I,L,M |
| 426 | A | V |
| 439 | R | C |
| 440 | F | L |
| 441 | E | G,H,K,N,P,R,S,T |
| 442 | D | A,C,E,G,H,L,M,N,Q,R,S,T |
| 443 | G | A,D,F,H,L,N,R,S,W,Y |
| 444 | P | A,E,F,H,I,L,Q,T |
| 445 | V | A,C,L,S,T |
| 446 | L | A,C,F,I,M,N,S,V |
| 447 | P | Q |
| 448 | L | F,W,Y |
| 449 | L | I,M,V |
| 450 | T | A,H,M,N,Q,S,V |
| 451 | C | N,S |
| 452 | Y | F |
| 453 | T | C,G,L,N,P,R,S |
| 454 | P | A,D,G,H,K,L,M,Q,R,S,T,V,VV |
| 455 | Q | A,E,H,K,N,S,V |
| 456 | E | Q |
| 457 | R | K |
| 458 | H | E,F,G,L,M,N,Q,R,S,Y |
| 459 | A | D,E,G,M,N,Q,R,T,Y |
| 460 | A | E,F,G,L,M,N,Q,R |
| 461 | R | A,G,L,M,Q,S,V |
| 462 | A | C,V |
| 473 | A | S |
| 479 | P | C,I,L,M,S,T,V |
| 496 | R | L |

## Claims

1. A variant of a 3-methylcrotonic acid decarboxylase (MDC) showing an improved activity in converting 3-methylcrotonic acid into isobutene over the MDC of SEQ ID NO:1,
wherein said variant is **characterized in that**:
it has an amino acid sequence having at least 80 % sequence identity to SEQ ID NO:1; and
it shows one or more amino acid substitutions with another amino acid residue at a position selected from the group consisting of positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462 and 473 in the amino acid sequence shown in SEQ ID NO:1 or at a position corresponding to any of these positions.

2. The MDC variant of claim 1, wherein
(1) an amino acid residue at position 198 in the amino acid sequence shown in SEQ ID NO:1 or at a position corresponding to this position, is substituted with glutamine or tryptophan; and/or
(2) an amino acid residue at position 240 in the amino acid sequence shown in SEQ ID NO:1 or at a position corresponding to this position, is substituted with isoleucine or asparagine; and/or
(3) an amino acid residue at position 390 in the amino acid sequence shown in SEQ ID NO:1 or at a position corresponding to this position, is substituted with serine, aspartic acid, leucine, asparagine or glutamine; and/or
(4) an amino acid residue at position 402 in the amino acid sequence shown in SEQ ID NO:1 or at a position corresponding to this position, is substituted with alanine; and/or
(5) an amino acid residue at position 406 in the amino acid sequence shown in SEQ ID NO:1 or at a position corresponding to this position, is substituted with glycine, alanine or serine; and/or
(6) an amino acid residue at position 408 in the amino acid sequence shown in SEQ ID NO:1 or at a position corresponding to this position, is substituted with isoleucine, alanine, cysteine, leucine, or methionine; and/or
(7) an amino acid residue at position 449 in the amino acid sequence shown in SEQ ID NO:1 or at a position corresponding to this position, is substituted with isoleucine, methionine or valine; and/or
(8) an amino acid residue at position 25 in the amino acid sequence shown in SEQ ID NO:1 or at a position corresponding to this position, is substituted with glutamic acid; and/or
(9) an amino acid residue at position 35 in the amino acid sequence shown in SEQ ID NO:1 or at a position corresponding to this position, is substituted with isoleucine; and/or
(10) an amino acid residue at position 36 in the amino acid sequence shown in SEQ ID NO:1 or at a position corresponding to this position, is substituted with tyrosine; and/or
(11) an amino acid residue at position 74 in the amino acid sequence shown in SEQ ID NO:1 or at a position corresponding to this position, is substituted with valine; and/or
(12) an amino acid residue at position 80 in the amino acid sequence shown in SEQ ID NO:1 or at a position corresponding to this position, is substituted with serine; and/or
(13) an amino acid residue at position 86 in the amino acid sequence shown in SEQ ID NO:1 or at a position corresponding to this position, is substituted with asparagine or tyrosine; and/or
(14) an amino acid residue at position 126 in the amino acid sequence shown in SEQ ID NO:1 or at a position corresponding to this position, is substituted with valine; and/or
(15) an amino acid residue at position 129 in the amino acid sequence shown in SEQ ID NO:1 or at a position corresponding to this position, is substituted with valine; and/or
(16) an amino acid residue at position 166 in the amino acid sequence shown in SEQ ID NO:1 or at a position corresponding to this position, is substituted with aspartic acid; and/or
(17) an amino acid residue at position 196 in the amino acid sequence shown in SEQ ID NO:1 or at a position corresponding to this position, is substituted with isoleucine; and/or
(18) an amino acid residue at position 204 in the amino acid sequence shown in SEQ ID NO:1 or at a position corresponding to this position, is substituted with arginine or serine; and/or
(19) an amino acid residue at position 208 in the amino acid sequence shown in SEQ ID NO:1 or at a position corresponding to this position, is substituted with leucine; and/or
(20) an amino acid residue at position 236 in the amino acid sequence shown in SEQ ID NO:1 or at a position corresponding to this position, is substituted with leucine or valine; and/or
(21) an amino acid residue at position 237 in the amino acid sequence shown in SEQ ID NO:1 or at a position corresponding to this position, is substituted with alanine, aspartic acid, asparagine or proline; and/or
(22) an amino acid residue at position 238 in the amino acid sequence shown in SEQ ID NO:1 or at a position corresponding to this position, is substituted with histidine, arginine, serine or tryptophan; and/or
(23) an amino acid residue at position 298 in the amino acid sequence shown in SEQ ID NO:1 or at a position corresponding to this position, is substituted with serine; and/or
(24) an amino acid residue at position 302 in the amino acid sequence shown in SEQ ID NO:1 or at a position corresponding to this position, is substituted with alanine, cysteine, phenylalanine, lysine, methionine, glutamine, arginine or serine; and/or
(25) an amino acid residue at position 359 in the amino acid sequence shown in SEQ ID NO:1 or at a position corresponding to this position, is substituted with cysteine or valine; and/or
(26) an amino acid residue at position 366 in the amino acid sequence shown in SEQ ID NO:1 or at a position corresponding to this position, is substituted with isoleucine or phenylalanine; and/or
(27) an amino acid residue at position 368 in the amino acid sequence shown in SEQ ID NO:1 or at a position corresponding to this position, is substituted with isoleucine or methionine; and/or
(28) an amino acid residue at position 395 in the amino acid sequence shown in SEQ ID NO:1 or at a position corresponding to this position, is substituted with cysteine, glutamic acid or valine; and/or
(29) an amino acid residue at position 396 in the amino acid sequence shown in SEQ ID NO:1 or at a position corresponding to this position, is substituted with isoleucine; and/or
(30) an amino acid residue at position 407 in the amino acid sequence shown in SEQ ID NO:1 or at a position corresponding to this position, is substituted with alanine, cysteine, isoleucine, lysine, arginine, serine or threonine; and/or
(31) an amino acid residue at position 426 in the amino acid sequence shown in SEQ ID NO:1 or at a position corresponding to this position, is substituted with valine; and/or
(32) an amino acid residue at position 450 in the amino acid sequence shown in SEQ ID NO:1 or at a position corresponding to this position, is substituted with alanine, histidine, methionine, asparagine, glutamine, serine or valine; and/or
(33) an amino acid residue at position 451 in the amino acid sequence shown in SEQ ID NO:1 or at a position corresponding to this position, is substituted with asparagine or serine; and/or
(34) an amino acid residue at position 452 in the amino acid sequence shown in SEQ ID NO:1 or at a position corresponding to this position, is substituted with phenylalanine; and/or
(35) an amino acid residue at position 454 in the amino acid sequence shown in SEQ ID NO:1 or at a position corresponding to this position, is substituted with alanine, aspartic acid, glycine, histidine, lysine, leucine, methionine, glutamine, arginine, serine, threonine, valine or tryptophan; and/or
(36) an amino acid residue at position 455 in the amino acid sequence shown in SEQ ID NO:1 or at a position corresponding to this position, is substituted with alanine, glutamic acid, histidine, lysine, asparagine, serine or valine; and/or
(37) an amino acid residue at position 456 in the amino acid sequence shown in SEQ ID NO:1 or at a position corresponding to this position, is substituted with glutamine; and/or
(38) an amino acid residue at position 457 in the amino acid sequence shown in SEQ ID NO:1 or at a position corresponding to this position, is substituted with lysine; and/or
(39) an amino acid residue at position 458 in the amino acid sequence shown in SEQ ID NO:1 or at a position corresponding to this position, is substituted with glutamic acid, phenylalanine, glycine, leucine, methionine, asparagine, glutamine, arginine, serine or tyrosine; and/or
(40) an amino acid residue at position 462 in the amino acid sequence shown in SEQ ID NO:1 or at a position corresponding to this position, is substituted with cysteine or valine; and/or
(41) an amino acid residue at position 473 in the amino acid sequence shown in SEQ ID NO:1 or at a position corresponding to this position, is substituted with serine.

3. The MDC variant of claim 1 or 2, wherein said variant is **characterized in that** it has one or more further amino acid substitutions at a position selected from the group consisting of 2, 3, 5, 6, 24, 64, 66, 85, 154, 168, 175, 195, 197, 214, 241, 277, 296, 305, 339, 343, 347, 349, 350, 363, 391, 394, 398, 401, 403, 404, 405, 439, 440, 441, 442, 443, 444, 445, 446, 447, 448, 453, 459, 460, 461, 479 and 496 in the amino acid sequence shown in SEQ ID NO:1 or at a position corresponding to any of these positions, and wherein said MDC has an improved activity in converting 3-methylcrotonic acid into isobutene over the corresponding MDC from which it is derived.

4. The MDC variant of claim 3, wherein
(42) an amino acid residue at position 85 in the amino acid sequence shown in SEQ ID NO:1 or at a position corresponding to this position, is substituted with leucine; and/or
(43) an amino acid residue at position 241 in the amino acid sequence shown in SEQ ID NO:1 or at a position corresponding to this position, is substituted with aspartic acid, asparagine or valine; and/or
(44) an amino acid residue at position 401 in the amino acid sequence shown in SEQ ID NO:1 or at a position corresponding to this position, is substituted with tyrosine; and/or
(45) an amino acid residue at position 403 in the amino acid sequence shown in SEQ ID NO:1 or at a position corresponding to this position, is substituted with alanine, cysteine, glycine, histidine, isoleucine, asparagine, proline, glutamine, arginine or valine; and/or
(46) an amino acid residue at position 404 in the amino acid sequence shown in SEQ ID NO:1 or at a position corresponding to this position, is substituted with phenylalanine, leucine or methionine; and/or
(47) an amino acid residue at position 405 in the amino acid sequence shown in SEQ ID NO:1 or at a position corresponding to this position, is substituted with histidine, isoleucine, methionine, threonine or valine; and/or
(48) an amino acid residue at position 443 in the amino acid sequence shown in SEQ ID NO:1 or at a position corresponding to this position, is substituted with alanine, aspartic acid, phenylalanine, histidine, leucine, asparagine, arginine, serine, tryptophan or tyrosine; and/or
(49) an amino acid residue at position 444 in the amino acid sequence shown in SEQ ID NO:1 or at a position corresponding to this position, is substituted with alanine, glutamic acid, phenylalanine, histidine, isoleucine, leucine, glutamine or threonine; and/or
(50) an amino acid residue at position 445 in the amino acid sequence shown in SEQ ID NO:1 or at a position corresponding to this position, is substituted with leucine, alanine, cysteine, serine or threonine; and/or
(51) an amino acid residue at position 446 in the amino acid sequence shown in SEQ ID NO:1 or at a position corresponding to this position, is substituted with alanine, cysteine, phenylalanine, isoleucine, methionine, asparagine, serine or valine; and/or
(52) an amino acid residue at position 448 in the amino acid sequence shown in SEQ ID NO:1 or at a position corresponding to this position, is substituted with phenylalanine, tryptophan or tyrosine; and/or
(53) an amino acid residue at position 2 in the amino acid sequence shown in SEQ ID NO:1 or at a position corresponding to this position, is substituted with asparagine; and/or
(54) an amino acid residue at position 3 in the amino acid sequence shown in SEQ ID NO:1 or at a position corresponding to this position, is substituted with leucine or methionine; and/or
(55) an amino acid residue at position 5 in the amino acid sequence shown in SEQ ID NO:1 or at a position corresponding to this position, is substituted with isoleucine; and/or
(56) an amino acid residue at position 6 in the amino acid sequence shown in SEQ ID NO:1 or at a position corresponding to this position, is substituted with methionine; and/or
(57) an amino acid residue at position 24 in the amino acid sequence shown in SEQ ID NO:1 or at a position corresponding to this position, is substituted with alanine, aspartic acid, leucine or valine; and/or
(58) an amino acid residue at position 64 in the amino acid sequence shown in SEQ ID NO:1 or at a position corresponding to this position, is substituted with histidine; and/or
(59) an amino acid residue at position 66 in the amino acid sequence shown in SEQ ID NO:1 or at a position corresponding to this position, is substituted with histidine; and/or
(60) an amino acid residue at position 154 in the amino acid sequence shown in SEQ ID NO:1 or at a position corresponding to this position, is substituted with aspartic acid; and/or
(61) an amino acid residue at position 168 in the amino acid sequence shown in SEQ ID NO:1 or at a position corresponding to this position, is substituted with valine; and/or
(62) an amino acid residue at position 175 in the amino acid sequence shown in SEQ ID NO:1 or at a position corresponding to this position, is substituted with glutamic acid; and/or
(63) an amino acid residue at position 195 in the amino acid sequence shown in SEQ ID NO:1 or at a position corresponding to this position, is substituted with isoleucine or valine; and/or
(64) an amino acid residue at position 197 in the amino acid sequence shown in SEQ ID NO:1 or at a position corresponding to this position, is substituted with valine; and/or
(65) an amino acid residue at position 214 in the amino acid sequence shown in SEQ ID NO:1 or at a position corresponding to this position, is substituted with threonine; and/or
(66) an amino acid residue at position 277 in the amino acid sequence shown in SEQ ID NO:1 or at a position corresponding to this position, is substituted with tyrosine; and/or
(67) an amino acid residue at position 296 in the amino acid sequence shown in SEQ ID NO:1 or at a position corresponding to this position, is substituted with glutamine; and/or
(68) an amino acid residue at position 305 in the amino acid sequence shown in SEQ ID NO:1 or at a position corresponding to this position, is substituted with isoleucine or threonine; and/or
(69) an amino acid residue at position 339 in the amino acid sequence shown in SEQ ID NO:1 or at a position corresponding to this position, is substituted with histidine; and/or
(70) an amino acid residue at position 343 in the amino acid sequence shown in SEQ ID NO:1 or at a position corresponding to this position, is substituted with serine; and/or
(70) an amino acid residue at position 347 in the amino acid sequence shown in SEQ ID NO:1 or at a position corresponding to this position, is substituted with cysteine; and/or
(72) an amino acid residue at position 349 in the amino acid sequence shown in SEQ ID NO:1 or at a position corresponding to this position, is substituted with methionine or serine; and/or
(73) an amino acid residue at position 350 in the amino acid sequence shown in SEQ ID NO:1 or at a position corresponding to this position, is substituted with serine; and/or
(74) an amino acid residue at position 363 in the amino acid sequence shown in SEQ ID NO:1 or at a position corresponding to this position, is substituted with glycine; and/or
(75) an amino acid residue at position 391 in the amino acid sequence shown in SEQ ID NO:1 or at a position corresponding to this position, is substituted with aspartic acid or tryptophan; and/or
(76) an amino acid residue at position 394 in the amino acid sequence shown in SEQ ID NO:1 or at a position corresponding to this position, is substituted with cysteine, aspartic acid, glutamine or threonine; and/or
(77) an amino acid residue at position 398 in the amino acid sequence shown in SEQ ID NO:1 or at a position corresponding to this position, is substituted with cysteine or arginine; and/or
(78) an amino acid residue at position 439 in the amino acid sequence shown in SEQ ID NO:1 or at a position corresponding to this position, is substituted with cysteine; and/or
(79) an amino acid residue at position 440 in the amino acid sequence shown in SEQ ID NO:1 or at a position corresponding to this position, is substituted with leucine; and/or
(80) an amino acid residue at position 441 in the amino acid sequence shown in SEQ ID NO:1 or at a position corresponding to this position, is substituted with glycine, histidine, lysine, asparagine, proline, arginine, serine or threonine; and/or
(81) an amino acid residue at position 442 in the amino acid sequence shown in SEQ ID NO:1 or at a position corresponding to this position, is substituted with alanine, cysteine, glutamic acid, glycine, histidine, leucine, methionine, asparagine, glutamine, arginine, serine or threonine; and/or
(82) an amino acid residue at position 447 in the amino acid sequence shown in SEQ ID NO:1 or at a position corresponding to this position, is substituted with glutamine; and/or
(83) an amino acid residue at position 453 in the amino acid sequence shown in SEQ ID NO:1 or at a position corresponding to this position, is substituted with cysteine, glycine, leucine, asparagine, proline, arginine or serine; and/or
(84) an amino acid residue at position 459 in the amino acid sequence shown in SEQ ID NO:1 or at a position corresponding to this position, is substituted with aspartic acid, glutamic acid, glycine, methionine, asparagine, glutamine, arginine, threonine or tyrosine; and/or
(85) an amino acid residue at position 460 in the amino acid sequence shown in SEQ ID NO:1 or at a position corresponding to this position, is substituted with glutamic acid, phenylalanine, glycine, leucine, methionine, asparagine, glutamine or arginine; and/or
(86) an amino acid residue at position 461 in the amino acid sequence shown in SEQ ID NO:1 or at a position corresponding to this position, is substituted with alanine, glycine, leucine, methionine, glutamine, serine or valine; and/or
(87) an amino acid residue at position 479 in the amino acid sequence shown in SEQ ID NO:1 or at a position corresponding to this position, is substituted with cysteine, isoleucine, leucine, methionine, serine, threonine or valine; and/or
(88) an amino acid residue at position 496 in the amino acid sequence shown in SEQ ID NO:1 or at a position corresponding to this position, is substituted with leucine.

5. A nucleic acid molecule encoding the MDC variant of any one of claims 1 to 4.

6. A vector comprising the nucleic acid molecule of claim 5.

7. A host cell comprising the nucleic acid molecule of claim 5 or the vector of claim 6.

8. Use of the MDC variant of any one of claims 1 to 4 or the host cell of claim 7 for the conversion of 3-methylcrotonic acid into isobutene.

9. A method for producing isobutene from 3-methylcrotonic acid by incubating 3-methylcrotonic acid with the MDC variant of any one of claims 1 to 4.

10. The method of claim 9, wherein the enzymatic conversion is carried out in vitro.

11. A composition comprising a variant of an MDC of any one of claims 1 to 4, the nucleic acid molecule of claim 5, the vector of claim 6 or the host cell of claim 7.

12. A composition comprising a variant of an MDC of any one of claims 1 to 4, the nucleic acid molecule of claim 5, the vector of claim 6 or the host cell of claim 7 and 3-methylcrotonic acid.

## Patentansprüche

1. Variante einer 3-Methylcrotonsäure-Decarboxylase (MDC), die eine verbesserte Aktivität bei der Umsetzung von 3-Methylcrotonsäure in Isobuten gegenüber der MDC von SEQ ID NO:1 zeigt, wobei die Variante **dadurch gekennzeichnet ist, dass**:
sie eine Aminosäure aufweist, die mindestens 80% Sequenzidentität zu SEQ ID NO:1 aufweist; und eine oder mehrere Aminosäuresubstitutionen durch einen anderen Aminosäurerest an einer Position zeigt, die ausgewählt ist aus der Gruppe bestehend aus den Positionen 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462 und 473 in der in SEQ ID NO:1 gezeigten Aminosäuresequenz oder an einer dieser Positionen entsprechenden Position.

2. MDC-Variante nach Anspruch 1 wobei,
(1) ein Aminosäurerest an Position 198 in der in SEQ ID NO: 1 gezeigten Aminosäuresequenz oder an einer dieser Position entsprechenden Position durch Glutamin oder Tryptophan substituiert ist; und/oder
(2) ein Aminosäurerest an Position 240 in der in SEQ ID NO: 1 gezeigten Aminosäuresequenz oder an einer dieser Position entsprechenden Position durch Isoleucin oder Asparagin substituiert ist; und/oder
(3) ein Aminosäurerest an Position 390 in der in SEQ ID NO: 1 gezeigten Aminosäuresequenz oder an einer dieser Position entsprechenden Position durch Serin, Asparaginsäure, Leucin, Asparagin oder Glutamin substituiert ist; und/oder
(4) ein Aminosäurerest an Position 402 in der in SEQ ID NO: 1 gezeigten Aminosäuresequenz oder an einer dieser Position entsprechenden Position durch Alanin substituiert ist; und/oder
(5) ein Aminosäurerest an Position 406 in der in SEQ ID NO: 1 gezeigten Aminosäuresequenz oder an einer dieser Position entsprechenden Position durch Glycin, Alanin oder Serin substituiert ist; und/oder
(6) ein Aminosäurerest an Position 408 in der in SEQ ID NO: 1 gezeigten Aminosäuresequenz oder an einer dieser Position entsprechenden Position durch Isoleucin, Alanin, Cystein, Leucin oder Methionin substituiert ist; und/oder
(7) ein Aminosäurerest an Position 449 in der in SEQ ID NO: 1 gezeigten Aminosäuresequenz oder an einer dieser Position entsprechenden Position durch Isoleucin, Methionin oder Valin substituiert ist; und/oder
(8) ein Aminosäurerest an Position 25 in der in SEQ ID NO: 1 gezeigten Aminosäuresequenz oder an einer dieser Position entsprechenden Position durch Glutaminsäure substituiert ist; und/oder
(9) ein Aminosäurerest an Position 35 in der in SEQ ID NO: 1 gezeigten Aminosäuresequenz oder an einer dieser Position entsprechenden Position durch Isoleucin substituiert ist; und/oder
(10) ein Aminosäurerest an Position 36 in der in SEQ ID NO: 1 gezeigten Aminosäuresequenz oder an einer dieser Position entsprechenden Position durch Tyrosin substituiert ist; und/oder
(11) ein Aminosäurerest an Position 74 in der in SEQ ID NO: 1 gezeigten Aminosäuresequenz oder an einer dieser Position entsprechenden Position durch Valin substituiert ist; und/oder
(12) ein Aminosäurerest an Position 80 in der in SEQ ID NO: 1 gezeigten Aminosäuresequenz oder an einer dieser Position entsprechenden Position durch Serin substituiert ist; und/oder
(13) ein Aminosäurerest an Position 86 in der in SEQ ID NO: 1 gezeigten Aminosäuresequenz oder an einer dieser Position entsprechenden Position durch Asparagin oder Tyrosin substituiert ist; und/oder
(14) ein Aminosäurerest an Position 126 in der in SEQ ID NO: 1 gezeigten Aminosäuresequenz oder an einer dieser Position entsprechenden Position durch Valin substituiert ist; und/oder
(15) ein Aminosäurerest an Position 129 in der in SEQ ID NO: 1 gezeigten Aminosäuresequenz oder an einer dieser Position entsprechenden Position durch Valin substituiert ist; und/oder
(16) ein Aminosäurerest an Position 166 in der in SEQ ID NO: 1 gezeigten Aminosäuresequenz oder an einer dieser Position entsprechenden Position durch Asparaginsäure substituiert ist; und/oder
(17) ein Aminosäurerest an Position 196 in der in SEQ ID NO: 1 gezeigten Aminosäuresequenz oder an einer dieser Position entsprechenden Position durch Isoleucin substituiert ist; und/oder
(18) ein Aminosäurerest an Position 204 in der in SEQ ID NO: 1 gezeigten Aminosäuresequenz oder an einer dieser Position entsprechenden Position durch Arginin oder Serin substituiert ist; und/oder
(19) ein Aminosäurerest an Position 208 in der in SEQ ID NO: 1 gezeigten Aminosäuresequenz oder an einer dieser Position entsprechenden Position durch Leucin substituiert ist; und/oder
(20) ein Aminosäurerest an Position 236 in der in SEQ ID NO: 1 gezeigten Aminosäuresequenz oder an einer dieser Position entsprechenden Position durch Leucin oder Valin substituiert ist; und/oder
(21) ein Aminosäurerest an Position 237 in der in SEQ ID NO: 1 gezeigten Aminosäuresequenz oder an einer dieser Position entsprechenden Position durch Alanin, Asparaginsäure, Asparagin oder Prolin substituiert ist; und/oder
(22) ein Aminosäurerest an Position 238 in der in SEQ ID NO: 1 gezeigten Aminosäuresequenz oder an einer dieser Position entsprechenden Position durch Histidin, Arginin, Serin oder Tryptophan substituiert ist; und/oder
(23) ein Aminosäurerest an Position 298 in der in SEQ ID NO: 1 gezeigten Aminosäuresequenz oder an einer dieser Position entsprechenden Position durch Serin substituiert ist; und/oder
(24) ein Aminosäurerest an Position 302 in der in SEQ ID NO: 1 gezeigten Aminosäuresequenz oder an einer dieser Position entsprechenden Position durch Alanin, Cystein, Phenylalanin, Lysin, Methionin, Glutamin, Arginin oder Serin substituiert ist; und/oder
(25) ein Aminosäurerest an Position 359 in der in SEQ ID NO: 1 gezeigten Aminosäuresequenz oder an einer dieser Position entsprechenden Position durch Cystein oder Valin substituiert ist; und/oder
(26) ein Aminosäurerest an Position 366 in der in SEQ ID NO: 1 gezeigten Aminosäuresequenz oder an einer dieser Position entsprechenden Position durch Isoleucin oder Phenylalanin substituiert ist; und/oder
(27) ein Aminosäurerest an Position 368 in der in SEQ ID NO: 1 gezeigten Aminosäuresequenz oder an einer dieser Position entsprechenden Position durch Isoleucin oder Methionin substituiert ist; und/oder
(28) ein Aminosäurerest an Position 395 in der in SEQ ID NO: 1 gezeigten Aminosäuresequenz oder an einer dieser Position entsprechenden Position durch Cystein, Glutaminsäure oder Valin substituiert ist; und/oder
(29) ein Aminosäurerest an Position 396 in der in SEQ ID NO: 1 gezeigten Aminosäuresequenz oder an einer dieser Position entsprechenden Position durch Isoleucin substituiert ist; und/oder
(30) ein Aminosäurerest an Position 407 in der in SEQ ID NO: 1 gezeigten Aminosäuresequenz oder an einer dieser Position entsprechenden Position durch Alanin, Cystein, Isoleucin, Lysin, Arginin, Serin oder Threonin substituiert ist; und/oder
(31) ein Aminosäurerest an Position 426 in der in SEQ ID NO: 1 gezeigten Aminosäuresequenz oder an einer dieser Position entsprechenden Position durch Valin substituiert ist; und/oder
(32) ein Aminosäurerest an Position 450 in der in SEQ ID NO: 1 gezeigten Aminosäuresequenz oder an einer dieser Position entsprechenden Position durch Alanin, Histidin, Methionin, Asparagin, Glutamin, Serin oder Valin substituiert ist; und/oder
(33) ein Aminosäurerest an Position 451 in der in SEQ ID NO: 1 gezeigten Aminosäuresequenz oder an einer dieser Position entsprechenden Position durch Asparagin oder Serin substituiert ist; und/oder
(34) ein Aminosäurerest an Position 452 in der in SEQ ID NO: 1 gezeigten Aminosäuresequenz oder an einer dieser Position entsprechenden Position durch Phenylalanin substituiert ist; und/oder
(35) ein Aminosäurerest an Position 454 in der in SEQ ID NO: 1 gezeigten Aminosäuresequenz oder an einer dieser Position entsprechenden Position durch Alanin, Asparaginsäure, Glycin, Histidin, Lysin, Leucin, Methionin, Glutamin, Arginin, Serin, Threonin, Valin oder Tryptophan substituiert ist; und/oder
(36) ein Aminosäurerest an Position 455 in der in SEQ ID NO: 1 gezeigten Aminosäuresequenz oder an einer dieser Position entsprechenden Position durch Alanin, Glutaminsäure, Histidin, Lysin, Asparagin, Serin oder Valin substituiert ist; und/oder
(37) ein Aminosäurerest an Position 456 in der in SEQ ID NO: 1 gezeigten Aminosäuresequenz oder an einer dieser Position entsprechenden Position durch Glutamin substituiert ist; und/oder
(38) ein Aminosäurerest an Position 457 in der in SEQ ID NO: 1 gezeigten Aminosäuresequenz oder an einer dieser Position entsprechenden Position durch Lysin substituiert ist; und/oder
(39) ein Aminosäurerest an Position 458 in der in SEQ ID NO: 1 gezeigten Aminosäuresequenz oder an einer dieser Position entsprechenden Position durch Glutaminsäure, Phenylalanin, Glycin, Leucin, Methionin, Asparagin, Glutamin, Arginin, Serin oder Tyrosin substituiert ist; und/oder
(40) ein Aminosäurerest an Position 462 in der in SEQ ID NO: 1 gezeigten Aminosäuresequenz oder an einer dieser Position entsprechenden Position durch Cystein oder Valin substituiert ist; und/oder
(41) ein Aminosäurerest an Position 473 in der in SEQ ID NO: 1 gezeigten Aminosäuresequenz oder an einer dieser Position entsprechenden Position durch Serin substituiert ist.

3. MDC-Variante nach Anspruch 1 oder 2, wobei die Variante **dadurch gekennzeichnet ist, dass** sie eine oder mehrere weitere Aminosäuresubstitutionen an einer Position aufweist, die ausgewählt ist aus der Gruppe bestehend aus 2, 3, 5, 6, 24, 64, 66, 85, 154, 168, 175, 195, 197, 214, 241, 277, 296, 305, 339, 343, 347, 349, 350, 363, 391, 394, 398, 401, 403, 404, 405, 439, 440, 441, 442, 443, 444, 445, 446, 447, 448, 453, 459, 460, 461, 479 und 496 in der in SEQ ID NO:1 gezeigten Aminosäuresequenz oder an einer diesen Positionen entsprechenden Position, und wobei die MDC eine verbesserte Aktivität bei der Umsetzung von 3-Methylcrotonsäure in Isobuten gegenüber der entsprechenden MDC, von der sie stammt, aufweist.

4. MDC-Variante nach Anspruch 3, wobei
(42) ein Aminosäurerest an Position 85 in der in SEQ ID NO: 1 gezeigten Aminosäuresequenz oder an einer dieser Position entsprechenden Position durch Leucin substituiert ist; und/oder
(43) ein Aminosäurerest an Position 241 in der in SEQ ID NO: 1 gezeigten Aminosäuresequenz oder an einer dieser Position entsprechenden Position durch Asparaginsäure, Asparagin oder Valin substituiert ist; und/oder
(44) ein Aminosäurerest an Position 401 in der in SEQ ID NO: 1 gezeigten Aminosäuresequenz oder an einer dieser Position entsprechenden Position durch Tyrosin substituiert ist; und/oder
(45) ein Aminosäurerest an Position 403 in der in SEQ ID NO: 1 gezeigten Aminosäuresequenz oder an einer dieser Position entsprechenden Position durch Alanin, Cystein, Glycin, Histidin, Isoleucin, Asparagin, Prolin, Glutamin, Arginin oder Valin substituiert ist; und/oder
(46) ein Aminosäurerest an Position 404 in der in SEQ ID NO: 1 gezeigten Aminosäuresequenz oder an einer dieser Position entsprechenden Position durch Phenylalanin, Leucin oder Methionin substituiert ist; und/oder
(47) ein Aminosäurerest an Position 405 in der in SEQ ID NO: 1 gezeigten Aminosäuresequenz oder an einer dieser Position entsprechenden Position durch Histidin, Isoleucin, Methionin, Threonin oder Valin substituiert ist; und/oder
(48) ein Aminosäurerest an Position 443 in der in SEQ ID NO: 1 gezeigten Aminosäuresequenz oder an einer dieser Position entsprechenden Position durch Alanin, Asparaginsäure, Phenylalanin, Histidin, Leucin, Asparagin, Arginin, Serin, Tryptophan oder Tyrosin substituiert ist; und/oder
(49) ein Aminosäurerest an Position 444 in der in SEQ ID NO: 1 gezeigten Aminosäuresequenz oder an einer dieser Position entsprechenden Position durch Alanin, Glutaminsäure, Phenylalanin, Histidin, Isoleucin, Leucin, Glutamin oder Threonin substituiert ist; und/oder
(50) ein Aminosäurerest an Position 445 in der in SEQ ID NO: 1 gezeigten Aminosäuresequenz oder an einer dieser Position entsprechenden Position durch Leucin, Alanin, Cystein, Serin oder Threonin substituiert ist; und/oder
(51) ein Aminosäurerest an Position 446 in der in SEQ ID NO: 1 gezeigten Aminosäuresequenz oder an einer dieser Position entsprechenden Position durch Alanin, Cystein, Phenylalanin, Isoleucin, Methionin, Asparagin, Serin oder Valin substituiert ist; und/oder
(52) ein Aminosäurerest an Position 448 in der in SEQ ID NO: 1 gezeigten Aminosäuresequenz oder an einer dieser Position entsprechenden Position durch Phenylalanin, Tryptophan oder Tyrosin substituiert ist; und/oder
(53) ein Aminosäurerest an Position 2 in der in SEQ ID NO: 1 gezeigten Aminosäuresequenz oder an einer dieser Position entsprechenden Position durch Asparagin substituiert ist; und/oder
(54) ein Aminosäurerest an Position 3 in der in SEQ ID NO: 1 gezeigten Aminosäuresequenz oder an einer dieser Position entsprechenden Position durch Leucin oder Methionin substituiert ist; und/oder
(55) ein Aminosäurerest an Position 5 in der in SEQ ID NO: 1 gezeigten Aminosäuresequenz oder an einer dieser Position entsprechenden Position durch Isoleucin substituiert ist; und/oder
(56) ein Aminosäurerest an Position 6 in der in SEQ ID NO: 1 gezeigten Aminosäuresequenz oder an einer dieser Position entsprechenden Position durch Methionin substituiert ist; und/oder
(57) ein Aminosäurerest an Position 24 in der in SEQ ID NO: 1 gezeigten Aminosäuresequenz oder an einer dieser Position entsprechenden Position durch Alanin, Asparaginsäure, Leucin oder Valin substituiert ist; und/oder
(58) ein Aminosäurerest an Position 64 in der in SEQ ID NO: 1 gezeigten Aminosäuresequenz oder an einer dieser Position entsprechenden Position durch Histidin substituiert ist; und/oder
(59) ein Aminosäurerest an Position 66 in der in SEQ ID NO: 1 gezeigten Aminosäuresequenz oder an einer dieser Position entsprechenden Position durch Histidin substituiert ist; und/oder
(60) ein Aminosäurerest an Position 154 in der in SEQ ID NO: 1 gezeigten Aminosäuresequenz oder an einer dieser Position entsprechenden Position durch Asparaginsäure substituiert ist; und/oder
(61) ein Aminosäurerest an Position 168 in der in SEQ ID NO: 1 gezeigten Aminosäuresequenz oder an einer dieser Position entsprechenden Position durch Valin substituiert ist; und/oder
(62) ein Aminosäurerest an Position 175 in der in SEQ ID NO: 1 gezeigten Aminosäuresequenz oder an einer dieser Position entsprechenden Position durch Glutaminsäure substituiert ist; und/oder
(63) ein Aminosäurerest an Position 195 in der in SEQ ID NO: 1 gezeigten Aminosäuresequenz oder an einer dieser Position entsprechenden Position durch Isoleucin oder Valin substituiert ist; und/oder
(64) ein Aminosäurerest an Position 197 in der in SEQ ID NO: 1 gezeigten Aminosäuresequenz oder an einer dieser Position entsprechenden Position durch Valin substituiert ist; und/oder
(65) ein Aminosäurerest an Position 214 in der in SEQ ID NO: 1 gezeigten Aminosäuresequenz oder an einer dieser Position entsprechenden Position durch Threonin substituiert ist; und/oder
(66) ein Aminosäurerest an Position 277 in der in SEQ ID NO: 1 gezeigten Aminosäuresequenz oder an einer dieser Position entsprechenden Position durch Tyrosin substituiert ist; und/oder
(67) ein Aminosäurerest an Position 296 in der in SEQ ID NO: 1 gezeigten Aminosäuresequenz oder an einer dieser Position entsprechenden Position durch Glutamin substituiert ist; und/oder
(68) ein Aminosäurerest an Position 305 in der in SEQ ID NO: 1 gezeigten Aminosäuresequenz oder an einer dieser Position entsprechenden Position durch Isoleucin oder Threonin substituiert ist; und/oder
(69) ein Aminosäurerest an Position 339 in der in SEQ ID NO: 1 gezeigten Aminosäuresequenz oder an einer dieser Position entsprechenden Position durch Histidin substituiert ist; und/oder
(70) ein Aminosäurerest an Position 343 in der in SEQ ID NO: 1 gezeigten Aminosäuresequenz oder an einer dieser Position entsprechenden Position durch Serin substituiert ist; und/oder
(70) ein Aminosäurerest an Position 347 in der in SEQ ID NO: 1 gezeigten Aminosäuresequenz oder an einer dieser Position entsprechenden Position durch Cystein substituiert ist; und/oder
(72) ein Aminosäurerest an Position 349 in der in SEQ ID NO: 1 gezeigten Aminosäuresequenz oder an einer dieser Position entsprechenden Position durch Methionin oder Serin substituiert ist; und/oder
(73) ein Aminosäurerest an Position 350 in der in SEQ ID NO: 1 gezeigten Aminosäuresequenz oder an einer dieser Position entsprechenden Position durch Serin substituiert ist; und/oder
(74) ein Aminosäurerest an Position 363 in der in SEQ ID NO: 1 gezeigten Aminosäuresequenz oder an einer dieser Position entsprechenden Position durch Glycin substituiert ist; und/oder
(75) ein Aminosäurerest an Position 391 in der in SEQ ID NO: 1 gezeigten Aminosäuresequenz oder an einer dieser Position entsprechenden Position durch Asparaginsäure oder Tryptophan substituiert ist; und/oder
(76) ein Aminosäurerest an Position 394 in der in SEQ ID NO: 1 gezeigten Aminosäuresequenz oder an einer dieser Position entsprechenden Position durch Cystein, Asparaginsäure, Glutamin oder Threonin substituiert ist; und/oder
(77) ein Aminosäurerest an Position 398 in der in SEQ ID NO: 1 gezeigten Aminosäuresequenz oder an einer dieser Position entsprechenden Position durch Cystein oder Arginin substituiert ist; und/oder
(78) ein Aminosäurerest an Position 439 in der in SEQ ID NO: 1 gezeigten Aminosäuresequenz oder an einer dieser Position entsprechenden Position durch Cystein substituiert ist; und/oder
(79) ein Aminosäurerest an Position 440 in der in SEQ ID NO: 1 gezeigten Aminosäuresequenz oder an einer dieser Position entsprechenden Position durch Leucin substituiert ist; und/oder
(80) ein Aminosäurerest an Position 441 in der in SEQ ID NO: 1 gezeigten Aminosäuresequenz oder an einer dieser Position entsprechenden Position durch Glycin, Histidin, Lysin, Asparagin, Prolin, Arginin, Serin oder Threonin substituiert ist; und/oder
(81) ein Aminosäurerest an Position 442 in der in SEQ ID NO: 1 gezeigten Aminosäuresequenz oder an einer dieser Position entsprechenden Position durch Alanin, Cystein, Glutaminsäure, Glycin, Histidin, Leucin, Methionin, Asparagin, Glutamin, Arginin, Serin oder Threonin substituiert ist; und/oder
(82) ein Aminosäurerest an Position 447 in der in SEQ ID NO: 1 gezeigten Aminosäuresequenz oder an einer dieser Position entsprechenden Position durch Glutamin substituiert ist; und/oder
(83) ein Aminosäurerest an Position 453 in der in SEQ ID NO: 1 gezeigten Aminosäuresequenz oder an einer dieser Position entsprechenden Position durch Cystein, Glycin, Leucin, Asparagin, Prolin, Arginin oder Serin substituiert ist; und/oder
(84) ein Aminosäurerest an Position 459 in der in SEQ ID NO: 1 gezeigten Aminosäuresequenz oder an einer dieser Position entsprechenden Position durch Asparaginsäure, Glutaminsäure, Glycin, Methionin, Asparagin, Glutamin, Arginin, Threonin oder Tyrosin substituiert ist; und/oder
(85) ein Aminosäurerest an Position 460 in der in SEQ ID NO: 1 gezeigten Aminosäuresequenz oder an einer dieser Position entsprechenden Position durch Glutaminsäure, Phenylalanin, Glycin, Leucin, Methionin, Asparagin, Glutamin oder Arginin substituiert ist; und/oder
(86) ein Aminosäurerest an Position 461 in der in SEQ ID NO: 1 gezeigten Aminosäuresequenz oder an einer dieser Position entsprechenden Position durch Alanin, Glycin, Leucin, Methionin, Glutamin, Serin oder Valin substituiert ist; und/oder
(87) ein Aminosäurerest an Position 479 in der in SEQ ID NO: 1 gezeigten Aminosäuresequenz oder an einer dieser Position entsprechenden Position durch Cystein, Isoleucin, Leucin, Methionin, Serin, Threonin oder Valin substituiert ist; und/oder
(88) ein Aminosäurerest an Position 496 in der in SEQ ID NO: 1 gezeigten Aminosäuresequenz oder an einer dieser Position entsprechenden Position durch Leucin substituiert ist.

5. Nucleinsäuremolekül, das die MDC-Variante nach einem der Ansprüche 1 bis 4 codiert.

6. Vektor, der das Nucleinsäuremolekül nach Anspruch 5 umfasst.

7. Wirtszelle, die das Nucleinsäuremolekül nach Anspruch 5 oder den Vektor nach Anspruch 6 umfasst.

8. Verwendung der MDC-Variante nach einem der Ansprüche 1 bis 4 oder der Wirtszelle nach Anspruch 7 zur Umsetzung von 3-Methylcrotonsäure in Isobuten.

9. Verfahren zur Herstellung von Isobuten aus 3-Methylcrotonsäure durch Inkubieren von 3-Methylcrotonsäure mit der MDC-Variante nach einem der Ansprüche 1 bis 4.

10. Verfahren nach Anspruch 9, wobei die enzymatische Umsetzung *in vitro* durchgeführt wird.

11. Zusammensetzung, die eine Variante einer MDC nach einem der Ansprüche 1 bis 4, das Nucleinsäuremolekül nach Anspruch 5, den Vektor nach Anspruch 6 oder die Wirtszelle nach Anspruch 7 umfasst.

12. Zusammensetzung, die eine Variante einer MDC nach einem der Ansprüche 1 bis 4, das Nucleinsäuremolekül nach Anspruch 5, den Vektor nach Anspruch 6 oder die Wirtszelle nach Anspruch 7 und 3-Methylcrotonsäure umfasst.

## Revendications

1. Variant d'une décarboxylase de l'acide 3-méthylcrotonique (MDC) présentant une activité améliorée dans la conversion de l'acide 3-méthylcrotonique en isobutène par rapport à la MDC de SEQ ID NO:1,
ledit variant étant **caractérisé en ce que**:
il a une séquence d'acides aminés ayant une identité de séquence d'au moins 80% avec la SEQ ID NO:1; et
il présente une ou plusieurs substitutions d'acide aminé par un autre résidu d'acide aminé en une position choisie dans le groupe constitué des positions 406, 25, 35, 36, 74, 80, 86, 126, 129, 166, 196, 198, 204, 208, 236, 237, 238, 240, 298, 302, 359, 366, 368, 390, 395, 396, 402, 407, 408, 426, 449, 450, 451, 452, 454, 455, 456, 457, 458, 462 et 473 dans la séquence d'acides aminés présentée dans la SEQ ID NO: 1 ou en une position correspondant à l'une quelconque desdites positions.

2. Variant de la MDC selon la revendication 1, dans lequel
(1) un résidu d'acide aminé en position 198 dans la séquence d'acides aminés présentée dans la SEQ ID NO:1 ou en une position correspondant à ladite position est substitué par la glutamine ou le tryptophane; et/ou
(2) un résidu d'acide aminé en position 240 dans la séquence d'acides aminés présentée dans la SEQ ID NO:1 ou en une position correspondant à ladite position est substitué par l'isoleucine ou l'asparagine ; et/ou
(3) un résidu d'acide aminé en position 390 dans la séquence d'acides aminés présentée dans la SEQ ID NO:1 ou en une position correspondant à ladite position est substitué par la sérine, l'acide aspartique, la leucine, l'asparagine ou la glutamine ; et/ou
(4) un résidu d'acide aminé en position 402 dans la séquence d'acides aminés présentée dans la SEQ ID NO:1 ou en une position correspondant à ladite position est substitué par l'alanine ; et/ou
(5) un résidu d'acide aminé en position 406 dans la séquence d'acides aminés présentée dans la SEQ ID NO:1 ou en une position correspondant à ladite position est substitué par la glycine, l'alanine ou la sérine ; et/ou
(6) un résidu d'acide aminé en position 408 dans la séquence d'acides aminés présentée dans la SEQ ID NO:1 ou en une position correspondant à ladite position est substitué par l'isoleucine, l'alanine, la cystéine, la leucine ou la méthionine ; et/ou
(7) un résidu d'acide aminé en position 449 dans la séquence d'acides aminés présentée dans la SEQ ID NO:1 ou en une position correspondant à ladite position est substitué par l'isoleucine, la méthionine ou la valine ; et/ou
(8) un résidu d'acide aminé en position 25 dans la séquence d'acides aminés présentée dans la SEQ ID NO:1 ou en une position correspondant à ladite position est substitué par l'acide glutamique ; et/ou
(9) un résidu d'acide aminé en position 35 dans la séquence d'acides aminés présentée dans la SEQ ID NO:1 ou en une position correspondant à ladite position est substitué par l'isoleucine ; et/ou
(10) un résidu d'acide aminé en position 36 dans la séquence d'acides aminés présentée dans la SEQ ID NO:1 ou en une position correspondant à ladite position est substitué par la tyrosine ; et/ou
(11) un résidu d'acide aminé en position 74 dans la séquence d'acides aminés présentée dans la SEQ ID NO:1 ou en une position correspondant à ladite position est substitué par la valine ; et/ou
(12) un résidu d'acide aminé en position 80 dans la séquence d'acides aminés présentée dans la SEQ ID NO:1 ou en une position correspondant à ladite position est substitué par la sérine ; et/ou
(13) un résidu d'acide aminé en position 86 dans la séquence d'acides aminés présentée dans la SEQ ID NO:1 ou en une position correspondant à ladite position est substitué par l'asparagine ou la tyrosine; et/ou
(14) un résidu d'acide aminé en position 126 dans la séquence d'acides aminés présentée dans la SEQ ID NO:1 ou en une position correspondant à ladite position est substitué par la valine ; et/ou
(15) un résidu d'acide aminé en position 129 dans la séquence d'acides aminés présentée dans la SEQ ID NO:1 ou en une position correspondant à ladite position est substitué par la valine ; et/ou
(16) un résidu d'acide aminé en position 166 dans la séquence d'acides aminés présentée dans la SEQ ID NO:1 ou en une position correspondant à ladite position est substitué par l'acide aspartique ; et/ou
(17) un résidu d'acide aminé en position 196 dans la séquence d'acides aminés présentée dans la SEQ ID NO:1 ou en une position correspondant à ladite position est substitué par l'isoleucine ; et/ou
(18) un résidu d'acide aminé en position 204 dans la séquence d'acides aminés présentée dans la SEQ ID NO:1 ou en une position correspondant à ladite position est substitué par l'arginine ou la sérine ; et/ou
(19) un résidu d'acide aminé en position 208 dans la séquence d'acides aminés présentée dans la SEQ ID NO:1 ou en une position correspondant à ladite position est substitué par la leucine ; et/ou
(20) un résidu d'acide aminé en position 236 dans la séquence d'acides aminés présentée dans la SEQ ID NO:1 ou en une position correspondant à ladite position est substitué par la leucine ou la valine ; et/ou
(21) un résidu d'acide aminé en position 237 dans la séquence d'acides aminés présentée dans la SEQ ID NO:1 ou en une position correspondant à ladite position est substitué par l'alanine, l'acide aspartique, l'asparagine ou la proline, et/ou
(22) un résidu d'acide aminé en position 238 dans la séquence d'acides aminés présentée dans la SEQ ID NO:1 ou en une position correspondant à ladite position est substitué par l'histidine, l'arginine, la sérine ou le tryptophane ; et/ou
(23) un résidu d'acide aminé en position 298 dans la séquence d'acides aminés présentée dans la SEQ ID NO:1 ou en une position correspondant à ladite position est substitué par la sérine; et/ou
(24) un résidu d'acide aminé en position 302 dans la séquence d'acides aminés présentée dans la SEQ ID NO:1 ou en une position correspondant à ladite position est substitué par l'alanine, la cystéine, la phénylalanine, la lysine, la méthionine, la glutamine, l'arginine ou la sérine ; et/ou
(25) un résidu d'acide aminé en position 359 dans la séquence d'acides aminés présentée dans la SEQ ID NO:1 ou en une position correspondant à ladite position est substitué par la cystéine ou la valine ; et/ou
(26) un résidu d'acide aminé en position 366 dans la séquence d'acides aminés présentée dans la SEQ ID NO:1 ou en une position correspondant à ladite position est substitué par l'isoleucine ou la phénylalanine ; et/ou
(27) un résidu d'acide aminé en position 368 dans la séquence d'acides aminés présentée dans la SEQ ID NO:1 ou en une position correspondant à ladite position est substitué par l'isoleucine ou la méthionine ; et/ou
(28) un résidu d'acide aminé en position 395 dans la séquence d'acides aminés présentée dans la SEQ ID NO:1 ou en une position correspondant à ladite position est substitué par la cystéine, l'acide glutamique ou la valine ; et/ou
(29) un résidu d'acide aminé en position 396 dans la séquence d'acides aminés présentée dans la SEQ ID NO:1 ou en une position correspondant à ladite position est substitué par l'isoleucine ; et/ou
(30) un résidu d'acide aminé en position 407 dans la séquence d'acides aminés présentée dans la SEQ ID NO:1 ou en une position correspondant à ladite position est substitué par l'alanine, la cystéine, l'isoleucine, la lysine, l'arginine, la sérine ou la thréonine ; et/ou
(31) un résidu d'acide aminé en position 426 dans la séquence d'acides aminés présentée dans la SEQ ID NO:1 ou en une position correspondant à ladite position est substitué par la valine ; et/ou
(32) un résidu d'acide aminé en position 450 dans la séquence d'acides aminés présentée dans la SEQ ID NO:1 ou en une position correspondant à ladite position est substitué par l'alanine, l'histidine, la méthionine, l'asparagine, la glutamine, la sérine ou la valine ; et/ou
(33) un résidu d'acide aminé en position 451 dans la séquence d'acides aminés présentée dans la SEQ ID NO:1 ou en une position correspondant à ladite position est substitué par l'asparagine ou la sérine ; et/ou
(34) un résidu d'acide aminé en position 452 dans la séquence d'acides aminés présentée dans la SEQ ID NO:1 ou en une position correspondant à ladite position est substitué par la phénylalanine ; et/ou
(35) un résidu d'acide aminé en position 454 dans la séquence d'acides aminés présentée dans la SEQ ID NO:1 ou en une position correspondant à ladite position est substitué par l'alanine, l'acide aspartique, la glycine, l'histidine, la lysine, la leucine, la méthionine, la glutamine, l'arginine, la sérine, la thréonine, la valine ou le tryptophane ; et/ou
(36) un résidu d'acide aminé en position 455 dans la séquence d'acides aminés présentée dans la SEQ ID NO:1 ou en une position correspondant à ladite position est substitué par l'alanine, l'acide glutamique, l'histidine, la lysine, l'asparagine, la sérine, ou la valine ; et/ou
(37) un résidu d'acide aminé en position 456 dans la séquence d'acides aminés présentée dans la SEQ ID NO:1 ou en une position correspondant à ladite position est substitué par la glutamine; et/ou
(38) un résidu d'acide aminé en position 457 dans la séquence d'acides aminés présentée dans la SEQ ID NO:1 ou en une position correspondant à ladite position est substitué par la lysine; et/ou
(39) un résidu d'acide aminé en position 458 dans la séquence d'acides aminés présentée dans la SEQ ID NO:1 ou en une position correspondant à ladite position est substitué par l'acide glutamique, la phénylalanine, la glycine, la leucine, la méthionine, l'asparagine, la glutamine, l'arginine, la sérine, ou la tyrosine ; et/ou
(40) un résidu d'acide aminé en position 462 dans la séquence d'acides aminés présentée dans la SEQ ID NO:1 ou en une position correspondant à ladite position est substitué par la cystéine ou la valine ; et/ou
(41) un résidu d'acide aminé en position 473 dans la séquence d'acides aminés présentée dans la SEQ ID NO:1 ou en une position correspondant à ladite position est substitué par la sérine.

3. Variant de la MDC selon la revendication 1 ou 2, ledit variant étant **caractérisé en ce qu'**il possède une ou plusieurs autres substitutions d'acide aminé en une position choisie dans le groupe constitué de 2, 3, 5, 6, 24, 64, 66, 85, 154, 168, 175, 195, 197, 214, 241, 277, 296, 305, 339, 343, 347, 349, 350, 363, 391, 394, 398, 401, 403, 404, 405, 439, 440, 441, 442, 443, 444, 445, 446, 447, 448, 453, 459, 460, 461, 479 et 496 dans la séquence d'acides aminés présentée dans la SEQ ID NO:1 ou en une position correspondant à l'une quelconque de ces positions, et ladite MDC ayant une activité améliorée dans la conversion de l'acide 3-méthylcrotonique en isobutène par rapport à la MDC dont elle est dérivée.

4. Variant de la MDC selon la revendication 3, dans lequel
(42) un résidu d'acide aminé en position 85 dans la séquence d'acides aminés présentée dans la SEQ ID NO:1 ou en une position correspondant à ladite position est substitué par la leucine ; et/ou
(43) un résidu d'acide aminé en position 241 dans la séquence d'acides aminés présentée dans la SEQ ID NO:1 ou en une position correspondant à ladite position est substitué par l'acide aspartique, l'asparagine ou la valine ; et/ou
(44) un résidu d'acide aminé en position 401 dans la séquence d'acides aminés présentée dans la SEQ ID NO:1 ou en une position correspondant à ladite position est substitué par la tyrosine ; et/ou
(45) un résidu d'acide aminé en position 403 dans la séquence d'acides aminés présentée dans la SEQ ID NO:1 ou en une position correspondant à ladite position est substitué par l'alanine, la cystéine, la glycine, l'histidine, l'isoleucine, l'asparagine, la proline, la glutamine, l'arginine ou la valine ; et/ou
(46) un résidu d'acide aminé en position 404 dans la séquence d'acides aminés présentée dans la SEQ ID NO:1 ou en une position correspondant à ladite position est substitué par la phénylalanine, la leucine ou la méthionine ; et/ou
(47) un résidu d'acide aminé en position 405 dans la séquence d'acides aminés présentée dans la SEQ ID NO:1 ou en une position correspondant à ladite position est substitué par l'histidine, l'isoleucine, la méthionine, la thréonine ou la valine ; et/ou
(48) un résidu d'acide aminé en position 443 dans la séquence d'acides aminés présentée dans la SEQ ID NO:1 ou en une position correspondant à ladite position est substitué par l'alanine, l'acide aspartique, la phénylalanine, l'histidine, la leucine, l'asparagine, l'arginine, la sérine, le tryptophane ou la tyrosine ; et/ou
(49) un résidu d'acide aminé en position 444 dans la séquence d'acides aminés présentée dans la SEQ ID NO:1 ou en une position correspondant à ladite position est substitué par l'alanine, l'acide glutamique, la phénylalanine, l'histidine, l'isoleucine, la leucine, la glutamine ou la thréonine ; et/ou
(50) un résidu d'acide aminé en position 445 dans la séquence d'acides aminés présentée dans la SEQ ID NO:1 ou en une position correspondant à ladite position est substitué par la leucine, l'alanine, la cystéine, la sérine ou la thréonine; et/ou
(51) un résidu d'acide aminé en position 446 dans la séquence d'acides aminés présentée dans la SEQ ID NO:1 ou en une position correspondant à ladite position est substitué par l'alanine, la cystéine, la phénylalanine, l'isoleucine, la méthionine, l'asparagine, la sérine ou la valine ; et/ou
(52) un résidu d'acide aminé en position 448 dans la séquence d'acides aminés présentée dans la SEQ ID NO:1 ou en une position correspondant à ladite position est substitué par la phénylalanine, le tryptophane ou la tyrosine ; et/ou
(53) un résidu d'acide aminé en position 2 dans la séquence d'acides aminés présentée dans la SEQ ID NO:1 ou en une position correspondant à ladite position est substitué par l'asparagine ; et/ou
(54) un résidu d'acide aminé en position 3 dans la séquence d'acides aminés présentée dans la SEQ ID NO:1 ou en une position correspondant à ladite position est substitué par la leucine ou la méthionine ; et/ou
(55) un résidu d'acide aminé en position 5 dans la séquence d'acides aminés présentée dans la SEQ ID NO:1 ou en une position correspondant à ladite position est substitué par l'isoleucine ; et/ou
(56) un résidu d'acide aminé en position 6 dans la séquence d'acides aminés présentée dans la SEQ ID NO:1 ou en une position correspondant à ladite position est substitué par la méthionine ; et/ou
(57) un résidu d'acide aminé en position 24 dans la séquence d'acides aminés présentée dans la SEQ ID NO:1 ou en une position correspondant à ladite position est substitué par l'alanine, l'acide aspartique, la leucine ou la valine ; et/ou
(58) un résidu d'acide aminé en position 64 dans la séquence d'acides aminés présentée dans la SEQ ID NO:1 ou en une position correspondant à ladite position est substitué par l'histidine ; et/ou
(59) un résidu d'acide aminé en position 66 dans la séquence d'acides aminés présentée dans la SEQ ID NO:1 ou en une position correspondant à ladite position est substitué par l'histidine ; et/ou
(60) un résidu d'acide aminé en position 154 dans la séquence d'acides aminés présentée dans la SEQ ID NO:1 ou en une position correspondant à ladite position est substitué par l'acide aspartique ; et/ou
(61) un résidu d'acide aminé en position 168 dans la séquence d'acides aminés présentée dans la SEQ ID NO:1 ou en une position correspondant à ladite position est substitué par la valine ; et/ou
(62) un résidu d'acide aminé en position 175 dans la séquence d'acides aminés présentée dans la SEQ ID NO:1 ou en une position correspondant à ladite position est substitué par l'acide glutamique ; et/ou
(63) un résidu d'acide aminé en position 195 dans la séquence d'acides aminés présentée dans la SEQ ID NO:1 ou en une position correspondant à ladite position est substitué par l'isoleucine ou la valine ; et/ou
(64) un résidu d'acide aminé en position 197 dans la séquence d'acides aminés présentée dans la SEQ ID NO:1 ou en une position correspondant à ladite position est substitué par la valine ; et/ou
(65) un résidu d'acide aminé en position 214 dans la séquence d'acides aminés présentée dans la SEQ ID NO:1 ou en une position correspondant à ladite position est substitué par la thréonine ; et/ou
(66) un résidu d'acide aminé en position 277 dans la séquence d'acides aminés présentée dans la SEQ ID NO:1 ou en une position correspondant à ladite position est substitué par la tyrosine ; et/ou
(67) un résidu d'acide aminé en position 296 dans la séquence d'acides aminés présentée dans la SEQ ID NO:1 ou en une position correspondant à ladite position est substitué par la glutamine ; et/ou
(68) un résidu d'acide aminé en position 305 dans la séquence d'acides aminés présentée dans la SEQ ID NO:1 ou en une position correspondant à ladite position est substitué par l'isoleucine ou la thréonine ; et/ou
(69) un résidu d'acide aminé en position 339 dans la séquence d'acides aminés présentée dans la SEQ ID NO:1 ou en une position correspondant à ladite position est substitué par l'histidine ; et/ou
(70) un résidu d'acide aminé en position 343 dans la séquence d'acides aminés présentée dans la SEQ ID NO:1 ou en une position correspondant à ladite position est substitué par la sérine; et/ou
(70) un résidu d'acide aminé en position 347 dans la séquence d'acides aminés présentée dans la SEQ ID NO:1 ou en une position correspondant à ladite position est substitué par la cystéine; et/ou
(72) un résidu d'acide aminé en position 349 dans la séquence d'acides aminés présentée dans la SEQ ID NO:1 ou en une position correspondant à ladite position est substitué par la méthionine ou la sérine; et/ou
(73) un résidu d'acide aminé en position 350 dans la séquence d'acides aminés présentée dans la SEQ ID NO:1 ou en une position correspondant à ladite position est substitué par la sérine; et/ou
(74) un résidu d'acide aminé en position 363 dans la séquence d'acides aminés présentée dans la SEQ ID NO:1 ou en une position correspondant à ladite position est substitué par la glycine; et/ou
(75) un résidu d'acide aminé en position 391 dans la séquence d'acides aminés présentée dans la SEQ ID NO:1 ou en une position correspondant à ladite position est substitué par l'acide aspartique ou le tryptophane; et/ou
(76) un résidu d'acide aminé en position 394 dans la séquence d'acides aminés présentée dans la SEQ ID NO:1 ou en une position correspondant à ladite position est substitué par la cystéine, l'acide aspartique, la glutamine ou la thréonine; et/ou
(77) un résidu d'acide aminé en position 398 dans la séquence d'acides aminés présentée dans la SEQ ID NO:1 ou en une position correspondant à ladite position est substitué par la cystéine ou l'arginine; et/ou
(78) un résidu d'acide aminé en position 439 dans la séquence d'acides aminés présentée dans la SEQ ID NO:1 ou en une position correspondant à ladite position est substitué par la cystéine ; et/ou
(79) un résidu d'acide aminé en position 440 dans la séquence d'acides aminés présentée dans la SEQ ID NO:1 ou en une position correspondant à ladite position est substitué par la leucine; et/ou
(80) un résidu d'acide aminé en position 441 dans la séquence d'acides aminés présentée dans la SEQ ID NO:1 ou en une position correspondant à ladite position est substitué par la glycine, l'histidine, la lysine, l'asparagine, la proline, l'arginine, la sérine ou la thréonine; et/ou
(81) un résidu d'acide aminé en position 442 dans la séquence d'acides aminés présentée dans la SEQ ID NO:1 ou en une position correspondant à ladite position est substitué par l'alanine, la cystéine, l'acide glutamique, la glycine, l'histidine, la leucine, la méthionine, l'asparagine, la glutamine, l'arginine, la sérine ou la thréonine; et/ou
(82) un résidu d'acide aminé en position 447 dans la séquence d'acides aminés présentée dans la SEQ ID NO:1 ou en une position correspondant à ladite position est substitué par la glutamine; et/ou
(83) un résidu d'acide aminé en position 453 dans la séquence d'acides aminés présentée dans la SEQ ID NO:1 ou en une position correspondant à ladite position est substitué par la cystéine, la glycine, la leucine, l'asparagine, la proline, l'arginine ou la sérine; et/ou
(84) un résidu d'acide aminé en position 459 dans la séquence d'acides aminés présentée dans la SEQ ID NO:1 ou en une position correspondant à ladite position est substitué par l'acide aspartique, l'acide glutamique, la glycine, la méthionine, l'asparagine, la glutamine, l'arginine, la thréonine ou la tyrosine; et/ou
(85) un résidu d'acide aminé en position 460 dans la séquence d'acides aminés présentée dans la SEQ ID NO:1 ou en une position correspondant à ladite position est substitué par l'acide glutamique, la phénylalanine, la glycine, la leucine, la méthionine, l'asparagine, la glutamine, ou l'arginine; et/ou
(86) un résidu d'acide aminé en position 461 dans la séquence d'acides aminés présentée dans la SEQ ID NO:1 ou en une position correspondant à ladite position est substitué par l'alanine, la glycine, la leucine, la méthionine, la glutamine, la sérine ou la valine; et/ou
(87) un résidu d'acide aminé en position 479 dans la séquence d'acides aminés présentée dans la SEQ ID NO:1 ou en une position correspondant à ladite position est substitué par la cystéine, l'isoleucine, la leucine, la méthionine, la sérine, la thréonine ou la valine; et/ou
(88) un résidu d'acide aminé en position 496 dans la séquence d'acides aminés présentée dans la SEQ ID NO:1 ou en une position correspondant à ladite position est substitué par la leucine.

5. Molécule d'acide nucléique codant pour le variant de la MDC selon l'une quelconque des revendications 1 à 4.

6. Vecteur comprenant la molécule d'acide nucléique selon la revendication 5.

7. Cellule hôte comprenant la molécule d'acide nucléique selon la revendication 5 ou le vecteur selon la revendication 6.

8. Utilisation du variant de la MDC selon l'une quelconque des revendications 1 à 4 ou cellule hôte selon la revendication 7 pour la conversion de l'acide 3-méthylcrotonique en isobutène.

9. Méthode de production de l'isobutène à partir de l'acide 3-méthylcrotonique par incubation de l'acide 3-méthylcrotonique avec le variant de la MDC selon l'une quelconque des revendications 1 à 4.

10. Méthode selon la revendication 9, dans laquelle la conversion enzymatique est réalisée in vitro.

11. Composition comprenant un variant d'une MDC selon l'une quelconque des revendications 1 à 4, la molécule d'acide nucléique selon la revendication 5, le vecteur selon la revendication 6 ou la cellule hôte selon la revendication 7.

12. Composition comprenant un variant d'une MDC selon l'une quelconque des revendications 1 à 4, la molécule d'acide nucléique selon la revendication 5, le vecteur selon la revendication 6 ou la cellule hôte selon la revendication 7 et de l'acide 3-méthylcrotonique.
